# EUROPEAN PATENT APPLICATION

(11) **EP 2 471 802 A1**
(43) Date of publication of application: **04.07.2012**
(21) Application number: 10197017.6
(22) Date of filing: 27.12.2010
(51) Int. Cl.: C07H 11/04, A61K 31/7024, A61P 29/00

(54) **Phosphoramidates of monosaccharides**

(71) Applicant: Bioiberica, S.A., 08029 Barcelona (ES)
(72) Inventor: McGuigan, Christopher, Cardiff South Wales CF14 2E (GB); Caterson, Bruce, Cardiff South Wales CF11 9DL (GB); Alaez Verson, Carlos Raul, 17300 Blanes (ES); Torrent Gibert, Ana Maria, 17458 Fornells de la Selva (ES); Ruhi Roura, Ramon, 08025 Barcelona (ES); Hughes, Clare E., Cardiff South Wales CF11 9DL (GB); Escaich Ferrer, Josep, 08021 Barcelona (ES)
(74) Representative: Sugrañes Patentes y Marcas

(57) **Abstract**

The present invention relates novel phosphoramidates of monosaccharides of formula (I), wherein R¹ is a monosaccharide group selected from A and B; R², R³, R⁴, R⁵, R⁶, R⁷, R⁸, R⁹, R¹⁰, R¹¹, R¹², R¹³, R¹⁴, R¹⁵, R¹⁶ and X are as defined herein. Preferably X is -O-. The present invention also provides processes for the production of compounds of formula (I), pharmaceutical compositions comprising these compounds, as well as to the use thereof in medical therapy, preferably in the treatment of osteoarthritis. 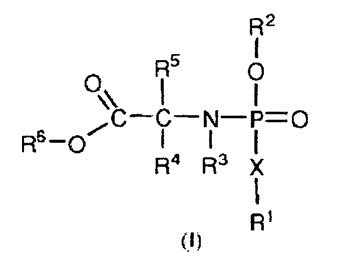 wherein:
R¹ is selected from A and B,

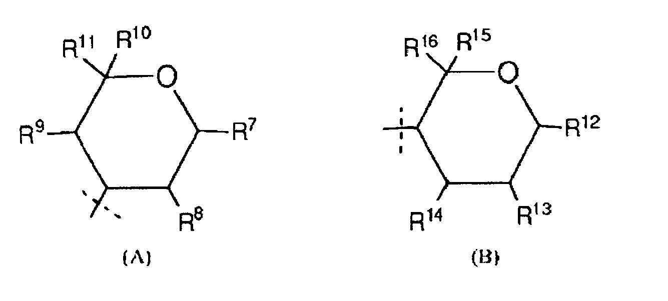

## Description

### Technical field of the invention

The present invention relates to phosphoramidates of monosaccharides, the process for preparing thereof, as well as to the use thereof in medical therapy.

### Background of the invention

Osteoarthritis (arthrosis) is a degenerative joint disease which affects most people from 65 years of age, and which is characterized by a gradual degradation of cartilaginous tissue. In addition, changes occur in the bone, synovium and muscle. Synovial inflammation predominantly develops secondarily to pathological processes in cartilage and bone.

Osteoarthritis can frequently occur in the hand, foot, knee and hips and it mostly affects only one or a few joints. It can be defined as the degeneration of hyaline articular cartilage. A secondary effect thereto is the damage of the synovial membrane and the subchondral bone (the bone in contact with the cartilage), as well as the formation of new bone at the margins of the surfaces of the joint (osteophytes).

Cartilage allows bones to move sliding over one another. It also absorbs the tension caused by the physical movement. In osteoarthritis, the cartilage surface breaks and wears away, causing the bones to move against one another, causing friction, pain, tenderness, swelling and loss of movement in the joint. As time goes on the joint can be deformed.

In normal conditions, cartilage renewal is a very slow process consisting of a constant synthesis (anabolism) and degradation (catabolism) of the components of the extracellular matrix. The chondrocyte is the cell responsible for this metabolism, a process which must be perfectly coordinated.

Although the etiology of osteoarthritis is still unknown, it is currently accepted that the first alterations occur at chondrocyte level. These alterations will subsequently give rise to the onset of an osteoarthritic joint.

A series of risk factors for the onset of the disease has been described, some of them include: ageing, genetic predisposition, obesity, overload disorders, decreased sex hormones (post menopausal women), physical overtraining in sportsmen, injuries or traumas, work activity and low bone mineral density.

Osteoarthritis is a disease that does not have a definitive treatment. No drugs are available with proven disease-modifying efficacy (P. Creamer et al., Lancet 350, 503-509 (1997); H.A. Wieland et al., Nature Rev. Drug Discov. 4, 331-344 (2005)).The current therapeutic possibilities include:

Fast acting symptomatic drugs like analgesics, non-steroidal anti-inflammatory drugs (NSAIDs), corticoids and cyclooxygenase-2 (COX-2) selective inhibitors. The use of some of them involves a high risk of potentially serious side-effects, such as gastrointestinal complications in the case of NSAIDs.

Slow acting symptomatic drugs, also known as SYSADOA (Symptomatic Slow Acting Drug for Osteoarthritis) (M.G. Lequesne, Rev. Rhum. (Eng./Ed.) 61, 69-73 (1994); B.F. Leeb et al., J. Rheumatol. 27, 205-211 (2000)). This group of substances include: hyaluronic acid, chondroitin sulphate, glucosamine hydrochloride and glucosamine sulphate. It is characterized by its higher safety compared with NSAIDs.

Glucosamine and chondroitin products are widely used for management of osteoarthritis and are recommended by the European League Against Rheumatism (EULAR) (K.M. Jordan et al., Ann. Rheum. Dis. 62, 1145-1155 (2003)).

Glucosamine is an aminomonosaccharide used by the cartilage cells as an intermediate substrate in the synthesis of glycosaminoglycans and proteoglycans. It is present as a natural compound in almost all human tissues.

Various research groups have studied the effects of glucosamine hydrochloride and glucosamine sulphate on osteoarthritis. H. Nakamura et al. (Clin. Exp. Rheumatol. 22 (3), 293-9, (2004)) described the effects of glucosamine hydrochloride in the production of prostaglandin E2, nitric oxide, and metalloproteases by chondrocytes and synoviocytes in osteoarthritis. They concluded that glucosamine hydrochloride modulates the metabolism of chondrocytes and synoviocytes. G. Herrero-Beaumont et al. (Arthritis Rheum. 52, S460 (2005)) described the effects of glucosamine sulphate on a 6-month double-blind, multicenter trial in Spain and Portugal, examining placebo vs glucosamine sulphate vs acetaminophen. Although there was a numeric difference in improvement in the Lequesme algofunctional index between acetaminophen and placebo, only the improvement in the Lequesne algofunctional index for glucosamine sulphate vs placebo was significant. Secondary analyses, including the OARSI responder indices were significant for glucosamine.

Despite multiple double-blind, controlled clinical trials on the use of glucosamine in osteoarthritis of the knee, controversy on efficacy related to symptomatic improvement continues. Indeed, meta-analyses have produced conflicting results (R. D. Altman et al., Osteoarthritis & Cartilage 14, 963-966 (2006)).

Phosphoramidates of monosaccharides, including phosphoramidates of glucosamine, have been disclosed. EP 1709056 discloses phosphoramidates in position 6- of pyranose monosaccharides. The compounds of the present invention differ from the compounds of EP 1709056 in that they are phosphoramidates in position 3- or/and 4- of pyranose monosaccharides. In addition, EP 1709056 does not disclose any compound with a proline moiety, while the present invention discloses some compounds including a proline moiety.

In the light of state of the art, there is a need for finding effective compounds in order to obtain effective medicaments for the treatment of osteoarthritis and other musculoskeletal diseases.

### Disclosure of the invention

In a first aspect the present invention relates to a compound of formula (I): 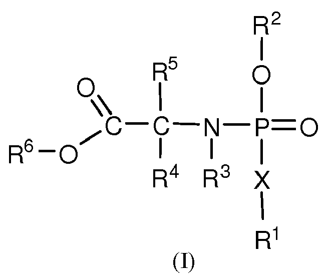 wherein:
R¹ is selected from A and B, 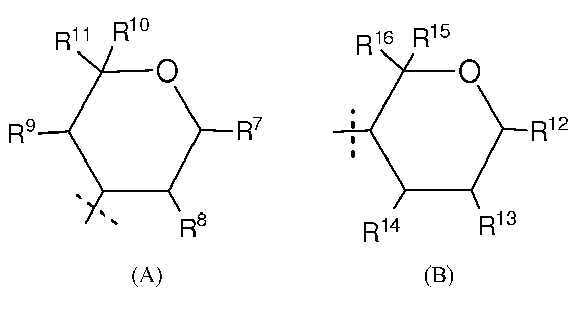
X is selected from -O- and -CH₂-;
R² is selected from the group consisting of hydrogen, C₁-C₁₆ alkyl, C₂-C₁₆ alkenyl, C₂-C₁₆ alkynyl, C₃-C₂₀ cycloalkyl, C₃-C₂₀ cycloalkenyl, C₄-C₂₀ cycloalkynyl, C₇-C₂₀ aralkyl, C₇-C₂₀ alkaryl and C₅-C₂₀ aryl, wherein each of said aralkyl, alkaryl or aryl is optionally substituted with one or more substituents independently selected from the group consisting of halogen, C₁-C₁₀ alkyl, hydroxy, C₁-C₁₀ alcoxy, alcoxyalkyl, amino, C₁-C₁₀ alkylamino, hydroxyakylamino and C₁-C₁₀ alcanoyloxyl;
R³ is selected from the group consisting of hydrogen, C₁-C₁₀ alkyl and C₅-C₁₀ aryl; or R³ and R⁴ together are (CH₂)₃; or R³ and R⁵ together are (CH₂)₃, wherein the group (CH₂)₃ is optionally substituted with one or more substituents independently selected from the group consisting of C₁-C₆ alkyl, hydroxy, C₁-C₆ alcoxy, amino, carboxyl and esters thereof, alcanoyloxyl and halogen;
R⁴ and R⁵ are independently selected from the group consisting of hydrogen, C₁-C₁₆ alkyl,
C₂-C₁₆ alkenyl, C₂-C₁₆ alkynyl, C₃-C₂₀ cycloalkyl, C₃-C₂₀ cycloalkenyl, C₄-C₂₀ cycloalkynyl, C₇-C₂₀ aralkyl, C₇-C₂₀ alkaryl and C₅-C₂₀ aryl, wherein each of said alkyl, alkenyl, alkynyl, cycloalkyl, cycloalkenyl, cycloalkynyl, aralkyl, alkaryl or aryl is optionally substituted with one or more substituents independently selected from the group consisting of hydroxy, C₁-C₆ alcoxy, thiol, C₁-C₆ alkylthio, carboxyl and esters thereof, amido, carbamoyl, imino, H₂NC(=NH)NH-, amino, aminoalkyl and C₁-C₆ alkylamino; or R⁴ and R⁵ together with the carbon atom to which they are attached, form a ring from 3 to 6 members, selected from the group consisting of C₃-C₆ cycloalkyl, C₃-C₆ cycloalkenyl and C₄-C₆ cycloalkynyl; or R⁴ and R³ together are (CH₂)₃; or R⁵ and R³ together are (CH₂)₃, wherein the group (CH₂)₃ is optionally substituted with one or more substituents independently selected from the group consisting of C₁-C₆ alkyl, hydroxy, C₁-C₆ alcoxy, amino, carboxyl and esters thereof, alcanoyloxyl and halogen;
R⁶ is selected from the group consisting of hydrogen, protecting group, C₁-C₁₆ alkyl, C₂-C₁₆ alkenyl, C₂-C₁₆ alkynyl, C₃-C₂₀ cycloalkyl, C₃-C₂₀ cycloalkenyl, C₄-C₂₀ cycloalkynyl, C₇-C₂₀ aralkyl, C₇-C₂₀ alkaryl and C₅-C₂₀ aryl, wherein each of said alkyl, alkenyl, alkynyl, cycloalkyl, cycloalkenyl, cycloalkynyl, aralkyl, alkaryl or aryl is optionally substituted with one or more substituents independently selected from the group consisting of halogen, hydroxy, C₁-C₆ alcoxy, thiol and amino;
R⁷, R⁸, R⁹, R¹⁰, R¹², R¹³, R¹⁴ and R¹⁵ are independently selected from the group consisting of hydrogen, N₃, halogen, -OR¹⁷, -SR¹⁸, -NHR¹⁹, -NR¹⁹₂ and C 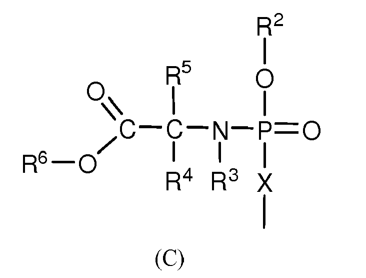
wherein X, R², R³, R⁴, R⁵ and R⁶ are as hereinbefore defined for formula (I), and wherein R¹⁷, R¹⁸ and R¹⁹ are independently selected from the group consisting of hydrogen, protecting group, C₁-C₁₅ alkyl, C₂-C₁₅ alkenyl, C₂-C₁₅ alkynyl, C₃-C₂₀ cycloalkyl, C₃-C₂₀ cycloalkenyl, C₄-C₂₀ cycloalkynyl, C₇-C₂₀ aralkyl, C₇-C₂₀ alkaryl, C₅-C₂₀ aryl, -C(O)R²⁰ and -C(O)OR²⁰, wherein R²⁰ is selected from the group consisting of C₁-C₁₅ alkyl, C₂-C₁₅ alkenyl, C₂-C₁₅ alkynyl, C₇-C₂₀ aralkyl, C₇-C₂₀ alkaryl and C₅-C₂₀ aryl;
R¹¹ and R¹⁶ are independently selected from the group consisting of -CH₃, -CH₂-halogen, -CH₂OR¹⁷, -CH₂SR¹⁸, -CH₂NHR¹⁹, -CH₂NR¹⁹₂ and -C(O)OR²¹, wherein R¹⁷, R¹⁸ and R¹⁹ are independently selected from the group consisting of hydrogen, protecting group, C₁-C₁₅ alkyl, C₂-C₁₅ alkenyl, C₂-C₁₅ alkynyl, C₃-C₂₀ cycloalkyl, C₃-C₂₀ cycloalkenyl, C₄-C₂₀ cycloalkynyl, C₇-C₂₀ aralkyl, C₇-C₂₀ alkaryl, C₅-C₂₀ aryl, -C(O)R²⁰ and -C(O)OR²⁰, wherein R²⁰ is selected from the group consisting of C₁-C₁₅ alkyl, C₂-C₁₅ alkenyl, C₂-C₁₅ alkynyl, C₇-C₂₀ aralkyl, C₇-C₂₀ alkaryl and C₅-C₂₀ aryl, and wherein R²¹ is selected from the group consisting of hydrogen, protecting group, C₁-C₁₅ alkyl, C₇-C₂₀ aralkyl and C₇-C₂₀ alkaryl; or any pharmaceutically acceptable salts, solvates and prodrugs thereof.

Compounds of the present invention have a stereocentre at the phosphorus, an anomeric carbon (C1) in the monosaccharide and they may also have asymmetric centers located on different moieties. All stereoisomers are contemplated, either in admixture or in pure or substantially pure form, including among them α/β anomers (at C1) and R/S at the phosphorus.

Preferred compounds are those wherein:
R² is selected from the group consisting of C₁-C₆ alkyl, C₇-C₁₂ aralkyl, C₇-C₁₂ alkaryl and C₅-C₁₂ aryl, wherein each of said aralkyl, alkaryl or aryl is optionally substituted with one or more substituents independently selected from the group consisting of halogen, C₁-C₆ alkyl, hydroxy, C₁-C₆ alcoxy, amino and C₁-C₆ alcanoyloxyl;
R³ is selected from hydrogen and C₁-C₆ alkyl; or R³ and R⁴ together are (CH₂)₃; or R³ and R⁵ together are (CH₂)₃, wherein the group (CH₂)₃ is optionally substituted with one or more substituents independently selected from the group consisting of C₁-C₆ alkyl, hydroxy, C₁-C₆ alcoxy, amino, carboxyl and esters thereof, alcanoyloxyl and halogen;
R⁴ and R⁵ are independently selected from the group consisting of hydrogen, C₁-C₆ alkyl and C₇-C₁₂ aralkyl, wherein each of said alkyl or aralkyl is optionally substituted with a substituent selected from the group consisting of hydroxy, C₁-C₆ alcoxy, thiol, C₁-C₆ alkylthio, carboxyl and esters thereof, amido, carbamoyl, imino, H₂NC(=NH)NH-, amino, aminoalkyl and C₁-C₆ alkylamino; or R⁴ and R³ together are (CH₂)₃; or R⁵ and R³ together are (CH₂)₃, wherein the group (CH₂)₃ is optionally substituted with one or more substituents independently selected from the group consisting of C₁-C₆ alkyl, hydroxy, C₁-C₆ alcoxy, amino, carboxyl and esters thereof, alcanoyloxyl and halogen;
R⁶ is selected from the group consisting of hydrogen, C₁-C₆ alkyl, C₂-C₆ alkenyl, C₃-C₇ cycloalkyl, C₇-C₁₂ aralkyl, C₇-C₁₂ alkaryl and C₅-C₁₂ aryl, wherein each of said alkyl, alkenyl, cycloalkyl, aralkyl, alkaryl or aryl is optionally substituted with one or more substituents independently selected from the group consisting of halogen, hydroxy, C₁-C₆ alcoxy, thiol and amino;
R⁷, R⁸, R⁹, R¹⁰, R¹², R¹³, R¹⁴ and R¹⁵ are independently selected from the group consisting of hydrogen, -OR¹⁷, -SR¹⁸, -NHR¹⁹ and C 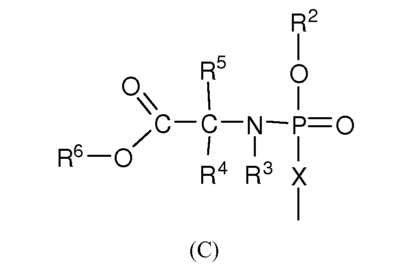
wherein X, R², R³, R⁴, R⁵ and R⁶ are as hereinbefore defined for formula (I), and wherein R¹⁷, R¹⁸ and R¹⁹ are independently selected from the group consisting of hydrogen, C₁-C₆ alkyl, C₇-C₁₂ aralkyl, C₇-C₁₂ alkaryl, C₅-C₁₂ aryl, -C(O)R²⁰ and -C(O)OR²⁰, wherein R²⁰ is selected from the group consisting of C₁-C₁₂ alkyl, C₂-C₁₂ alkenyl, C₇-C₁₂ aralkyl, C₇-C₁₂ alkaryl and C₅-C₁₂ aryl;
R¹¹ and R¹⁶ are independently selected from the group consisting of -CH₃, -CH₂-halogen, -CH₂OR¹⁷, -CH₂SR¹⁸, -CH₂NHR¹⁹, -CH₂NR¹⁹₂ and -C(O)OR²¹, wherein R¹⁷, R¹⁸ and R¹⁹ are independently selected from the group consisting of hydrogen, C₁-C₆ alkyl, C₇-C₁₂ aralkyl,
C₇-C₁₂ alkaryl, C₅-C₁₂ aryl, -C(O)R²⁰ and -C(O)OR²⁰, wherein R²⁰ is selected from the group consisting of C₁-C₁₂ alkyl, C₂-C₁₂ alkenyl, C₇-C₁₂ aralkyl, C₇-C₁₂ alkaryl and C₅-C₁₂ aryl, and wherein R²¹ is selected from the group consisting of hydrogen, C₁-C₁₀ alkyl, C₇-C₁₂ aralkyl and C₇-C₁₂ alkaryl.

Other preferred compounds are those wherein R² is selected from the group consisting of methyl, ethyl, n-propyl, isopropyl, phenyl, naphthyl, pyridyl and benzyl, wherein each of said phenyl, naphthyl, pyridyl or benzyl is optionally substituted with a substituent selected from the group consisting of halogen, C₁-C₆ alkyl, hydroxy and C₁-C₆ alcoxy.

Other preferred compounds are those wherein R³ is selected from hydrogen and methyl; or R³ and R⁴ together are (CH₂)₃; or R³ and R⁵ together are (CH₂)₃, wherein the group (CH₂)₃ is optionally substituted with one or more substituents independently selected from the group consisting of C₁-C₆ alkyl, hydroxy, C₁-C₆ alcoxy, amino, carboxyl and esters thereof, alcanoyloxyl and halogen.

Other preferred compounds are those wherein R⁴ and R⁵ are independently selected from the group consisting of hydrogen, methyl, ethyl, n-propyl, isopropyl, butyl, isobutyl, 1-methylpropyl, hydroxymethyl, 1-hydroxyethyl, mercaptomethyl, methyltioethyl, aminobutyl, benzyl, hydroxybenzyl, indolylmethyl, imidazolylmethyl, carboxymethyl, carboxyethyl, H₂NC(=NH)NH-(CH₂)₃-, carbamoylmethyl and carbamoylethyl; or R⁴ and R³ together are (CH₂)₃; or R⁵ and R³ together are (CH₂)₃, wherein the group (CH₂)₃ is optionally substituted with one or more substituents independently selected from the group consisting of C₁-C₆ alkyl, hydroxy, C₁-C₆ alcoxy, amino, carboxyl and esters thereof, alcanoyloxyl and halogen.

Preferably, one of both of R⁴ and R⁵ corresponds to the side chain of any α-amino acid.

Especially preferred compounds are those wherein R³ and R⁴ or R⁵, together with the nitrogen atom and carbon atom to which they are attached form the chain of the α-amino acid proline.

Other preferred compounds are those wherein R⁶ is selected from the group consisting of hydrogen, methyl, ethyl, n-propyl, isopropyl, n-butyl, 2-butyl, *tert*-butyl, n-pentyl, 3-pentyl, 2,2-dimethylpropyl, cyclohexyl, vinyl, propenyl, benzyl, phenylethyl, phenylpropyl and phenyl.

Especially preferred compounds are those wherein R³ and R⁴ or R⁵, together are (CH₂)₃.

Especially preferred compounds are those wherein X is -O-.

Preferably, R¹ is a monosaccharide selected from the group consisting of glucosamine, *N*-acetylglucosamine, glucose, mannosamine, *N*-acetylmannosamine, mannose, galactosamine, *N*-acetylgalactosamine, galactose, muramic acid and N-acetylmuramic acid, iduronic acid and glucuronic acid.

In a particularly preferred embodiment of the present invention, compound of formula (I) has the formula (Ia), 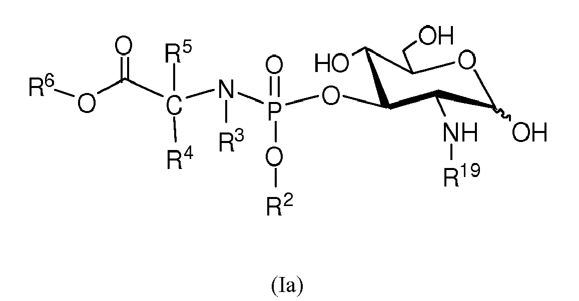 wherein R¹⁹ is selected from the group consisting of hydrogen, -C(O)R²⁰ and -C(O)OR²⁰, wherein R²⁰ is selected from the group consisting of C₁-C₁₀ alkyl, C₂-C₁₀ alkenyl, C₇-C₁₂ aralkyl, C₇-C₁₂ alkaryl and C₅-C₁₂ aryl;
R², R³, R⁴, R⁵ and R⁶ are as hereinbefore defined.

Preferably, the compound of formula (Ia) has the formula (Ib), 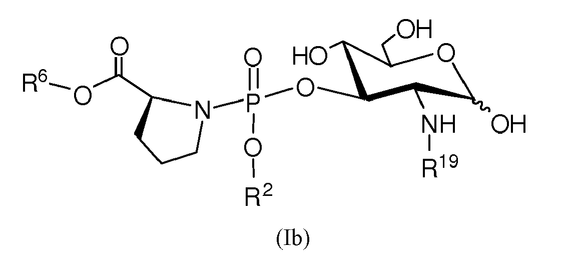 wherein R² is selected from the group consisting of methyl, ethyl, phenyl, naphthyl, pyridyl, benzyl, 4-metoxyphenyl and 4-chlorophenyl;
R⁶ is selected from the group consisting of hydrogen, methyl, ethyl, n-propyl, isopropyl, n-butyl, 2-butyl, *tert*-butyl, n-pentyl, 3-pentyl, 2,2-dimethylpropyl, cyclohexyl, vinyl, benzyl, phenylethyl and phenyl;
R¹⁹ is selected from the group consisting of hydrogen, -C(O)CH₃, -C(O)OCH₂Ph and -C(O)CH₂CH=CH₂.

In another particularly preferred embodiment of the present invention, compound of formula (I) has the formula (Ic), 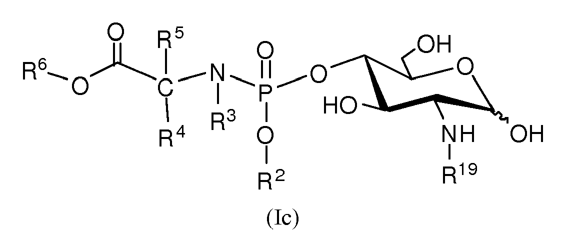 wherein R¹⁹ is selected from the group consisting of hydrogen, -C(O)R²⁰ and -C(O)OR²⁰, wherein R²⁰ is selected from the group consisting of C₁-C₁₀ alkyl, C₂-C₁₀ alkenyl, C₇-C₁₂ aralkyl, C₇-C₁₂ alkaryl and C₅-C₁₂ aryl;
R², R³, R⁴, R⁵ and R⁶ are as hereinbefore defined.

Preferably, the compound of formula (Ic) has the formula (Id), 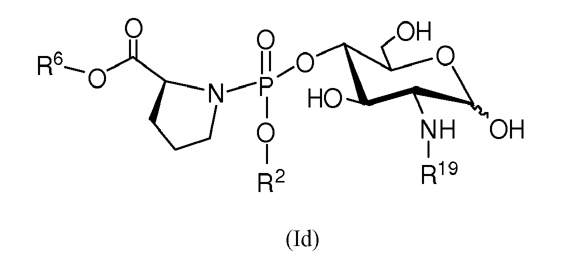 wherein R² is selected from the group consisting of methyl, ethyl, phenyl, naphthyl, pyridyl, benzyl, 4-metoxyphenyl and 4-chlorophenyl;
R⁶ is selected from the group consisting of hydrogen, methyl, ethyl, n-propyl, isopropyl, n-butyl, 2-butyl, *tert*-butyl, n-pentyl, 3-pentyl, 2,2-dimethylpropyl, cyclohexyl, vinyl, benzyl, phenylethyl and phenyl;
R¹⁹ is selected from the group consisting of hydrogen, -C(O)CH₃, -C(O)OCH₂Ph and -C(O)CH₂CH=CH₂.

Also, especially preferred are the compounds wherein
R² is selected from the group consisting of methyl, ethyl, phenyl, naphthyl, pyridyl, benzyl, 4-metoxyphenyl and 4-chlorophenyl;
R³ is selected from hydrogen and methyl;
R⁴ and R⁵ are independently selected from the group consisting of hydrogen, methyl, ethyl, n-propyl, isopropyl, isobutyl, 1-methylpropyl, hydroxymethyl, 1-hydroxyethyl, mercaptomethyl, methyltioethyl, aminobutyl, benzyl, hydroxybenzyl, indolylmethyl, imidazolylmethyl, carboxymethyl, carboxyethyl, H₂NC(=NH)NH-(CH₂)₃-, carbamoylmethyl and carbamoylethyl;
R⁶ is selected from the group consisting of hydrogen, methyl, ethyl, n-propyl, isopropyl, n-butyl, 2-butyl, *tert*-butyl, n-pentyl, 3-pentyl, 2,2-dimethylpropyl, cyclohexyl, vinyl, benzyl, phenylethyl and phenyl, and
R¹⁹ is selected from the group consisting of hydrogen, -C(O)CH₃, -C(O)OCH₂Ph and -C(O)CH₂CH=CH₂.

According to a preferred embodiment, the compound of formula (I) is selected from the following compounds:
2-acetamido-2-deoxy-3-*O*-[4-methoxyphenyl(benzyloxy-L-alanyl)]phosphoramidate-D-glucopyranose;
2-acetamido-2-deoxy-4-*O*-[4-methoxyphenyl(benzyloxy-L-alanyl)]phosphoramidate-D-glucopyranose;
benzyl 2-acetamido-2-deoxy-3-*O*-[4-methoxyphenyl(methoxy-L-alanyl)]phosphoramidate-D-glucopyranoside;
benzyl 2-acetamido-2-deoxy-4-*O*-[4-methoxyphenyl(methoxy-L-alanyl)]phosphoramidate-D-glucopyranoside;
methyl 2-acetamido-2-deoxy-3-*O*-[4-methoxyphenyl(benzyloxy-L-alanyl)]phosphoramidate-D-glucopyranoside;
methyl 2-benzyloxycarbonylamino-2-deoxy-3-*O*-[4-methoxyphenyl(ethoxy-L-alanyl)]phosphoramidate-D-glucopyranoside;
2-acetamido-2-deoxy-3-*O*-[4-methoxyphenyl(benzyloxy-dimethylglycinyl)]phosphoramidate-D-glucopyranose;
2-acetamido-2-deoxy-4-*O*-[4-methoxyphenyl(benzyloxy-dimethylglycinyl)]phosphoramidate-D-glucopyranose;
2-acetamido-2-deoxy-3-*O*-[4-methoxyphenyl(benzyloxy-L-isoleucinyl)]phosphoramidate-D-glucopyranose;
2-acetamido-2-deoxy-4-*O*-[4-methoxyphenyl(benzyloxy-L-isoleucinyl)]phosphoramidate-D-glucopyranose;
2-acetamido-2-deoxy-3-*O*-[4-methoxyphenyl(benzyloxy-L-leucinyl)]phosphoramidate-D-glucopyranose;
2-acetamido-2-deoxy-4-*O*-[4-methoxyphenyl(benzoxy-L-leucinyl)]phosphoramidate-D-glucopyranose;
2-acetamido-2-deoxy-3-*O*-[4-methoxyphenyl(benzyloxy-L-glycinyl)]phosphoramidate-D-glucopyranose;
2-acetamido-2-deoxy-4-*O*-[4-methoxyphenyl(benzoxy-L-glycinyl)]phosphoramidate-D-glucopyranose;
2-acetamido-2-deoxy-3-*O*-[4-methoxyphenyl(benzyloxy-L-methionyl)]phosphoramidate-D-glucopyranose;
2-acetamido-2-deoxy-4-*O*-[4-methoxyphenyl(benzoxy-L-methionyl)]phosphoramidate-D-glucopyranose;
2-deoxy-2-(pent-4-enoylamino)-3-*O*-[4-methoxyphenyl(benzyloxy-L-alanyl)]phosphoramidate-D-glucopyranose;
2-amino-2-deoxy-3-*O*-[4-methoxyphenyl(benzoxy-L-alanyl)]phosphoramidate-D-glucopyranose;
2-acetamido-2-deoxy-3-*O*-[4-methoxyphenyl(benzyloxy-D-alanyl)]phosphoramidate-D-glucopyranose;
2-acetamido-2-deoxy-4-*O*-[4-methoxyphenyl(benzyloxy-D-alanyl)]phosphoramidate-D-glucopyranose;
2-acetamido-2-deoxy-3-*O*-[1-naphtyl(benzyloxy-L-alanyl)]phosphoramidate-D-glucopyranose;
2-acetamido-2-deoxy-3-*O*-[4-methoxyphenyl(ethoxy-L-glycinyl)]phosphoramidate-D-glucopyranose;
2-acetamido-2-deoxy-4-*O*-[4-methoxyphenyl(ethoxy-L-glycinyl)]phosphoramidate-D-glucopyranose;
2-acetamido-2-deoxy-3-*O*-[methyl(benzyloxy-L-alanyl)]phosphoramidate-D-glucopyranose;
2-acetamido-2-deoxy-4-*O*-[methyl(benzyloxy-L-alanyl)]phosphoramidate-D-glucopyranose; benzyl 2-acetamido-2-deoxy-4,6-*O*-benzylidene-3-*O*-[4-methoxyphenyl(ethoxy-L-norvalinyl)]phosphoramidate-D-glucopyranoside;
2-acetamido-2-deoxy-3-*O*-[4-methoxyphenyl(ethoxy-L-norvalinyl)]phosphoramidate-D-glucopyranose;
2-acetamido-2-deoxy-3-*O*-[4-methoxyphenyl(benzyloxy-L-phenylalanyl)]phosphoramidate-D-glucopyranose;
2-acetamido-2-deoxy-3-*O*-[4-methoxyphenyl(benzyloxy-L-prolinyl)]phosphoramidate-D-glucopyranose;
2-acetamido-2-deoxy-4-*O*-[4-methoxyphenyl(benzyloxy-L-prolinyl)]phosphoramidate-D-glucopyranose;
methyl 2-acetamido-2-deoxy-3-*O*-[4-methoxyphenyl(benzyloxy-L-prolinyl)]phosphoramidate-D-glucopyranoside;
2-acetamido-2-deoxy-3-*O*-[4-methoxyphenyl(ethoxy-L-prolinyl)]phosphoramidate-D-glucopyranose;
2-acetamido-2-deoxy-4-*O*-[4-methoxyphenyl(ethoxy-L-prolinyl)]phosphoramidate-D-glucopyranose;
2-acetamido-2-deoxy-3-*O*-[4-methoxyphenyl(n-butoxy-L-prolinyl)]phosphoramidate-D-glucopyranose;
2-acetamido-2-deoxy-4-*O*-[4-methoxyphenyl(n-butoxy-L-prolinyl)]phosphoramidate-D-glucopyranose;
2-acetamido-2-deoxy-3-*O*-[4-methoxyphenyl(isopropoxy-L-prolinyl)]phosphoramidate-D-glucopyranose;
2-acetamido-2-deoxy-4-*O*-[4-methoxyphenyl(isopropoxy-L-prolinyl)]phosphoramidate-D-glucopyranose;
benzyl 2-acetamido-2-deoxy-4,6-*O*-benzylidene-3-*O*-[4-methoxyphenyl(tert-butoxy-L-prolinyl)]phosphoramidate-D-glucopyranoside;
2-acetamido-2-deoxy-3-*O*-[4-methoxyphenyl(tert butoxy-L-prolinyl)]phosphoramidate-D-glucopyranose;
2-acetamido-2-deoxy-3-*O*-[4-methoxyphenyl(2-butoxy-L-prolinyl)]phosphoramidate-D-glucopyranose;
2-acetamido-2-deoxy-4-*O*-[4-methoxyphenyl-(2-butoxy-L-prolinyl)]phosphoramidate-D-glucopyranose;
2-acetamido-2-deoxy-3-*O*-[4-methoxyphenyl(cyclohexyloxy-L-prolinyl)]phosphoramidate-D-glucopyranose;
2-acetamido-2-deoxy-4-*O*-[4-methoxyphenyl(cyclohexyloxy-L-prolinyl)]phosphoramidate-D-glucopyranose;
2-acetamido-2-deoxy-3-*O*-[4-methoxyphenyl(methoxy-L-prolinyl)]phosphoramidate-D-glucopyranose;
2-acetamido-2-deoxy-4-*O*-[4-methoxyphenyl(methoxy-L-prolinyl)]phosphoramidate-D-glucopyranose;
2-acetamido-2-deoxy-3-*O*-[4-methoxyphenyl(n-propoxy-L-prolinyl)]phosphoramidate-D-glucopyranose;
2-acetamido-2-deoxy-4-*O*-[4-methoxyphenyl(n-propoxy-L-prolinyl)]phosphoramidate-D-glucopyranose;
2-acetamido-2-deoxy-3-*O*-[4-methoxyphenyl(n-pentoxy-L-prolinyl)]phosphoramidate-D-glucopyranose;
2-acetamido-2-deoxy-4-*O*-[4-methoxyphenyl(n-pentoxy-L-prolinyl)]phosphoramidate-D-glucopyranose;
2-acetamido-2-deoxy-3-*O*-[4-methoxyphenyl(3-pentoxy-L-prolinyl)]phosphoramidate-D-glucopyranose;
2-acetamido-2-deoxy-4-*O*-[4-methoxyphenyl(3-pentoxy-L-prolinyl)]phosphoramidate-D-glucopyranose;
2-acetamido-2-deoxy-3-*O*-[4-methoxyphenyl(neopentoxy-L-prolinyl)]phosphoramidate-D-glucopyranose;
2-acetamido-2-deoxy-4-*O*-[4-methoxyphenyl(neopentoxy-L-prolinyl)]phosphoramidate-D-glucopyranose;
2-acetamido-2-deoxy-3-*O*-[phenyl(benzyloxy-L-prolinyl)]phosphoramidate-D-glucopyranose;
2-acetamido-2-deoxy-4-*O*-[4-phenyl(benzyloxy-L-prolinyl)]phosphoramidate-D-glucopyranose;
2-acetamido-2-deoxy-3-*O*-[phenyl(ethoxy-L-prolinyl)]phosphoramidate-D-glucopyranose;
2-acetamido-2-deoxy-4-*O*-[phenyl(ethoxy-L-prolinyl)]phosphoramidate-D-glucopyranose;
2-acetamido-2-deoxy-3-*O*-[1-naphtyl(benzyloxy-L-prolinyl)]phosphoramidate-D-glucopyranose;
2-acetamido-2-deoxy-4-*O*-[1-naphtyl(benzyloxy-L-prolinyl)]phosphoramidate-D-glucopyranose;
2-acetamido-2-deoxy-3-*O*-[1-naphtyl(ethoxy-L-prolinyl)]phosphoramidate-D-glucopyranose;
2-acetamido-2-deoxy-4-*O*-[1-naphtyl(ethoxy-L-prolinyl)]phosphoramidate-D-glucopyranose;
2-acetamido-2-deoxy-3-*O*-[4-chlorophenyl(benzyloxy-L-prolinyl)]phosphoramidate-D-glucopyranose;
2-acetamido-2-deoxy-4-*O*-[4-chlorophenyl(benzoxy-L-prolinyl)]phosphoramidate-D-glucopyranose;
2-acetamido-2-deoxy-3-*O*-[4-chlorophenyl(ethoxy-L-prolinyl)]phosphoramidate-D-glucopyranose;
2-acetamido-2-deoxy-4-*O*-[4-chlorophenyl(ethoxy-L-prolinyl)]phosphoramidate-D-glucopyranose;
2-deoxy-2-(pent-4-enoylamino)-3-*O*-[4-methoxyphenyl(benzyloxy-L-prolinyl)]phosphoramidate-D-glucopyranose;
2-deoxy-2-(pent-4-enoylamino)-4-*O*-[4-methoxyphenyl(benzyloxy-L-prolinyl)]phosphoramidate-D-glucopyranoside;
2-amino-2-deoxy-3-*O*-[4-methoxyphenyl(benzyloxy-L-prolinyl)]phosphoramidate-D-glucopyranose;
2-amino-2-deoxy-4-*O*-[4-methoxyphenyl(benzyloxy-L-prolinyl)]phosphoramidate-D-glucopyranose;
2-deoxy-2-(pent-4-enoylamino)-3-*O*-[4-methoxyphenyl(ethoxy-L-prolinyl)]phosphoramidate-D-glucopyranose;
2-deoxy-2-(pent-4-enoylamino)-4-*O*-[4-methoxyphenyl(ethoxy-L-prolinyl)]phosphoramidate-D-glucopyranose;
2-acetamido-2-deoxy-3-*O*-[4-methoxyphenyl(benzyloxy-D-prolinyl)]phosphoramidate-D-glucopyranose;
2-acetamido-2-deoxy-4-*O*-[4-methoxyphenyl(benzoxy-D-prolinyl)]phosphoramidate-D-glucopyranose;
2-acetamido-2-deoxy-3-*O*-[4-methoxyphenyl(ethoxy-D-prolinyl)]phosphoramidate-D-glucopyranose;
2-acetamido-2-deoxy-4-*O*-[4-methoxyphenyl(ethoxy-D-prolinyl)]phosphoramidate-D-glucopyranose;
methyl 2-acetamido-2-deoxy-3-*O*-[4-methoxyphenyl(ethoxy-L-sarcosinyl)]phosphoramidate-D-glucopyranoside;
methyl 2-acetamido-2-deoxy-3-*O*-[4-methoxyphenyl(benzyloxy-L-valinyl)]phosphoramidate-D-glucopyranoside;
methyl 2-acetamido-2-deoxy-4-*O*-[4-methoxyphenyl(benzyloxy-L-valinyl)]phosphoramidate-D-glucopyranoside;
methyl 2-acetamido-2-deoxy-3-*O*-[4-methoxyphenyl(ethoxy-L-valinyl)]phosphoramidate-D-glucopyranoside;
2-acetamido-2-deoxy-3-*O*-[4-methoxyphenyl(ethoxy-L-sarcosinyl)]phosphoramidate-D-glucopyranose;
2-acetamido-2-deoxy-3-*O*-[4-methoxyphenyl(benzyloxy-L-valinyl)]phosphoramidate-D-glucopyranose;
2-acetamido-2-deoxy-4-*O*-[4-methoxyphenyl(benzyloxy-L-valinyl)]phosphoramidate-D-glucopyranose;
2-acetamido-2-deoxy-3-*O*-[4-methoxyphenyl(isopropoxy-L-valinyl)]phosphoramidate-D-glucopyranose;
2-acetamido-2-deoxy-4-*O*-[4-methoxyphenyl(isopropoxy-L-valinyl)]phosphoramidate-D-glucopyranose;
2-acetamido-2-deoxy-3-*O*-[4-methoxyphenyl(ethoxy-L-valinyl)]phosphoramidate-D-glucopyranose;
2-acetamido-2-deoxy-4-*O*-[4-methoxyphenyl(ethoxy-L-valinyl)]phosphoramidate-D-glucopyranose;
2-acetamido-2-deoxy-3-*O*-[4-methoxyphenyl(ethoxy-L-valinyl)]phosphoramidate-D-glucopyranose;
2-acetamido-2-deoxy-3-*O*-[4-methoxyphenyl(tert-butoxy-L-valinyl)]phosphoramidate-D-glucopyranose;
2-acetamido-2-deoxy-4-*O*-[4-methoxyphenyl(*tert*-butoxy-L-valinyl)]phosphoramidate-D-glucopyranose;
benzyl 2-acetamido-2-deoxy-4,6-*O*-benzylidene-3-O-[4-methoxyphenyl(methoxy-L-valinyl)]phosphoramidate-D-glucopyranoside;
2-acetamido-2-deoxy-3-*O*-[4-methoxyphenyl(methoxy-L-valinyl)]phosphoramidate-D-glucopyranose;
2-acetamido-2-deoxy-3-*O*-[4-methoxyphenyl(cyclohexyloxy-L-valinyl)]phosphoramidate-D-glucopyranose;
2-acetamido-2-deoxy-4-*O*-[4-methoxyphenyl(cyclohexyloxy-L-valinyl)]phosphoramidate-D-glucopyranose;
2-acetamido-2-deoxy-3-*O*-[4-methoxyphenyl(2-butoxy-L-valinyl)]phosphoramidate-D-glucopyranose;
2-acetamido-2-deoxy-4-*O*-[4-methoxyphenyl(2-butoxy-L-valinyl)]phosphoramidate-D-glucopyranose;
2-deoxy-2-(pent-4-enoylamino)-4-*O*-[4-methoxyphenyl(benzyloxy-L-valinyl)]phosphoramidate-*D*-glucopyranose;
2-deoxy-2-(pent-4-enoylamino)-3-*O*-[4-methoxyphenyl(benzyloxy-L-valinyl)]phosphoramidate-*D*-glucopyranose;
2-amino-2-deoxy-4-*O*-[4-methoxyphenyl(benzyloxy-L-valinyl)]phosphoramidate-*D-*glucopyranose;
2-amino-2-deoxy-3-*O*-[4-methoxyphenyl(benzyloxy-L-valinyl)]phosphoramidate-D-glucopyranose;
2-deoxy-2-(pent-4-enoylamino)-4-*O*-[phenyl(ethoxy-L-prolinyl)]phosphoramidate-D-glucopyranose;
2-deoxy-2-(pent-4-enoylamino)-3-*O*-[phenyl(ethoxy-L-prolinyl)]phosphoramidate-D-glucopyranose;
2-amino-2-deoxy-4-*O*-[phenyl(ethoxy-L-prolinyl)]phosphoramidate-D-glucopyranose;
2-amino-2-deoxy-3-*O*-[phenyl(ethoxy-L-prolinyl)]phosphoramidate-D-glucopyranose
and pharmaceutically acceptable derivatives thereof.

Preferred compounds of formula (Ia) include the compounds listed in Table 1 and preferred compounds of formula (Ic) include the compounds listed in Table 2. These tables are provided to enable those skilled in the art to more clearly understand the present invention. They should not be considered as limiting the scope of the invention, but merely as being illustrative and representative thereof.

**Table 1.**

| | | | | | | |
|---|---|---|---|---|---|---|
| 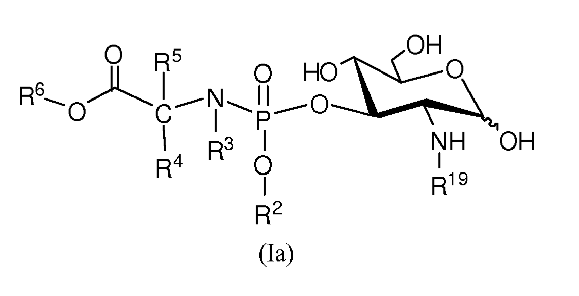 | | | | | | |

| **Compound** | **R²** | **R⁵** | **R³** | **R⁴** | **R⁶** | **R¹⁹** |
|---|---|---|---|---|---|---|
| 1 | 4-(MeO)Ph | H | -(CH₂)₃- | | PhCH₂ | Ac |
| 3 | 4-(MeO)Ph | H | H | CH₃ | PhCH₂ | Ac |
| 5 | 4-(MeO)Ph | CH₃ | H | CH₃ | PhCH₂ | Ac |
| 7 | 4-(MeO)Ph | H | H | 1-Methylpropyl | PhCH₂ | Ac |
| 9 | 4-(MeO)Ph | H | H | iPrCH₂ | PhCH₂ | Ac |
| 11 | 4-(MeO)Ph | H | H | H | PhCH₂ | Ac |
| 13 | 4-(MeO)Ph | H | H | CH₃S(CH₂)₂ | PhCH₂ | Ac |
| 15 | 4-(MeO)Ph | H | H | PhCH₂ | PhCH₂ | Ac |
| 16 | 4-(MeO)Ph | H | H | iPr | PhCH₂ | Ac |
| 18 | 4-(MeO)Ph | H | H | CH₃ | PhCH₂ | Pnt |
| 19 | 4-(MeO)Ph | H | H | CH₃ | PhCH₂ | H |
| 20 | 4-(MeO)Ph | CH₃ | H | H | PhCH₂ | Ac |
| 28 | Naph | H | H | CH₃ | PhCH₂ | Ac |
| 29 | 4-(MeO)Ph | H | H | H | Et | Ac |
| 31 | 4-(MeO)Ph | H | -(CH₂)₃- | | Et | Ac |
| 34 | 4-(MeO)Ph | H | H | iPr | iPr | Ac |
| 37 | 4-(MeO)Ph | H | CH₃ | H | Et | Ac |
| 38 | 4-(MeO)Ph | H | H | iPr | Et | Ac |
| 40 | 4-(MeO)Ph | H | -(CH₂)₃- | | nBu | Ac |
| 42 | 4-(MeO)Ph | H | | -(CH₂)₃- | iPr | Ac |
| 45 | 4-(MeO)Ph | H | H | nPr | Et | Ac |
| 47 | 4-(MeO)Ph | H | -(CH₂)₃- | | tBu | Ac |
| 48 | 4-(MeO)Ph | H | -(CH₂)₃- | | 2Bu | Ac |
| 50 | 4-(MeO)Ph | H | H | iPr | tBu | Ac |
| 53 | 4-(MeO)Ph | H | H | iPr | CH₃ | Ac |
| 54 | 4-(MeO)Ph | H | -(CH₂)₃- | | cHex | Ac |
| 56 | 4-(MeO)Ph | H | H | iPr | cHex | Ac |
| 58 | 4-(MeO)Ph | H | H | iPr | 2Bu | Ac |
| 61 | 4-(MeO)Ph | H | H | iPr | PhCH₂ | Pnt |
| 63 | 4-(MeO)Ph | H | H | iPr | PhCH₂ | H |
| 64 | 4-(MeO)Ph | H | -(CH₂)₃- | | CH₃ | Ac |
| 66 | 4-(MeO)Ph | H | -(CH₂)₃- | | nPr | Ac |
| 68 | 4-(MeO)Ph | H | -(CH₂)₃- | | nPen | Ac |
| 70 | 4-(MeO)Ph | H | -(CH₂)₃- | | 3Pen | Ac |
| 72 | 4-(MeO)Ph | H | -(CH₂)₃- | | tBuCH₂ | Ac |
| 74 | Ph | H | -(CH₂)₃- | | PhCH₂ | Ac |
| 76 | Ph | H | -(CH₂)₃- | | Et | Ac |
| 78 | Naph | H | -(CH₂)₃- | | PhCH₂ | Ac |
| 80 | Naph | H | -(CH₂)₃- | | Et | Ac |
| 82 | 4-(Cl)Ph | H | -(CH₂)₃- | | PhCH₂ | Ac |
| 83 | 4-(Cl)Ph | H | -(CH₂)₃- | | Et | Ac |
| 84 | 4-(MeO)Ph | H | -(CH₂)₃- | | PhCH₂ | Pnt |
| 86 | 4-(MeO)Ph | H | -(CH₂)₃- | | PhCH₂ | H |
| 87 | 4-(MeO)Ph | H | -(CH₂)₃- | | Et | Pnt |
| 88 | CH₃ | H | H | CH₃ | PhCH₂ | Ac |
| 90 | 4-(MeO)Ph | -(CH₂)₃- | | H | PhCH₂ | Ac |
| 91 | 4-(MeO)Ph | -(CH₂)₃- | | H | Et | Ac |

**Table 2.**

| | | | | | | |
|---|---|---|---|---|---|---|
| 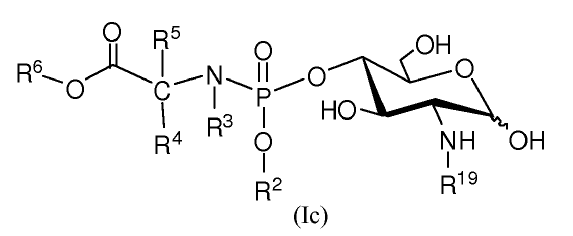 | | | | | | |

| **Compound** | **R²** | **R⁵** | **R³** | **R⁴** | **R⁶** | **R¹⁹** |
|---|---|---|---|---|---|---|
| 2 | 4-(MeO)Ph | H | -(CH₂)₃- | | PhCH₂ | Ac |
| 4 | 4-(MeO)Ph | H | H | CH₃ | PhCH₂ | Ac |
| 6 | 4-(MeO)Ph | CH₃ | H | CH₃ | PhCH₂ | Ac |
| 8 | 4-(MeO)Ph | H | H | 1-Methylpropyl | PhCH₂ | Ac |
| 10 | 4-(MeO)Ph | H | H | iPrCH₂ | PhCH₂ | Ac |
| 12 | 4-(MeO)Ph | H | H | H | PhCH₂ | Ac |
| 14 | 4-(MeO)Ph | H | H | CH₃S(CH₂)₂ | PhCH₂ | Ac |
| 17 | 4-(MeO)Ph | H | H | iPr | PhCH₂ | Ac |
| 21 | 4-(MeO)Ph | CH₃ | H | H | PhCH₂ | Ac |
| 30 | 4-(MeO)Ph | H | H | H | Et | Ac |
| 32 | 4-(MeO)Ph | H | -(CH₂)₃- | | Et | Ac |
| 35 | 4-(MeO)Ph | H | H | iPr | iPr | Ac |
| 39 | 4-(MeO)Ph | H | H | iPr | Et | Ac |
| 41 | 4-(MeO)Ph | H | -(CH₂)₃- | | nBu | Ac |
| 43 | 4-(MeO)Ph | H | -(CH₂)₃- | | iPr | Ac |
| 49 | 4-(MeO)Ph | H | -(CH₂)₃- | | 2Bu | Ac |
| 51 | 4-(MeO)Ph | H | H | iPr | tBu | Ac |
| 55 | 4-(MeO)Ph | H | -(CH₂)₃- | | cHex | Ac |
| 57 | 4-(MeO)Ph | H | H | iPr | cHex | Ac |
| 59 | 4-(MeO)Ph | H | H | iPr | 2Bu | Ac |
| 60 | 4-(MeO)Ph | H | H | iPr | PhCH₂ | Pnt |
| 62 | 4-(MeO)Ph | H | H | iPr | PhCH₂ | H |
| 65 | 4-(MeO)Ph | H | -(CH₂)₃- | | CH₃ | Ac |
| 67 | 4-(MeO)Ph | H | -(CH₂)₃- | | nPr | Ac |
| 69 | 4-(MeO)Ph | H | -(CH₂)₃- | | nPen | Ac |
| 71 | 4-(MeO)Ph | H | -(CH₂)₃- | | 3Pen | Ac |
| 73 | 4-(MeO)Ph | H | -(CH₂)₃- | | tBuCH₂ | Ac |
| 75 | Ph | H | -(CH₂)₃- | | PhCH₂ | Ac |
| 77 | Ph | H | -(CH₂)₃- | | Et | Ac |
| 79 | Naph | H | -(CH₂)₃- | | PhCH₂ | Ac |
| 81 | Naph | H | -(CH₂)₃- | | Et | Ac |
| 85 | 4-(MeO)Ph | H | -(CH₂)₃- | | PhCH₂ | Pnt |
| 89 | CH₃ | H | H | CH₃ | PhCH₂ | Ac |
| 92 | 4-(MeO)Ph | -(CH₂)₃- | | H | Et | Ac |
| 93 | Ph | H | -(CH₂)₃- | | Et | Pnt |
| 94 | Ph | H | -(CH₂)₃- | | Et | H |

PhCH₂: Benzyl; iPr: Isopropyl; Pnt: n-Pent-4-enoyl; Naph: Naphtyl; nBu: n-Butyl; nPr: n-Propyl; 2Bu: 2-Butyl; cHex: Cyclohexyl; 3Pen: 3-Pentyl

The phrase "a" or "an" entity as used herein refers to one or more of that entity; for example, a compound refers to one or more compounds or a least one compound. As such, the terms "a" (or "an"), "one or more", and "at least one" can be used interchangeably herein.

The term "optional" or "optionally" as used herein means that a subsequently described event or circumstance may, but need not, occur, and that the description includes instances where the event or circumstance occurs and instances in which it does not. For example, "optionally substituted" means that the moiety may be hydrogen or a substituent.

As used herein, the term "alkyl" refers to a straight or branched saturated monovalent hydrocarbon radical.

As used herein, the term "alkenyl" refers to a straight or branched unsaturated monovalent hydrocarbon radical, having one or two olefinic double bonds.

As used herein, the term "alkynyl" refers to a straight or branched unsaturated monovalent hydrocarbon radical, having one or where possible two triple bonds.

As used herein, the term "cicloalkyl" refers to a cyclic saturated monovalent hydrocarbon radical.

As used herein, the terms "cicloalkenyl" and "cicloalkynyl" refer to cyclic unsaturated monovalent hydrocarbon radicals. A "cicloalkenyl" is characterized by a carbon-carbon double bond and a "cicloalkynyl" is characterized by a carbon-carbon triple bond.

As used herein, the term "aryl" refers to a monovalent unsaturated aromatic carbocyclic radical having one or two rings, or a monovalent unsaturated aromatic heterocyclic radical having one or two rings. Where the aryl group comprises more than one ring, the rings may be fused or bicyclic.

As used herein, the term "alkaryl" refers to an aryl radical with an alkyl substituent.

As used herein, the term "aralkyl," refers to an alkyl radical with an aryl substituent. Examples of aralkyl radicals are benzyl, phenylethyl.

As used herein, the term "halogen" means fluorine, chlorine, bromine or iodine.

As used herein, the term "alkoxy" refers to an -O-alkyl group, wherein alkyl is a defined above.

As used herein, the term "alkoxyalkyl" refers to an alkyl radical with an alkoxy substituent.

As used herein, the term "alkylamino" refers to an -NH-alkyl group.

As used herein, the term "aminoalkyl" refers to an alkyl radical with an amino substituent.

As used herein, the term "alkylthio" refers to an -S-alkyl group.

As used herein, the term "hydroxyalkylamino" refers to an -NH-alkylene-OH group,

As used herein, the term "alcanoyloxyl" refers to an -O-C(=O)-alkyl group.

As used herein, the term "carbamoyl" refers to an -C(=O)-NH₂ group.

As used herein, the term "protecting group" refers to any group which when bound to one or more hydroxyl groups, thiols groups, amino groups or carboxyl groups prevents reactions from occurring at these groups and which protecting group can be removed by conventional chemical or enzymatic steps to re-establish the hydroxyl, thiol, carboxyl or amino group.

As used herein, the term "α-amino acid" refers preferably to naturally occurring α-amino acids, as well as to optical isomers (enantiomers and diastereomers), synthetic analogs and derivatives thereof. Examples of α-amino acid are proline, alanine, arginine, asparagines, aspartic acid, cysteine, cystine, glycine, glutamic acid, glutamine, histidine, isoleucine, leucine, lysine, methionine, phenylalanine, serine, threonine, tryptophan, tyrosine, valine, ornithine, norvaline, sarcosine.

The term "pharmaceutically acceptable salts, solvates or prodrugs" refers to any pharmaceutically acceptable salt, solvate, or any other compound which, upon administration to the recipient is capable of providing (directly or indirectly) a compound as described herein. However, it will be appreciated that non-pharmaceutically acceptable salts also fall within the scope of the invention since those may be useful in the preparation of pharmaceutically acceptable salts. The preparations of salts, prodrugs and derivatives can be carried out by methods known in the art.

For instance, pharmaceutically acceptable salts of the novel compounds of formula (I) are synthesized from the parent compound which contains a basic or acidic moiety by conventional chemical methods. Generally, such salts are, for example, prepared by reacting the free acid or base forms of these compounds with a stoichiometric amount of the appropriate base or acid in water or in an organic solvent or in a mixture of the two. Examples of the acid addition salts include mineral acid addition salts such as, for example, hydrochloride, hydrobromide and sulphate, and organic acid addition salts such as, for example, acetate, citrate and malate. Examples of the alkali addition salts include inorganic salts such as, for example, sodium, potassium and calcium salts and organic alkali salts such as, for example, ethanolamine, triethanolamine and basic aminoacids salts.

Particularly favored derivatives are those that increase the bioavailability of the compounds of the present invention when such novel compounds are administered to a patient.

The novel compounds of formula (I) may be in crystalline form either as free compounds or as solvates (e.g. hydrates); both forms are within the scope of the present invention. Methods of solvation are generally known within the art. Preferably, the solvate is a hydrate.

In another aspect, the present invention is referred to a compound of formula (I) as hereinbefore defined, or a pharmaceutically acceptable salt, solvate and prodrug thereof, for use as a medicament.

In another aspect, the present invention is referred to a pharmaceutical composition comprising at least one compound of formula (I) as hereinbefore defined, or a pharmaceutically acceptable salt, solvate or prodrug thereof, and at least one pharmaceutically acceptable excipient.

In another aspect, the present invention is referred to a compound of formula (I) as hereinbefore defined, or a pharmaceutically acceptable salt, solvate or prodrug thereof, for use in the treatment, prevention or prophylaxis of a degenerative joint disease, preferably osteoarthritis, and autoimmune disease, preferably rheumatoid arthritis, a virus infection, cancer, inflammation, pain, a tendon or ligament disease, disorder or injury, or any musculoskeletal disease, preferably in a mammal.

The present invention also relates to a compound of formula (I) as hereinbefore defined, or a pharmaceutically acceptable salt, solvate or prodrug thereof, for use as chondroprotector.

The present invention also relates to the use of a compound of formula (I) as hereinbefore defined, or a pharmaceutically acceptable salt, solvate or prodrug thereof, in the preparation of a medicament for the treatment, prevention or prophylaxis of a degenerative joint disease, preferably osteoarthritis, and autoimmune disease, preferably rheumatoid arthritis, a virus infection, cancer, inflammation, pain, a tendon or ligament disease, disorder or injury, or any musculoskeletal disease, preferably in a mammal.

The present invention also relates to a method of treating or preventing a degenerative joint disease, preferably osteoarthritis, and autoimmune disease, preferably rheumatoid arthritis, a virus infection, cancer, inflammation, pain, a tendon or ligament disease, disorder or injury, or any musculoskeletal disease, which method comprises administering to a patient in need of such a treatment a therapeutically effective amount of at least one compound of formula (I) as hereinbefore defined, or a pharmaceutically acceptable salt, solvate or prodrug thereof.

The compounds of the present invention are formulated in suitable pharmaceutical compositions, using conventional techniques and excipients or carriers, such as those described in Remington: The Science and Practice of Pharmacy 2000, edited by Lippincott Williams and Wilkins, 20th edition, Philadelphia*.*

The administration of the pharmaceutical compositions of the present invention can be carried out by oral, parenteral (including intravenous, intramuscular, intraperitoneal, intra-articular, subcutaneous), topical (including buccal, sublingual and vaginal), transdermal, airway (aerosol) or rectal routes.

The pharmaceutical compositions of the invention include a therapeutically effective amount of active component, said amount depending on many factors, such as for example the physical condition of the patient, age, sex, particular compound, route of administration, severity of the symptoms and on other factors that are well known in the art. Furthermore, it will be understood that said dosage of active component can be administered in single or multiple dose units to provide the desired therapeutic effects.

The pharmaceutical preparations of the invention will generally be in solid, liquid form or as a gel. The pharmaceutical preparations in solid form that can be prepared according to the present invention include powders, tablets, pills, dispersible granules, capsules (including hard and soft gelatin capsules), cachets and suppositories. The pharmaceutical preparations in liquid form include solutions, suspensions, emulsions, syrups and elixirs. The preparations in solid form which are to be converted, immediately before being used, into preparations in liquid form are also contemplated.

The compounds of the invention may also be presented as liposome formulations.

In another aspect, the present invention is referred to a process for the preparation of a compound of formula (I) as hereinbefore defined, comprising the step of reacting a compound of formula (II), 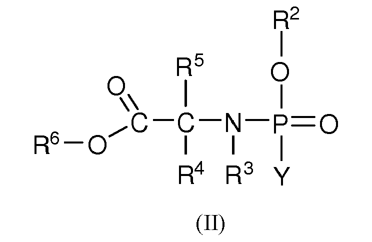 wherein
R², R³, R⁴, R⁵ and R⁶ are independently selected and are as hereinbefore defined for formula (I); and
Y is a leaving group;
with a compound of formula (III) or (IV), 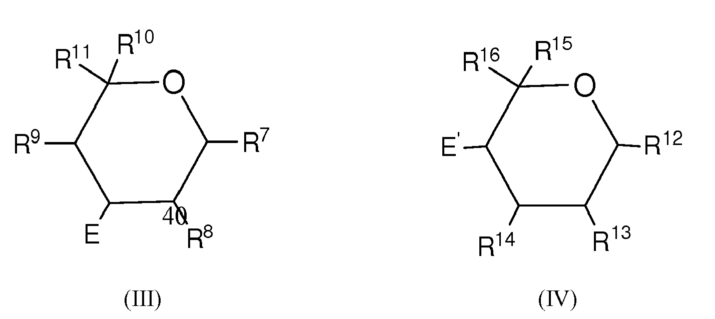 wherein
E and E' are independently selected from groups capable of displacing a group Y from a compound of formula (II); and
R⁷-R¹⁶ are independently selected and are as hereinbefore defined for formula (I).

The term "leaving group generally refers to groups readily displaceable by a nucleophile. Such leaving groups are well known in the art. Examples of such leaving groups include, but are not limited to, triflates, tosylates, mesylates and halides.
Y is preferably selected from Cl, Br and I, preferably Cl.
When Y is Cl, formula (II) is represented by formula (V) as shown below.

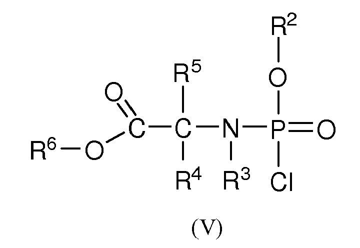

Preferably E and E' are both OH. In this embodiment, formula (III) is represented by formula (VI) and formula (IV) is represented by formula (VII) as shown below. 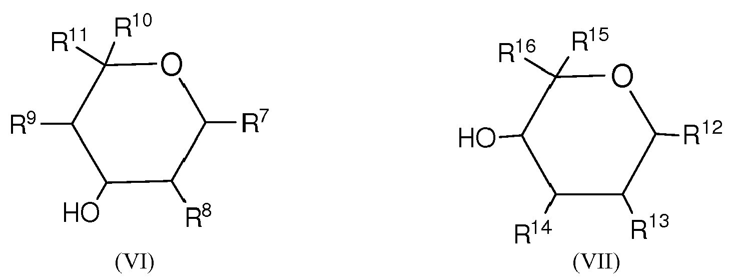 wherein R⁷-R¹⁶ are independently selected and are as hereinbefore defined for formula (I).
The process is preferably carried out in the presence of a solvent, preferably a dry organic solvent. Preferred solvents include diethylether, tetrahydrofuran, diphenylether, anisole, dimethoxybenzene, pyridine, trimethylamine and triethylamine.
The reaction is preferably carried out in the presence of a base, preferably an organic base. For example, N-methylimidazole is preferred.
The process is preferably carried out below 40 °C. More preferably, the reaction is carried out below 0 °C, more preferably below -20°C, preferably below -30 °C. For example, -40 °C is preferred.
The monosaccharide compound of formula (VI) or (VII) may be a commercial compound or may be prepared following processes well known in the art.
The phosphochloridate intermediate, compound of formula (V), may be prepared, according to Example 4 of the present invention, C. McGuigan et al. J. Med. Chem. 51, 5807-5812 (2008) or WO 2007/020193, starting from phosphorus oxychloride to afford phosphorodichloridate derivative of formula (VIII), which is subsequently treated with an acid addition salt of an α-amino acid ester of formula (IX) in the presence of, for example, triethylamine to afford a compound of formula (V), as shown below in Scheme 1.
Acid addition salt of α-amino acid ester of formula (IX) may be prepared starting from the appropriate amino acid, the appropriate alcohol and, for example, thionyl chloride or *para-*toluene sulphonic acid, following processes well known in the art (A. Isidro-Llobet et al.,Chem. Rev 109, 2455-2504(2009))
Phosphochloridate is the term used to refer to the phosphorylating reagent, which is coupled to the monosaccharide to form the phosphoramidate compound.

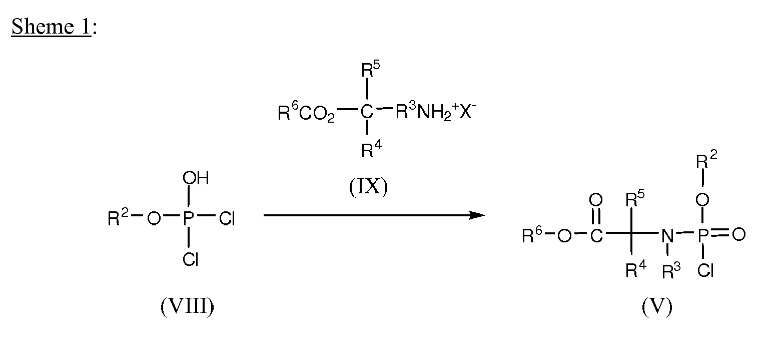

Compounds of formula (I) wherein R¹ is A can also be obtained starting from a pyranose monosaccharide protected in positions 4 and 6 with a protective group.
To illustrate this synthetic sequence, Scheme 2 shows how a protected compound of formula (Ie) can be obtained starting from a monosaccharide of formula (X) and the appropriate phosphorochloridate of formula (V), in the presence of a base, preferably *t*-BuMgCl, and a solvent, preferably tetrahydrofuran.

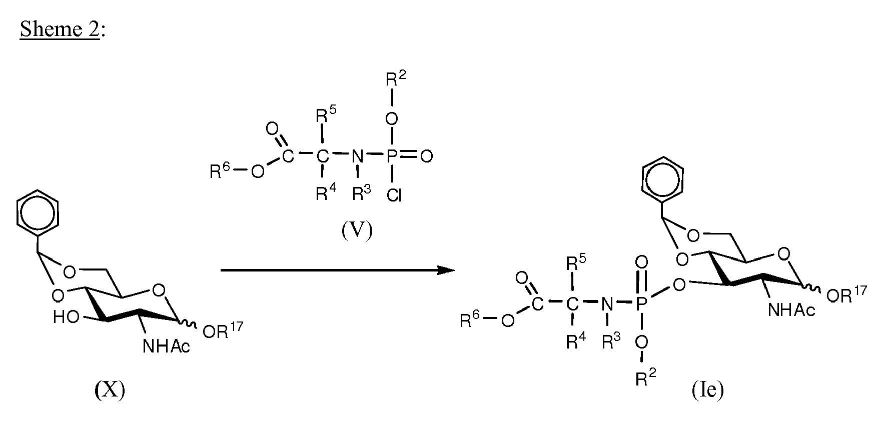

If desired, the benzylidene group of a compound of formula (Ie) can be removed, for example, by iodination (I₂, methanol) or by hydrogenolysis under pressure (H₂, 10% Pd/C, methanol). Compounds of formula (I) wherein R¹⁹ is hydrogen (compounds with a free amino group of the monosaccharide) can also be obtained by protecting the amino group using, for example, the pent-4-enoyl group which can be then removed, for example, by iodination (I₂, tetrahydrofuran).

According to the present invention, it has been found that the compounds of the present invention have advantages, such as: (i) compounds of the invention (phosphoramidates in position 3- or 4- of a glucosamine ring are more effective than phosphoramidates in position 6- of a glucosamine ring (ii) compounds of the invention are more effective than glucosamine hydrochloride.

### Detailed description of the preferred embodiments

### General Methodology

Thin Layer Chromatography (TLC) was carried out on precoated, aluminium backed plates (60 F₂₅₄, 0.2 mm thickness; supplied by E. Merck AG, Darmstad, Germany) developed by ascending method. Flash column chromatography was carried out using silica gel supplied by Fisher (60A, 35-70 µm).

¹H-NMR (500 MHz), ¹³C-NMR (125 MHz), ³¹P- NMR (202 MHz) were recorded on a Bruker Avance 500MHz spectrometer at 25 °C. Spectra were calibrated to the residual signal of the deuterated solvent used. Chemical shifts are quoted in parts per million (ppm) downfield from tetramethylsilane.For ³¹P experiments, chemical shifts are quoted in parts per million relative to an external phosphoric acid standard. Coupling constants are measured in Hertz (*J*). The following abbreviations are used in the assignment of NMR signals: s (singlet), d (doublet), t (triplet), q (quartet), m (multiplet), bs (broad singlet), dd (doublet of doublet), dt (doublet of triplet). The characterization of phosphoramidates involves the identification of α and β sugar derivatives and in some cases the phosphorous diastereoisomers of particular compounds.

Low-resolution mass spectra (MS) was performed on Bruker Daltonics microTof-LC, (atmospheric pressure ionisation, electron spray mass spectrometry) in either positive or negative mode.

### Example 1:

### General procedure for preparing amino acid ester hydrochloride salts

Thionyl chloride (2.0 mol eq.) was added dropwise to a stirred solution of the appropriate alcohol (15.0 mol eq.) at 0 °C under nitrogen. The mixture was stirred at 0°C for 30 minutes and then slowly allowed to warm to room temperature. The appropriate amino acid (1.0 mol eq.) was added and the mixture was heated at reflux overnight. The solvent was removed under reduced pressure (last traces of solvent removed by co-evaporation with increasingly volatile solvents) to give the crude product as the solid hydrochloride salt.

### Example 2:

### General procedure for preparing amino acid ester sulphonate salts

A mixture of the appropriate amino acid (1.0 mol eq.), the appropriate alcohol (15.0 mol eq.) and *para*-toluene sulphonic acid (*p*-TSA) monohydrate (1.1 mol eq.) in toluene was heated at reflux overnight, using Dean-Stark apparatus. The solvent was removed under reduced pressure (last traces of solvent removed by co-evaporation with increasingly volatile solvents) to give the crude product as the solid *p*-toluene sulphonate salt.

### Example 3:

### General procedure for preparing phosphorodichloridates

Phosphorus oxychloride (1.0 mol eq.) and the appropriate substituted phenol (1.0 mol eq.) were stirred with anhydrous diethyl ether (30 mol eq.). Anhydrous triethylamine (2.0 mol eq.) was added at -78 °C and allowed to warm to room temperature for 3-5 hours with stirring. The triethylamine salt was filtered off, and the filtrate evaporated to dryness to give the product as a clear liquid.

### Example 4:

### General procedure for preparing phosphorochloridates

Phosphorodichloridate (1.0 mol eq.) and the appropriate amino ester hydrochloride salt (1.0 mol eq.) were suspended in anhydrous dichloromethane (61.6 mol eq.). Anhydrous triethylamine (2.0 mol eq.) was added drop wise at -78 °C and after 15 minutes the reaction was left to rise to room temperature and stirred overnight. The formation of phosphorochloridate was monitored by ³¹P-NMR. The solvent was removed under reduced pressure, anhydrous Et₂O was added to solubilise the phosphorochloridate and the triethylamine salt was removed by filtration, the filtrate was reduced to dryness and the product purified by flash chromatography (ethyl acetate/hexane 1/1 (V/V)).

### Example 5:

General procedure for preparing phosphoramidates using *N*-methylimidazole (NMI) A solution of appropriate phosphorochloridate (15.63 mmol) in anhydrous THF (15 ml) was added to a -40°C stirred solution of *N*-acetyl-D-glucosamine or *N*-pentenoyl-D-glucosamine (13.56 mmol) and NMI (6.2 ml, 78.15 mmol) in pyridine (100 ml). After 15 min the reaction was allowed to slowly warm to room temperature and stirred at room temperature for 3h. Then methanol is added and the solvent was removed under reduced pressure. The crude residue was purified by flash chromatography.

### Example 6:

### General procedure for preparing phosphoramidates using t-BuMgCl

2-acetamido-2-deoxy-4,6-*O*-benzylidene-D-glucopyranose or benzyl 2-acetamido-2-deoxy-4,6-*O*-benzylidene-D-glucopyranoside (1.98 mmol) is suspended in a 20 ml of mixture of THF and pyridine (2:1) and *t*-BuMgCl (3.97 ml, 3.96 mmol) is added at rt. Then a solution of appropriate phosphorochloridate (5.98 mmol) in THF is added dropwise. After one hour the solvent is removed under vacuum, the crude purified by flash chromatography using like eluent a gradient of CH₂Cl₂ MeOH from 98:2 to 95:5.

### Example 7:

### General procedure for the removal of the benzylidene group

A solution of 2-acetamido-2-deoxy-4,6-*O*-benzylidene-3-*O*-(substitutedphosphate)-D-glucopyranose or benzyl 2-acetamido-2-deoxy-4,6-*O*-benzylidene-3-*O*-(substituted phosphate)-*D*-glucopyranoside (0.284 mmol) was hydrogenated in ethanol (5 ml) under pressure of 54 psi for 6h in presence of 10% Pd/C. The catalyst is removed by filtration through a celite pad and the solvent was removed under reduced pressure to obtain pure compounds unless specified otherwise.

### Example 8:

### General procedure for the removal of pentenoyl group

2-deoxy-2-(pent-4-enoylamino)-3-*O*-(substituted phosphate)-D-glucopyranose or 2-deoxy-2-(penten-4-enoylamino)-4-*O*-(substituted phosphate)-D-glucopyranose (0.082 mmol) was dissolved in THF (1 ml) and an equal volume of water (1 ml) was added subsequently. The reaction mixture was treated with I₂ (63mg, 0.246 mmol, 3 eq) and allowed to stir for 15 min. Then the reaction was quenched with solid NH₄S₂O₃ and the compound extracted with CH₂Cl₂. The organic phase dried on MgSO₄ was filtered and reduced to dryness. The crude was taken up with CH₂Cl₂ and Et₂O was added to precipitate the pure compound.

### Example 9:

### Preparation of 2-acetamido-2-deoxy-3-O-[4-methoxyphenyl(benzyloxy-L-prolinyl)]phosphoramidate-D-glucopyranose (compound 1) and 2-acetamido-2-deoxy-4-O-[4-methoxyphenyl(benzyloxy-L-prolinyl)] phosphoramidate -D-glucopyranose (compound 2)

### 1. Preparation of p-methoxyphenyl phosphorodichloridate

The compound was prepared according to general procedure of Example 3, from phosphorus oxychloride (2.16 mL, 23.2 mmol), *p*-methoxyphenol (2.9 g, 23.2 mmol) and triethylamine (3.23 mL, 23.2 mmol) in diethyl ether (100 mL) to give the compound as an oil (5.7 g, 23.6 mmol).
³¹P-NMR (CDCl₃, 202 MHz): δ 4.31; ¹H-NMR (CDCl₃, 500 MHz): δ 7.12 (2H, d, *J*= 9.2 Hz), 6.80 (2H, d, *J*= 9.2 Hz), 3.70 (3H, s).

### 2. Preparation of p-methoxyphenyl(benzyloxy-L-prolinyl) phosphorochloridate

The compound was prepared according to general procedure of Example 4, from L-proline benzyl ester hydrochloride (from Sigma-Aldrich) (2.2 g, 9.1 mmol), *p*-methoxyphenyl phosphorodichloridate (2.2 g, 9.1 mmol), and triethylamine (2.5 mL, 18.2 mmol) in dichloromethane (100 mL), to yield 3.2 g, 7.8 mmol (82%) of the desired compound as an oil. ³¹P-NMR (CDCl₃, 202 MHz): δ 8.45, 8.40, (ratio 1: 1); ¹H-NMR (CDCl₃, 500 MHz): δ 7.38-7.33 (5H, m), 7.20, (2H, d, *J=* 7.7 Hz), 7.13 (2H, d, J= 7.7 Hz), 6.86 (2H, d, *J*= 8.8 Hz), 5.24-5.17 (2H, m), 4.58- 4.54 (1H, m), 4.47- 4.43 (1H, m), 3.81 (3H, s), 3.62- 3.44 (2H, m), 2.31-2.22 (2H, m), 2.05- 1.97 (2H, m).

### 3. Preparation of 2-acetamido-2-deoxy-3-O-[4-methoxyphenyl(benzyloxy-L-prolinyl)] phosphoramidate -D-glucopyranose (compound 1) and 2-acetamido-deoxy-4-O-[4-methoxyphenyl(benzyloxy-L-prolinyl)] phosphoramidate -D-glucopyranose (compound 2)

A solution of 4-methoxyphenyl(benzyloxy-L-prolinyl) phosphorochloridate (7 g, 15.66 mmol) in anhydrous THF (16 ml) was added to a -40°C stirred solution of *N*-acetyl-D-glucosamine (3.48 g, 15.66 mmol) and *N*-methylimidazole (6.2 ml, 78.30 mmol) in pyridine (85 ml). After 15 min the reaction was allowed to slowly warm to room temperature and stirred at room temperature for 3h. Then methanol was added and the solvent was removed under reduced pressure. The crude residue was purified by flash chromatography using like eluent a gradient of dichloromethane: methanol from 100:0 to 97:3. The compound 2 was isolated as a white solid.
¹H-NMR (CD₃OD, 500 MHz), mixture of two diastereoisomers, one major as α form and one minor as β form : δ 7.22 (5H, m), 6.98 (2H, m), 7.76 (2H, m), 5.12 (1H, d, *J*= 3.7 Hz), 5.04 (2H), 4.67 (1H, m), 4.48 (1H, m), 4.42 (1H, m), 4.31 (1H, m), 4.04 (2H, m), 3.75 (3H, s), 3.74 (3H, s), 3.62 (1H, m), 3.46 (1H, m), 3.38 (1H, m), 2.21 (1H, m), 2.06-1.98 (3H, m), 1.79 (3H, s); ¹³C-NMR (CD₃OD, 125 MHz): δ 22.79, 26.14 (d, *J*_{(C-P)} = 8.7 Hz), 32.38, 48.21, 58.93 (d, *J*_{(C-P)} = 3.0 Hz), 56.11, 56.16, 62.01 (d, *J*_{(C-P)} = 6.5 Hz), 66.84, 70.09, 70.13, 71.81, 79.77 (d, *J*_{(C-P)} = 6.4 Hz), 92.67, 97.65, 115.78, 122.29 (d, *J*_{(C-P)} = 4.4 Hz), 128.03, 129.27, 129.46, 129.53, 129.76, 137.44, 145.51 (d, *J*_{(C-P)} = 8.1 Hz), 158.43, 173.25, 175.25, 176.31; ³¹P-NMR (CD₃OD, 202 MHz): δ 3.03 (one diastereoisomer as α-isomer), 2.23 (one diastereoisomer as α-isomer), 2.13 (one diastereoisomer as β-isomer); MS (ES+) *m*/*z* 617.31 (MNa⁺).

Further elution with dichloromethane : methanol 97:3 afforded compound 1.
¹H-NMR (CD₃OD, 500 MHz) mixture of two diastereoisomers, one major as α form and one minor as β form : δ 7.34 (1H, m), 7.21 (4H, m), 6.89 (2H, d, *J* = 8.6 Hz), 6.71 (2H, d, *J* = 9.0 Hz), 5.06 (2H, m), 4.97 (1H, m), 4.76 (1H, m), 4.09 (2H, m), 4.98 (2H, m), 3.68 (5H, m), 3.16 (1H, m), 2.96 (1H, m), 1.88 (3H, s), 1.59 (1H, m), 1.50 (1H, m), 1.30 (1H, m), 1.24 (1H, m); ¹³C-NMR (CD₃OD, 125 MHz): δ 22.98, 25.90 (d, *J*_{(C-P)} = 8.5 Hz), 32.18 (m), 48.76 (d, *J*_{(C-P)} = 5.5 Hz, overlap with the solvent), 55.61, 56.18, 61.80 (d, *J*_{(C-P)} = 6.5 Hz), 63.89, 67.82, 70.07, 74.17 (d, *J*_{(C-P)} = 6.5 Hz), 80.12 (d, *J*_{(C-P)} = 6.1 Hz), 93.46, 103.49, 115.76, 122.26 (d, *J*_{(C-P)} = 4.5 Hz), 128.01, 129.27, 129.46, 129.51, 129.77, 130.33, 137.45, 138.98, 145.5 (d, *J*_{(C-P)} = 6.7 Hz), 158.31, 173.43, 174.78; ³¹P-NMR (CD₃OD, 202 MHz): δ 3.16 (one diastereoisomer in α-form), 2.69, 2.60 (two diastereoisomers in β-form); MS (ES+) *m*/*z* 617.31 (MNa⁺).

### Example 10:

### Preparation of 2-acetamido-2-deoxy-3-O-[4-methoxyphenyl(benzyloxy-L-alanyl)] phosphoramidate -D-glucopyranose (compound 3) and 2-acetamido-2-deoxy-4-O-[4-methoxyphenyl(benzyloxy-L-alanyl)]phosphoramidate-D-glucopyranose (compound 4)

Prepared according to general procedure of Example 5, from 4-methoxyphenyl(benzyloxy-L-alanyl)phosphorochloridate and *N*-acetyl-D-glucosamine, to give a mixture of compound 3 and compound 4.
¹H-NMR (CD₃OD, 500 MHz): δ 7.34 (m), 7.13 (d, *J*= 8.6 Hz), 7.09 (d, *J*= 8.6 Hz), 6.86 (m), 5.16 (m), 4.64 (d, *J* = 8.4 Hz), 4.62 (m), 4.35 (m), 4.27 (m), 3.94 (m), 3.79 (s), 3.78 (s), 3.75 (m), 3.65 (m), 2.03 (s), 1.71 (s), 1.73 (s) 1.25 (m); ¹³C-NMR (CD₃OD, 125 MHz): δ 20.65 (d, *J*_{(C-P)} = 7.5 Hz), 22.83, 22.82, 22.95, 23.25, 51.75, 51.83, 54.66 (d, *J*= 2.5 Hz), 56.20, 62.15, 62.24, 62.52, 62.63, 68.08, 68.11, 70.92 (d, *J*_{(C-P)} = 2.0 Hz), 71.55 (d, *J*_{(C-P)} = 5.0 Hz), 73.09, 77.45, 77.95 (d, *J*_{(C-P)} = 6.1 Hz), 80.20 (d, *J*_{(C-P)} = 6.4 Hz), 82.38 (d, *J*_{(C-P)} = 6.2 Hz), 92.41, 92.74, 93.23, 96.66, 96.88, 115.69, 122.47 (d, *J*_{(C}-_{P)} = 4.2 Hz), 122.57 (d, *J*_{(C-P)} = 4.2 Hz), 122.70 (d, *J*_{(C-P)} = 4.4 Hz), 129.35, 129.36, 129.40, 129.66, 129.68, 137.22, 137.25, 145.63 (d, *J*_{(C-P)}= 7.3 Hz), 145.76 (d, *J*_{(C-P)} = 7.2 Hz), 158.30, 158.45, 173.51, 173.59, 173.80, 173.94, 175.13 (d, *J*_{(C-P)} =5.76 Hz), 175.22 (d, *J*_{(C-P)} = 5.8 Hz), 175.23 (d, *J*_{(C-P)} = 5.8 Hz); ³¹P-NMR (CD₃OD, 202 MHz): δ 4.97, 4.66, 4.62, 4.37

### Example 11:

### Preparation of 2-acetamido-2-deoxy-3-O-[4-methoxyphenyl(benzyloxy-dimethylglycinyl)]phosphoramidate-D-glucopyranose(compuesto 5) and 2-acetamido-2-deoxy-4-O-[4-methoxyphenyl(benzyloxy-dimethylglycinyl)]phosphoramidate-D-glucopyranose (compuesto 6)

Prepared according to general procedure of Example 5, from 4-methoxyphenyl(benzyloxy-dimethylglycinyl) phosphorochloridate and N-acetyl-D-glucosamine, to give a mixture of compound 5 and compound 6.
¹H-NMR (CD₃OD, 500 MHz) mixture of four diastereoiomers, two maior in α form and two minor in β form: δ 7.36 (5H, m), 7.12 (2H, m), 6.87 (2H, m), 5.17 (3H, m) 4.63 (2H, m), 4.32 (1H, m), 4.07 (1H, m), 3.91 (2H, m), 3.77 (6H, m), 2.01 (3H, s), 1.86 (3H, s), 1.81 (3H, s),1.57 (3H, s), 1.55 (3H, s) 1.50 (3H, s) 1.48 (3H, s); ¹³C-NMR (CD₃OD, 125 MHz): δ 22.61, 22.65, 27.73, 27.78, 27.85, 27.92, 54.59 (d, *J*_{(C-P)}= 2.5 Hz), 55.95, 56.03, 57.96, 62.15, 62.24, 62.32, 62.41, 68.18, 68.24, 68.28, 71.05 (d, *J*_{(C-P)}= 2.55 Hz), 71.41 (d, *J*_{(C-P)}= 5.0 Hz), 71.67, 73.08, 72.94, 77.23 (d, *J*_{(C-P)}= 6 Hz), 77.44, 79.40 (d, *J*_{(C-P)}= 6 Hz), 81.43 (d, *J*_{(C-P)}= 6.3 Hz), 92.60, 92.70, 96.56, 97.71, 115.47, 122.50 (d, *J*_{(C-P)}= 4.5 Hz), 122.61 (d, *J*_{(C-P)} = 4.2 Hz), 122.67 (d, *J*_{(C-P)} = 4.45 Hz), 129.21, 129.28, 129.32, 129.59, 129.67, 137.28, 137.40, 145.82 (d, *J*_{(C-P)} = 7.9 Hz), 145.93 (d, *J*_{(C-P)} = 7.9 Hz), 158.35, 158.24, 173.54, 173.76, 173.92, 176.58 (d, *J*_{(C-P)}= 4.5 Hz), 176.58 (d, *J*_{(C-P)}= 4.5 Hz); ³¹P-NMR (CD₃OD, 202 MHz): δ 3.94, 3.47, 3.34; MS (ES+) *m*/*z* 605.18 (MNa⁺).

### Example 12:

### Preparation of 2-acetamido-2-deoxy-3-O-[4-methoxyphenyl(benzyloxy-L-isoleucinyl)]phosphoramidate -D-glucopyranose (compound 7) and 2-acetamido-2-deoxy-4-O-[4-methoxyphenyl(benzyloxy-L-isoleucinyl)]phosphoramidate-D-glucopyranose (compound 8)

Prepared according to general procedure of Example 5, from 4-methoxyphenyl(benzyloxy-L-isoleucinyl)phosphorochloridate and *N*-acetyl-D-glucosamine, to give a mixture of compound 7 and compound 8.
¹H-NMR (CD₃OD, 500 MHz) mixture of four diastereoisomers, two maior in α form and two minor in β form: δ 7.36 (5H, m), 7.13 (2H, m), 6.89 (2H, m), 5.17 (3H, m), 4.64 (2H, m), 4.38 (2H, m), 4.09 (1H, m), 3.95 (3H, m), 3.80 (5H, m), 3.67 (1H, m), 3.57 (1H, m), 2.07 (3H, s), 1.85 (3H, s), 1.88 (3H, s), 1.77 (1H, m), 1.39 (1H, m), 1.16 (1H, m), 0.85 (6H, m); ¹³C-NMR (CD₃OD, 125 MHz): δ 11.81, 15.84, 15.91, 22.66, 22.84, 23.16, 25.80, 40.16 (d, *J*_{(C-P)} = 6.3 Hz), 55.51 (d, *J*_{(C-P)} = 3.75 Hz), 55.99, 56.10, 60.77, 60.84, 62.20, 62.44, 62.55, 67.96, 68.01, 71.07 (d, *J*_{(C-P)} = 2.5 Hz), 71.46 (d, *J*_{(C-P)} = 5.5 Hz), 71.61, 73.05, 77.48, 77.93 (d, *J*_{(C-P)} = 5 Hz), 80.14 (d, *J*_{(C-P)} = 5 Hz), 82.15 (d, *J*_{(C-P)} = 5Hz), 92.35, 92.77, 96.61, 115.61, 122.59 (d, *J*_{(C-P)} = 4.6 Hz), 122.46 (d, *J*_{(C-P)} = 4.6 Hz), 122.35 (d, *J*_{(C-P)} = 4.6 Hz), 129.40, 129.57, 129.61, 129.66, 129.70, 137.19, 145.80 (d, *J*_{(C-P)} = 7.5 Hz), 158.31, 158.45, 173.53, 173.78, 174.15 (d, *J*_{(C-P)} = 3.35 Hz); ³¹P-NMR (CD₃OD, 202 MHz): δ 5.90, 5.62, 5.34; MS (ES+) *m*/*z* 633.21 (MNa⁺).

### Example 13:

### Preparation of 2-acetamido-2-deoxy-3-O-[4-methoxyphenyl(benzyloxy-L-leucinyl)]phosphoramidate -D-glucopyranose (compound 9) and 2-acetamido-2-deoxy-4-O-[4-methoxyphenyl(benzyloxy-L-leucinyl)]phosphoramidate-D-glucopyranose (compound 10)

Prepared according to general procedure of Example 5, from 4-methoxyphenyl(benzyloxy-L-leucinyl) phosphorochloridate and *N*-acetyl-D-glucosamine, to give a mixture of compound 9 and compound 10.
¹H-NMR (CD₃OD, 500 MHz), mixture of four diastereoiomers, two maior in α form and two minor in β form: δ 7.36 (5H, m), 7.12 (2H, m), 6.88 (2H, m), 5.15 (3H, m), 4.66 (2H, m), 4.36 (2H, m), 4.11 (1H, m), 3.97 (2H, m), 3.81 (1H, m), 3.63 (1H, m), 3.57 (1H, m), 2.02 (3H, s), 1.87 (3H, s), 1.84 (3H, s), 1.55 (1H, m), 0.84 (6H, m); ¹³C-NMR (CD₃OD, 125 MHz): δ 22.09, 22.14, 22.20, 22.73, 22.90, 23.17, 23.22, 25.47, 25.51, 44.16 (d, *J*_{(C-P)}= 7.51 Hz), 44.11 (d, *J*_{(C-P)}= 7.45 Hz), 54.60 (d, *J*_{(C-P)}= 2.9 Hz), 54.86, 55.99, 56.16, 62.22, 62.50, 62.60, 68.07, 68.10, 71.11 (d, *J*_{(C-P)}= 2.5 Hz), 71.52 (d, *J*_{(C-P)}= 5.34 Hz), 71.59, 73.08, 77.49, 78.05 (d, *J*_{(C-P)}= 6.49 Hz), 80.19 (d, *J*_{(C-P)}= 6.40 Hz), 82.28 (d, *J*_{(C-P)}= 6.40 Hz), 92.37, 92.76, 96.85, 96.98, 115.66, 122.43 (d, *J*_{(C-P)}= 4.67 Hz), 122.52 (d, *J*_{(C-P)}= 4.4 Hz), 122.63 (d, *J*_{(C-P)}= 4.4 Hz), 129.41, 129.50, 129.52, 129.63, 129.65, 137.19, 145.75 (d, *J*_{(C-P)}= 7.5 Hz), 145.81 (d, *J*_{(C-P)}= 7.5 Hz), 158.35, 158.39, 173.52, 173.77, 175.39; ³¹P-NMR (CD₃OD, 202 MHz): δ 5.47, 4.05, 5.01; MS (ES+) *m*/*z* 633.21 (MNa⁺).

### Example 14:

### Preparation of 2-acetamido-2-deoxy-3-O-[4-methoxyphenyl(benzyloxy-L-glycinyl)]phosphoramidate -D-glucopyranose (compound 11) and 2-acetamido-2-deoxy-4-O-[4-methoxyphenyl(benzyloxy-L-glycinyl)]phosphoramidate-D-glucopyranose (compound 12)

Prepared according to general procedure of Example 5, from 4-methoxyphenyl(benzyloxy-L-glycinyl) phosphorochloridate and *N*-acetyl-D-glucosamine, to give a mixture of compound 11 and compound 12.
¹H-NMR (CD₃OD, 500 MHz) mixture of four diastereoiomers, two maior in α form and two minor in β form: δ 7.35 (5H, m), 7.15 (2H, d, *J=* 8.5 Hz), 7.12 (2H, d, *J=* 8.5 Hz), 6.86 (2H, m), 5.16 (3H, m), 4.69 (1H, d, *J=* 8.6), 4.62 (2H, m), 4.42 (1H, m), 4.35 (1H, m), 4.07 (1H, d, *J=* 10.34 Hz), 3.86 (10H, m), 3.63, 2.01 (3H, s), 1.96 (3H, s), 1.86 (3H, s), 1.83 (3H, s); ¹³C-NMR (CD₃O D, 125 MHz): δ 22.71, 22.92, 23.25 , 44.03, 44.17, 54.67 (d, *J*_{(C-P)}= 3.07), 55.93, 56.16, 62.08, 62.11, 62.50, 62.64, 68.09, 70.94 (d, *J*_{(C-P)}= 2.2 Hz), 71.46, 71.56 (d, *J*_{(C-P)}= 5.5 Hz), 77.50, 78.02 (d, *J*_{(C-P)}= 6.76 Hz), 80.31 (d, *J*_{(C-P)}= 6.76 Hz), 82.36 (d, *J*_{(C-P)}= 6.36 Hz), 92.42, 92.74, 96.76, 96.87, 115.71, 122.46 (d, *J*_{(C-P)}= 4.62 Hz), 122.58 (d, *J*_{(C-P)}= 4.5 Hz), 122.64 (d, *J*_{(C-P)}= 4.52 Hz), 129.42, 129.63, 137.18, 145.58 (d, *J*_{(C-P)}= 7.5 Hz), 145.70 (d, *J*_{(C-P)}= 7.5 Hz), 158.33, 158.45, 172.63, 172.69, 172.75, 173.53, 173.79, 173.97; ³¹P-NMR (CD₃OD, 202 MHz): δ 5.83, 5.51 5.21; MS (ES+) *m*/*z* 577.15 (MNa⁺).

### Example 15:

### Preparation of 2-acetamido-2-deoxy-3-O-[4-methoxyphenyl(benzyloxy-L-methionyl)lphosphoramidate-D-glucopyranose (compound 13) and 2-acetamido-2-deoxy-4-O-[4-methoxyphenyl(benzyloxy-L-methionyl)]phosphoramidate-D-glucopyranose (compound 14)

Prepared according to general procedure of Example 5, from 4-methoxyphenyl(benzyloxy-L-methionyl) phosphorochloridate and *N*-acetyl-D-glucosamine, to give a mixture of compound 13 and compound 14.
¹H-NMR (CD₃OD, 500 MHz) mixture of four diastereoiomers, two maior in α form and two minor in β form: δ 7.36 (5H, m), 7.11 (2H, m), 6.08 (2H, m), 5.14 (3H, m), 4.63 (2H, m) 4.41 (2H, m), 4.12 (2H, m), 3.83 (4H, m), 3.64 (2H, m), 2.37 (2H, m), 1.92 (8H, m); ¹³C-NMR (CD₃OD, 125 MHz): δ 15.18, 22.30, 30.49, 34.49 (d, *J*_{(C-P)}= 6.4 Hz), 54.88 (d, *J*_{(C-P)}= 2.8 Hz), 54.67 (d, *J*_{(C-P)}= 2.8 Hz), 55.13, 56.10, 62.49, 68.23, 71.05 (d, *J*_{(C-P)}= 2.75 Hz), 71.50 (d, *J*_{(C-P)}= 5.7 Hz), 71.59, 73.07, 78.08 (d, *J*_{(C-P)}= 6.6 Hz), 80.29 (d, *J*_{(C-P)}= 6.6 Hz), 82.36 (d, *J*_{(C-P)}= 6.6 Hz), 92.38, 92.76, 96.59, 115.71, 122.36 (d, *J*_{(C-P)}= 4.8 Hz), 122.49 (d, *J*_{(C-P)}= 4.8Hz), 122.58 (d, *J*_{(C-P)}= 4.8Hz) 129.31, 129.47, 129.67, 137.58, 145.82 (d, *J*_{(C-P)}= 7.2 Hz), 158.34, 158.48, 173.54, 174.39 (d, *J*_{(C-P)}= 4.5 Hz); ³¹P-NMR (CD₃OD, 202 MHz): δ 5.32, 4.94, 4.77; MS (E/I) 651.17 (MNa⁺)

### Example 16:

### Preparation of 2-acetamido-2-deoxy-3-O-[4-methoxyphenyl(benaloxy-L-phenylalanyl)]phosphoramidate-D-glucopyranose (compound 15)

Prepared according to general procedure of Example 5, from 4-methoxyphenyl(benzyloxy-L-phenylalanyl)phosphorochloridate and *N*-acetyl-D-glucosamine.
¹H-NMR (CD₃OD, 500 MHz), mixture of four diastereoiomers, two maior in α form and two minor in β form: δ 7.33 (3H, m), 7.24 (5H), 7.11(2H), 6.99 (2H, m), 6.82 (2H, m), 5.12 (1H, d, *J*= 3.5 Hz), 5.06 (2H, m), 4.62 (2H, m), 4.26 (1H, m), 4.08 (1H, dd, *J*= 3.5 and 10.5 Hz), 3.86 (1H, m), 3.77 (3H, m), 3.99 (1H, t, *J* = 9Hz), 3.00 (2H, d, *J* = 7.5Hz), 1.83 (3H, s); ¹³C-NMR (CD₃OD, 125 MHz): δ 22.88, 23.20, 41.20 (d, *J*_{(C-P)}= 6.25 Hz), 54.62, 56.12, 57.55, 62.48, 68.07, 70.97, 73.05, 80.15 (d, *J*_{(C-P)}= 6.25 Hz), 92.71, 101.32, 115.64, 122.33 (d, *J*_{(C-P)}= 4.7 Hz), 122.43 (d, *J*_{(C-P)}= 4.71 Hz), 127.96, 129.38, 129.53, 129.57, 129.62, 130.69, 136.91, 137.30, 145.74 (d, *J*_{(C-P)}= 7.5 Hz), 158.25, 173.55, 173.97, (d, *J*_{(C-P)}= 5 Hz); ³¹P-NMR (CD₃OD, 202 MHz): δ 5.09 (one diastereoisomer as α-isomer), 5.03 (one diastereoisomer as α-isomer).

### Example 17:

### Preparation of 2-acetamido-2-deoxy-3-O-[4-methoxyphenyl(benzyloxy-L-valinyl)]phosphoramidate-D-glucopyranose (compound 16) and 2-acetamido-2-deoxy-4-O-[4-methoxyphenyl(benzyloxy-L-valinyl)]phosphoramidate-D-glucopyranose (compound 17)

Prepared according to general procedure of Example 5 (addition at room temperature), from 4-methoxyphenyl(benzyloxy-L-valinyl)phosphorochloridate and *N*-acetyl-D-glucosamine to give a mixture of compound 16 and compound 17.
¹H-NMR (CD₃OD, 500 MHz), mixture of four diastereoiomers, two maior in α form and two minor in β form: δ 7.37 (5H, m), 7.10 (2H, m), 6.88 (2H, m), 5.13 (3H, m), 4.65 (1H, m) 4.10 (1H, dd, *J*= 10.55 and 3.45 Hz), 3.92 (3H, m), 3.87 (1H, m), 3.78 (6H, m), 3.64 (1H, m), 3.58 (1H, m), 2.05 (1H, m), 2.02 (3H, s), 1.88 (3H, s), 1.84 (3H, s), 0.85 (6H, m); ¹³C-NMR (CD₃OD, 125 MHz): δ 17.82, 17.95, 19.39, 19.42, 22.66, 22.85, 33.27 (d, *J*_{(C-P)}= 6.93 Hz), 33.15 (d, *J*_{(C-P)}= 6.93 Hz), 54.53 (d, *J*_{(C-P)}= 3.04 Hz), 55.99, 56.09, 56.22, 61.74, 61.70, 61.81, 62.19, 62.21, 62.45, 62.17, 67.96, 68.04, 71.07 (d, *J*_{(C-P)}= 2.7 Hz), 71.46 (d, *J*_{(C-P)}= 5.7 Hz), 71.62, 73.04, 77.47, 77.94 (d, *J*_{(C-P)}= 6.81 Hz), 80.16 (d, *J*_{(C-P)}= 6.46 Hz), 82.24 (d, *J*_{(C-P)}= 6.86 Hz) 92.36, 92.78, 96.60, 96.79, 115.59, 115.79, 116.82, 122.32 (d, *J*_{(C-P)}= 4.5 Hz), 122.61 (d, *J*_{(C-P)}= 4.75 Hz), 122.43 (d, *J*_{(C-P)}= 5 Hz), 129.39, 129.58, 129.61, 129.64, 129.67, 137.20, 137.23, 145.87 (d, *J*_{(C-P)}= 7.0 Hz), 145.95 (d, *J*_{(C-P)}= 7.15 Hz), 158.30, 158.45, 173.52, 173.77, 174.17 (d, *J*_{(C-P)}= 4 Hz); ³¹P-NMR (CD₃OD, 202 MHz): δ 5.91, 5.70, 5.33; MS (ES+) *m*/*z* 619.3 (MNa⁺).

### Example 18:

### Preparation of 2-deoxy-2-(pent-4-enoylamino)-3-O-[4-methoxyphenyl(benzyloxy-L-alanyl)] phosphoramidate-D-glucopyranose (compound 18)

### 1. Preparation of 2-deoxy-2-(pent-4-enoylamino)-D-glucopyranose

To a vigorously stirred solution of NaOMe (3.2 g, 56.4 mmol) in MeOH (75 ml) was added (D)-glucosamine hydrochloride (12.1 g, 56.37 mmol). The mixture was stirred for 5 min. The precipitate NaCl was removed by filtration through Celite and the filter cake was rinsed with MeOH. The filtrate was placed in an ice bath, and pent-4-enoic anhydride (12.4 g 67.6 mmol, obtained according to P. Perlmutter *et al., Org. Biomol. Chem.* 2, 2220-2228 (2004); R. Madsen *et al., J. Org. Chem. 60,* 7920-7926 (1995)) was added immediately. Crystallization began almost instantaneously. The mixture was stirred for 10 min and then kept in the freezer overnight. Filtration gave the title compound (11.1 g, 75% yield). ¹H-NMR (CD₃OD, 500MHz): δ 5.83 (1H, m), 5.05 (3H, m), 3.83 (2H, m), 3.66 (2H, m), 3.45 (2H, m), 2.25 (4H, m).

### 2. Preparation of 2-deoxy-2-(pent-4-enoylamino)-3-O-[4-methoxyphenyl(benzyloxy-L-alanyl)]phosphoramidate -D-glucopyranose (compound 18)

Prepared according to general procedure of Example 5, from 4-methoxyphenyl(benzyloxy-L-alanyl)phosphorochloridate and 2-deoxy-2-(pent-4-enoylamino)-D-glucopyranose.
¹H-NMR (CD₃OD, 500 MHz), mixture of two diastereoisomers, both in α form and in β form: δ 7.34 (5H, m), 7.08 (2H, d, *J* = 8.5 Hz), 6.84 (2H, d, *J* = 8.6 Hz), 5.76 (1H, m), 5.16 (3H, m), 4.99 (1H, d, *J* = 16.9 Hz) 4.93 (1H, d, *J* = 10.11 Hz), 4.63 (1H, m), 4.07 (2H, m), 3.89 (6H, m), 3.58 (1H, m), 2.17 (4H), 1.36 (3H, d, *J* = 6.7 Hz); ¹³C-NMR (CD₃OD, 125 MHz): δ 20.58 (d, *J*_{(C-P)}= 6.7 Hz), 30.54, 36.28, 36.33, 51.76, 54.64 (d, *J*_{(C-P)}= 3.4 Hz), 54.72 (d, *J*_{(C-P)}= 3.4 Hz), 56.16, 62.47, 62.50, 68.03, 70.93 (d, *J*_{(C-P)} = 2.7 Hz), 73.11, 80.03 (d, *J*_{(C-P)} = 6.41 Hz), 80.05 (d, *J* = 6.41 Hz), 92.72, 96.71, 115.62, 115.70, 122.60 (d, *J*_{(C-P)}= 4.3 Hz), 122.50 (d, *J*_{(C-P)}= 4.3 Hz), 129.33, 129.35, 129.61, 137.24, 138.33, 145.68 (d, *J*_{(C-P)}= 7.4 Hz), 158.31, 174.83 (d, *J*(_{C-P})= 5.6 Hz), 175.47, 175.55; ³¹P-NMR (CD₃OD, 202 MHz): δ 5.14, 4.41

### Example 19:

### Preparation of 2-amino-2-deoxy-3-O-[4-methoxyphenyl(benzyloxy-L-alanyl)]phosphoramidate-D-glucopyranose (compound 19)

Prepared as a white solid (0.032g, 74% yield) according to general procedure of Example 8, starting from 2-deoxy-2-(pent-4-enoylamino)-3-*O*-[4-methoxyphenyl(benzyloxy-L-alanyl)] phosphoramidate-D-glucopyranose (compound 18 of Example 18).
¹H-NMR (CD₃OD, 500 MHz), mixture of four diastereoiomers, two maior in α form and two minor in β form: δ 7.34 (5H, m), 7.16 (2H, d, *J* = 8.6 Hz), 6.90 (2H, d, *J* = 8.6 Hz), 5.38 (1H, d, *J* = 3.55 Hz), 5.43 (1H, s), 5.17 (2H, s), 4.73 (2H, m), 4.58 (2H, m), 4.12 (1H, m) 3.88 (1H, m), 3.78 (5H, m), 3.67 (1H, m), 1.35 (3H, d, *J* = 6.8 Hz); ¹³C-NMR (CD₃OD, 125 MHz): δ 20.49 (d, *J*_{(C-P)}= 7.6 Hz), 51.90, 55.58, 56.13, 61.96, 68.16, 69.96 (d, *J*_{(C-P)}= 3.25 Hz), 72.91, 79.50 (d, *J*_{(C-P)}= 6.5 Hz), 91.06, 94.79, 115.71, 122.59 (d, *J*(_{C-P})= 4.4 Hz), 129.31, 129.35, 129.62, 137.20, 145.39 (d, *J*(_{C-P})= 7.1 Hz), 158.57, 175.63; ³¹P-NMR (CD₃OD, 202 MHz): δ 5.44 (one diastereoisomer as α-isomer), 4.41 (one diastereoisomer as β-isomer).

### Example 20:

### Preparation of 2-acetamido-2-deoxy-3-O-[4-methoxyphenyl(benzyloxy-D-alanyl)]phosphoramidate-D-glucopyranose (compound 20) and 2-acetamido-2-deoxy-4-O-[4-methoxyphenyl(benzyloxy-D-alanyl)]phosphoramidate-D-glucopyranose (compound 21)

Prepared according to general procedure of Example 5, from 4-methoxyphenyl(benzyloxy-D-alanyl)phosphorochloridate and *N*-acetyl-D-glucosamine, to give a mixture of compound 20 and compound 21.
¹H-NMR (CD₃OD, 500 MHz), mixture of four diastereoiomers, two maior in α form and two minor in β form: δ 7.34 (5H, m), 7.14 (2H, d, *J*= 8.9 Hz), 7.09 (2H, d, *J*= 9.02 Hz), 6.85 (2H, m), 5.16 (2H, s), 5.15 (2H, s), 5.12 (d, *J*= 3.4 Hz), 4.69 (1H, d, *J*= 8.55 Hz), 4.66 (1H, d, *J*= 8.55 Hz), 4.55 (1H, m), 4.37 (1H, m), 4.23 (1H, m), 4.06 (2H, m), 3.93 (1H, m), 3.86 (1H, m), 3.76 (5H, m), 3.60 (1H, m), 3.53 (1H, m), 3.34 (1H, m), 2.08 (3H, s), 1.86 (3H, s), 1.82 (3H, s), 1.40 (3H, d, J= 7.34 Hz); ¹³C-NMR (CD₃OD, 125 MHz): δ 20.73 (d, *J*_{(C-P)}= 5.75 Hz), 20.78 (d, *J*_{(C-P)}= 5.75 Hz), 22.70, 22.86, 23.20, 51.63, 51.64, 54.62 (d, *J*_{(C-P)}= 2.54 Hz), 55.83, 56.12, 61.98, 62.48, 62.60, 68.07, 68.09, 70.90 (d, *J*_{(C-P)}= 2.61 Hz), 71.33, 71.40 (d, *J*_{(C-P)}= 5.4 Hz),73.00, 77.48, 78.25 (d, *J*_{(C-P)}= 6.5 Hz), 80.38 (d, *J*_{(C-P)}= 6.5 Hz), 82.40 (d, *J*_{(C-P)}= 6.5 Hz), 92.35, 92.71, 96.59, 115.66, 122.45 (d, *J*_{(C-P)}= 4.4 Hz), 122.55 (d, *J*_{(C-P)}= 4.2 Hz), 129.61 (d, *J*_{(C-P)}= 4.4 Hz), 129.29, 129.30, 129.33, 129.64, 137.25, 137.27, 145.65 (d, *J*_{(C-P)}= 7.1 Hz), 145.74 (d, *J*_{(C-P)}= 7.87 Hz), 158.29, 158.42, 173.54, 173.75, 175.00 (d, *J*_{(C-P)}= 6.26 Hz), 175.06, (d, *J*_{(C-P)}= 6.0 Hz); ³¹P-NMR (CD₃OD, 202 MHz): δ 4.83, 4.31, 4.14; MS (ES+) *m*/*z* 591.17 (MNa⁺).

### Example 21:

### Preparation of benzyl 2-acetamido-2-deoxy-3-O-[4-methoxyphenyl(methoxy-L-alanyl)]phosphoramidate-D-glucopyranoside (compound 22)

Prepared as a white solid (0.39 g, 11% yield) according to general procedure of Example 5, from 4-methoxyphenyl(methoxy-L-alaninyl)phosphorochloridate and 1-*O*-benzyl-*N*-acetyl-D-glucosamine.
¹H-NMR (CD₃OD, 500 MHz), as a mixture of four diastereoisomers, two in the α form and two in the β form: δ 7.26- 7.13 (5H, m), 7.03- 6.92 (2H, m), 6.75- 6.71 (2H, m), 4.73- 4.69 (1H, m), 4.63- 4.56 (1H, m), 4.48- 4.44 (3H, m), 4.21- 4.13 (1H, m), 3.99-3.95 (1H, m), 3.88- 3.74 (2H, m), 3.72- 3.43 (4H, m), 3.62 (3H, s), 3.61 (3H, s), 1.84, 1.78, 1.76 (3H, 3s), 1.34- 1.19 (3H, m), 1.22- 1.15 (3H, m); ¹³C-NMR (CD₃OD, 125 MHz): δ 175.91, 173.45, 158.36, 145.74, 138.69, 129.46, 129.43, 129.37, 129.00, 128.88, 122.54, 122.50, 115.79, 115.64, 101.51, 97.65, 97.32, 79.84 (d, *J*_{(C-P)}= 6.3 Hz), 70.84, 70.45, 62.31, 56.09, 52.83, 51.73, 51.65, 22.72, 20.50, 20.45; ³¹P-NMR (CD₃OD, 202 MHz): δ 5.05, 4.67, 4.23 (ratio 11.6: 3.2: 1.0); MS (ES+) *m*/*z* 605.18 (MNa⁺).

### Example 22:

### Preparation of methyl 2-acetamido-2-deoxy-3-O-[4-methoxyphenyl(benzyloxy-L-valinyl)]phosphoramidate-D-glucopyranoside (compound 23) and methyl 2-acetamido-2-deoxy-4-O-[4-methoxyphenyl(benzyloxy-L-valinyl)]phosphoramidate-D-glucopyranoside (compound 24)

Prepared according to general procedure of Example 5, from 4-methoxyphenyl(benzyloxy-L-valinyl)phosphorochloridate and 1-*O*-methyl-*N*-acetyl-D-glucosamine, to give a mixture of compound 23 and compound 24.
¹H-NMR (CD₃OD, 500 MHz): δ 7.45- 7.30 (5H, m), 7.11 (2H, d, *J*= 8.4 Hz), 6.86 (2H, d, *J*= 8.4 Hz), 5.16 (2H, app s), 4.70 (1H, app s), 4.58- 4.53 (1H, m), 4.21- 4.19 (1H, m), 3.88- 3.86 (1H, m), 3.83- 3.59 (3H, m), 3.78 (3H, s), 3.63-3.33 (1H, m), 3.40 (3H, s), 2.08- 1.99 (1H, m), 1.85 (3H, s), 0.89 (3H, d, *J*= 7.0 Hz), 0.82 (3H, d, *J*= 7.0 Hz).

### Example 23:

### Preparation of methyl 2-acetamido-2-deoxy-3-O-[4-methoxyphenyl(benzyloxy-L-alanyl)] phosphoramidate -D-glucopyranoside (compound 25)

Prepared as a white solid (1.6 g, 13% yield) according to general procedure of Example 5, from 4-methoxyphenyl(benzyloxy-L-alanyl)phosphorochloridate and 1-*O*-methyl-N-acetyl-D-glucosamine.
¹H-NMR (CD₃OD, 500 MHz), a mixture of two diastereoisomers in the α form: δ 7.43- 7.29 (5H, m), 7.19- 7.05 (2H, m), 6.91- 6.82 (2H, m), 5.21- 5.11 (2H, m), 4.69 (1H, app s), 4.55-4.48 (1H, m), 4.34- 4.26 (1H, m), 4.14 (1H, m), 4.10- 3.99 (1H, m), 3.88- 3.82 (1H, m), 3.78 (3H, s), 3.75- 3.69 (1H, m), 3.68- 3.58 (2H, m), 3.30 (3H, s), 2.01 (3H, s), 1.83 (3H, s), 1.41-1.30 (3H, m); ¹³C-NMR (CD₃OD, 125 MHz): δ 175.13 (d, *J_{(C-P)}*= 6.3 Hz), 173.53, 158.32, 145.71 (d, *J_{(C-P)}*= 7.3 Hz), 137.25, 129.60, 129.34, 129.30, 122.63, 122.59, 122.52, 122.48, 115.78, 99.83, 99.58, 80.18 (d, *J_{(C-P)}*= 6.3 Hz), 77.76, 73.67, 70.80, 70.78, 68.04, 67.99, 62.35, 62.04, 56.08, 55.73, 55.65, 54.01, 53.98, 51.86, 51.73, 22.75, 22.59, 20.55, 20.49; ³¹P-NMR (CD₃OD, 202 MHz): δ 4.83, 4.56 (ratio 3.0: 1.0); MS (ES+) *m*/*z* 605.18 (MNa⁺).

### Example 24:

### Preparation of methyl 2-benzyloxycarbonylamino-2-deoxy-3-O-[4-methoxyphenyl(ethoxy-L-alanyl)]phosphoramidate-D-glucopyranoside (compound 26)

### 1. Preparation of N-benzyloxycarbonyl-D-glucosamine

To a stirring solution of benzyl chloroformate (5.5 mL, 6.7 g, 39.1 mmol) and amberlite (OH⁻) resin (1.4 mmol/ ml eq), (41 mL, 57.5 mmol) in water (50 mL) at 0°C, glucosamine HCl (5.0 g, 23.0 mmol), was added.The reaction was warmed to room temperature whilst stirring and monitored by TLC (dichloromethane/methanol 8/2 V/V). After 16 h the reaction was completed, the solution was filtered to remove the resin, then evaporated under reduced pressure and vacuum gun to give a white solid (5.3g, 17.0 mmol, 74%).
¹H-NMR ((CD₃)₂SO, 500 MHz), mixture of α and β anomers (ratio 2.4: 1.0): δ 7.49- 7.41 (5H, m), 5.20 (1H, app. s), 4.99 (2H, s), 4.95 (1H, app. s), 4.65 (bs), 4.45 (bs), 3.57- 3.00 (6H, m), 3.30 (bs), 2.59 (bs), 2.50 (bs); ¹³C-NMR (DMSO, 125 MHz): δ 156.08, 137.08, 128.30, 127.72, 95.43, 90.66, 71.98, 70.97, 70.26, 65.22, 65.02, 61.17, 61.07, 56.35

### 2. Preparation of methyl 2-benzyloxycarbonylamino-2-deoxy3-O-[4-methoxyphenyl(ethoxy-L-alanyl)]phosphoramidate-D-glucopyranoside (compound 26)

Prepared as a white solid (127.0 mg, 7% yield) according to general procedure of Example 5, from p-methoxyphenyl(ethoxy-L-alanyl) phosphorochloridate and *N*-benzyloxycarbonyl-D-glucosamine.
¹H-NMR (CD₃OD, 500 MHz): δ 7.42- 7.25 (5H, m), 7.20- 7.09 (2H, m), 6.94- 6.79 (2H, m), 5.20- 4.91 (3H, m), 4.74- 4.67 (1H, m), 4.62- 4.54 (1H, m), 4.47- 4.29 (1H, m), 4.23- 4.08 (2H, m), 4.03- 3.93 (1H, m), 3.92- 3.72 (3H, m), 3.78, 3.74 (3H, 2s), 3.71- 3.58 (1H, m), 3.50- 3.35 (1H, m), 1.38- 1.31 (3H, m), 1.29- 1.19 (3H, m); ¹³C-NMR (CD₃OD, 125 MHz): δ 175.42, 175.42, 175.26, 158.39, 158.22, 144.80 (d, *J_{(C-P)}*= 7.2 Hz), 173.98, 129.46, 128.95 (d, *J_{(C-P)}*= 8.1 Hz), 128.85, 122.50, 122.46, 122.41, 122.36, 122.33, 115.67, 115.64, 115.56, 97.34, 93.02, 80.54 (d, *J_{(C-P)}*= 6.6 Hz), 73.06, 72.73, 71.96, 71.77, 71.61, 71.06, 67.80, 67.45, 67.60, 67.45, 62.46, 62.39, 57.56 (d, *J_{(C-P)}* = 2.8 Hz), 56.13, 56.09, 51.71, 51.61, 51.52 , 20.65 (d, *J_{(C-P)}*= 6.4 Hz), 14.52, 14.48; ³¹P-NMR (CD₃OD, 202 MHz): δ 4.88, 4.47 (α diastereoisomers) 4.28, 4.14 (β diastereoisomers).

### Example 25:

### Preparation of methyl 2-acetamido-2-deoxy-3-O-[4-methoxyphenyl(benzyloxy-L-prolinyl)]phosphoramidate-D-glucopyranoside (compound 27)

Prepared as a white solid (0.151 g, 3% yield) according to general procedure of Example 5, from 4-methoxyphenyl(benzyloxy-L-prolinyl)phosphorochloridate and 1-*O*-methyl-N-acetyl-D-glucosamine.
¹H-NMR (CD₃OD, 500 MHz), mixture of two diastereoisomers in the α form: δ 7.37- 7.34 (5H, m), 7.09 (2H, d, *J*= 8.2 Hz), 6.87 (2H, d, *J*= 8.2 Hz), 5.17 (2H, 2d, *J*= 12.1 Hz), 4.70 (1H, app. s), 4.63- 4.54 (1H, m), 4.39- 4.33 (1H, m), 4.09 (2H, d, *J*= 10.5 Hz), 3.87 (1H, d, *J*= 12.0 Hz), 3.78 (3H, s), 3.78- 3.75 (1H, m), 3.65- 3.58 (2H, m), 3.58- 3.51 (1H, m), 3.45- 3.33 (1H, m), 3.40 (3H, s), 2.26- 2.16 (1H, m), 2.03-1.95 (1H, m), 1.94- 1.81 (2H, m), 1.80 (3H, s); ¹³C-NMR (CD₃OD, 125 MHz): δ 175.77, 173.38, 158.39, 145.00, 137.15, 129.63, 129.40, 129.31, 122.20 (d, *J_{(C-P)}*= 4.6 Hz), 115.75, 99.79, 80.09 (d, *J_{(C-P)}*= 7.5 Hz), 70.87, 70.79, 68.16, 62.39, 62.11 (d, *J*= 6.4 Hz), 56.09, 55.66, 54.21, 48.74 (d, *J_{(C-P)}*= 4.7 Hz), 32.29 (d, *J_{(C-P)}*= 9.0 Hz), 26.16 (d, *J*_{(C-P)}= 8.9 Hz), 22.76; ³¹P-NMR (CD₃OD, 202 MHz): δ 3.04, 2.54 (ratio 12.2: 1.0); MS (ES+) *m*/*z* 631.20 (MNa⁺).

### Example 26:

### Preparation of 2-acetamido-2-deoxy-3-O-[1-naphtyl(benzyloxy-L-alanyl)]phosphoramidate-D-glucopyranose (compound 28)

Prepared according to general procedure of Example 5, from 1-naphthyl(benzyloxy-L-alanyl) phosphorochloridate and *N*-acetyl-D-glucosamine.
¹H-NMR (CD₃OD, 500 MHz), mixture of four diastereoiomers, two maior in α form and two minor in β form: δ 8.16 (1H, m), 7.88 (1H, m), 7.70 (1H, m), 7.27 (9H, m), 5.15 (3H, m), 4.71 (1H, m) 4.18 (1H, m), 4.07 (1H, m), 3.89 (1H, m), 3.80 (2H, m), 1.80 (3H, s), 1.70 (3H, s), 1.41 (3H, m); ¹³C-NMR (CD₃OD, 125 MHz): δ 20.50 (d, *J*_{(C-P)}= 7.5 Hz), 22.72, 51.86, 54.81, 62.46, 62.58, 67.99, 70.95, 73.21, 80.13 (d, *J*_{(C-P)}= 6.4 Hz), 92.57, 96.68, 116.68, 123.01, 126.01, 126.63, 127.19, 127.45, 128.82, 128.04, 129.25, 129.27, 129.36, 129.53, 129.64, 136.28, 137.16, 148.14 (d, *J*_{(C-P)}= 7 Hz), 173.45, 175.31; ³¹P-NMR (CD₃OD, 202 MHz): δ 5.00 (one diastereoisomer as α-isomer), 4.29 (one diastereoisomer as β-isomer). MS (ES+) *m*/*z* 611.17 (MNa⁺).

### Example 27:

### Preparation of 2-acetamido-2-deoxy-3-O-[4-methoxyphenyl(ethoxy-L-glycinyl)lphosphoramidate-D-glucopyranose (compound 29) and 2-acetamido-2-deoxy-4-O-[4-methoxyphenyl(ethoxy-L-glycinyl)]phosphoramidate-D-glucopyranose (compound 30)

Prepared according to general procedure of Example 5 (addition at room temperature), from 4-methoxyphenyl(ethoxy-L-glicinyl)phosphorochloridate and *N*-acetyl-D-glucosamine, to give a mixture of compound 29 and compound 30.
¹H-NMR (CD₃OD, 500 MHz): δ 7.15 (2H, m), 6.9 (2H, m), 5.13 (1H, d, *J*= 3.73 Hz), 4.7 (1H, d, *J*= 8.8 Hz), 4.63 (1H, m), 4.61 (2H, m), 4.07 (1H, dd, *J*= 3.46 and 10.49 Hz), 3.88 (1H, m), 3.87 (1H, m), 3.63 (1H, m), 1.88 (3H, s), 1.84 (3H, s), 1.28 (3H, m); ³¹P-NMR (CD₃OD, 202 MHz): δ 5.90, 5.66, 5.25

### Example 28:

### Preparation of 2-acetamido-2-deoxy-3-O-[4-methoxyphenyl(ethoxy-L-prolinyl)lphosphoramidate-D-glucopyranose (compound 31) and 2-acetamido-2-deoxy-4-O-[4-methoxyphenyl(ethoxy-L-prolinyl)]phosphoramidate-D-glucopyranose (compound 32)

Prepared according to general procedure of Example 5 (addition at room temperature), from *N*-acetyl-D-glucosamine and 4-methoxyphenyl(ethoxy-L-prolinyl)phosphorochloridate. The crude product was purified by flash chromatography on silica gel (under gradient elution system of dichloromethane : methanol, 100:0 - 97:3). Compound 32 was isolated as a white solid, a mixture of two diastereoisomers, one major in α form and the other minor in β form.
¹H-NMR (CD₃OD, 500 MHz), mixture of four diastereoisomers, two major in α form and two minor in β form: δ 7.17 (2H, d, *J*= 8.6 Hz), 7.11 (2H, d, *J*= 8.6 Hz), 6.91 (2H, d, *J*= 9.2 Hz), 6.87 (2H, d, *J*= 9.2 Hz), 5.12 (1H, d, *J*= 3.2 Hz), 4.61 (1H, m), 4.49 (1H, m), 4.41 (1H, m), 4.30 (1H, m), 4.22 (2H, m), 4.04 (2H, m), 3.71 (3H, m), 3.51 (1H, m), 3.48 (1H, m), 3.31 (1H, m), 2.21 (1H, m), 2.00 (1H, m), 1.90 (2H, m), 1.81 (3H, s), 1.21 (3H, m); ¹³C-NMR (CD₃OD, 125 MHz): δ 14.48, 22.81, 26.17 (d, *J*_{(C-P)}= 9.2 Hz), 32.31 (d, *J*_{(C-P)}= 9.3 Hz), 48.29, 54.85, 56.12 (d, *J*_{(C-P)}= 6.7 Hz), 61.78 (d, *J*_{(C-P)}= 6.7 Hz), 62.32, 62.71, 66.83, 70.17 (d, *J*_{(C-P)}= 3.6 Hz), 71.78 (d, *J*_{(C-P)}= 3.9 Hz), 79.43 (d, *J*_{(C-P)}= 7.6 Hz), 92.72, 96.88, 115.68, 122.30 (d, *J*_{(C-P)}= 4.4 Hz), 145.48 (d, *J*_{(C-P)}= 7.6 Hz), 158.40, 173.29, 175.22, 176.13; ³¹P-NMR (CD₃OD, 202 MHz): δ 3.11 (one diastereoisomer as α-isomer), 2.55 (one diastereoisomer as α-isomer) 2.47 (one diastereoisomer as β-isomer), 2.24 (one diastereoisomer as β-isomer); MS (ES+) *m*/z 555.17 (MNa⁺).

Further elution with dichloromethane : methanol 97:3 afforded compound 31.
¹H-NMR (CD₃OD, 500 MHz), mixture of four diastereoisomers, two major in α form and two minor in β form: δ 7.03 (2H, d, *J* = 8.6 Hz), 7.01 (2H, d, *J* = 8.6 Hz), 6.81 (2H, d, *J* = 9.2 Hz), 6.78 (2H, d, *J*= 9.2 Hz), 5.01 (1H, d, *J*= 3.2 Hz), 4.57 (1H, d, *J*= 7.0 Hz), 4.53 (1H, m), 4.33 (1H, m), 4.18 (1H, m), 4.06 (1H, q, *J*= 7.4 Hz), 3.97 (1H, dd, *J*= 10.6 Hz, *J*= 3.8 Hz), 3.82 (1H, m), 3.75 (1H, m), 3.69 (5H, m), 3.53 (1H, m), 3.46 (1H, m), 3.25 (1H, m), 2.09 (1H, m), 1.90 (3H, s), 1.87 (1H, m), 1.80 (5H, m), 1.69 (3H, s), 1.15 (3H, t, *J*= 7.3 Hz); ¹³C-NMR (CD₃OD, 125 MHz): δ 14.49, 22.87, 26.25 (d, *J*_{(C-P)}= 9.2 Hz), 32.35 (d, *J*_{(C-P)}= 9.3 Hz), 48.73, 54.91 (d, *J*_{(C-P)}= 3.0 Hz), 56.14, 62.17 (d, *J*_{(C-P)}= 6.7 Hz), 62.50, 62.71, 70.92 (d, *J*_{(C-P)}= 3.6 Hz), 73.91, 79.93 (d, *J*_{(C-P)}= 7.6 Hz), 82.43 (d, *J*_{(C-P)}= 7.6Hz), 92.67, 96.76, 115.76, 122.02 (d, *J*_{(C-P)}= 4.4 Hz), 145.49 (d, *J*_{(C-P)}= 7.6 Hz), 158.39, 173.31, 173.39, 175.26; ³¹P-NMR (CD₃OD, 202 MHz): δ 3.19 (one diastereoisomer as α-isomer), 2.83 (one diastereoisomer as α-isomer) 2.74 (one diastereoisomer as β-isomer), 2.58 (one diastereoisomer as β -isomer); MS (ES+) *m*/*z* 555.17 (MNa⁺).

### Example 29:

### Preparation of methyl 2-acetamido-2-deoxy-3-O-[4-methoxyphenyl(ethoxy-L-valinyl)]phosphoramidate-D-glucopyranoside (compound 33)

Prepared according to general procedure of Example 5, from 4-methoxyphenyl(ethoxy-L-valinyl)phosphorochloridate and 1-*O*-methyl-*N*-acetyl-D-glucosamine.
¹H-NMR (CD₃OD, 500 MHz), mixture of two diastereoisomers: δ 7.83, 7.81 (2bs), 7.19-7.11 (2H, m), 6.92- 6.87 (2H, m), 4.70 (1H, d, *J*= 3.4 Hz), 4.57-4.51 (1H, m), 4.24- 4.14 (3H, m), 3.89- 3.85, 3.80- 3.71 (2H, m), 3.76 (3H, s), 3.69- 3.61 (2H, m), 3.42 (3H, s), 3.41 (3H, s), 2.10- 2.03 (1H, m), 1.87 (3H, s), 1.29 (3H, t, *J*= 7.0 Hz), 1.19 (3H, t, *J*= 7.0 Hz), 0.92 (3H, d, *J*= 7.0 Hz), 0.91 (3H, d, *J*= 7.0 Hz), 0.91- 0.85 (3H, m); ¹³C-NMR (CD₃OD, 125 MHz): δ 172.88 (d, *J_{(C-P)}*= 3.6 Hz), 177.08, 156.80, 144.46 (d, *J_{(C-P)}*= 7.3 Hz), 122.40 (d, *J_{(C-P)}*= 4.5 Hz), 115.92, 115.59, 99.90, 98.11, 80.07 (d, *J_{(C-P)}*= 6.0 Hz), 73.67, 70.94, 70.92, 62.32, 62.28, 61.67, 56.08, 55.63, 53.87, 33.21, 33.15, 22.78, 19.39, 17.91, 14.53; ³¹P-NMR (CD₃OD, 202 MHz): δ 5.90, 5.74 (Ratio, 9.0: 1.0); MS (ES+) *m*/*z* 571.20 (MNa⁺).

### Example 30:

### Preparation of 2-acetamido-2-deoxy-3-O-[4-methoxyphenyl(isopropoxy-L-valinyl)]phosphoramidate-D-glucopyranose (compound 34) and 2-acetamido-2-deoxy-4-O-[4-methoxyphenyl(isopropoxy-L-valinyl)]phosphoramidate-D-glucopyranose (compound 35)

Prepared according to general procedure of Example 5, from 4-methoxyphenyl(isopropoxy-L-valinyl)phosphorochloridate and *N*-acetyl-D-glucosamine, to give a mixture of compound 34 and compound 35.
¹H-NMR (CD₃OD, 500 MHz): δ 7.11 (2H, m), 6.86 (2H, m), 5.10 (1H, s), 5.01 (1H, m), 4.65 (1H, m), 4.57 (1H, m) 4.36 (1H, m), 4.08(1H, m), 3.95 (2H, m), 3.87 (1H, m), 3.78 (7H, m), 2.04 (4H, m), 1.99 (3H, s), 1.88 (3H, s), 1.84 (3H, s), 1.25 (6H, m), 0.89 (6H, m); ¹³C-NMR (CD₃OD, 125 MHz): δ 16.45, 17.90, 19.39, 19.42, 22.66, 22.85, 33.09 (d, *J*_{(C-P)}= 7.2 Hz), 33.24 (d, *J*_{(C-P)}= 6.4 Hz), 54.55 (d, *J*_{(C-P)}= 3.0 Hz), 56.01, 56.10, 61.74, 61.70, 61.83, 62.17, 62.45, 70.28, 70.31, 71.10 (d, *J*_{(C-P)}= 2.7 Hz), 71.44 (d, *J*_{(C-P)}= 5.7 Hz), 71.62, 73.08, 77.83 (d, *J*_{(C-P)}= 6.25 Hz), 80.07 (d, *J*_{(C-P)}= 6.18 Hz), 92.36, 92.73, 115.59, 122.41 (d, *J*_{(C-P)}= 4.5 Hz), 122.54 (d, *J*_{(C-P)}= 4.75 Hz), 145.94 (d, *J*_{(C-P)}= 7.2 Hz), 158.29, 158.43, 173.49, 173.78, 173.92; ³¹P-NMR (CD₃OD, 202 MHz): δ 4.61, 4.39, 3.96

### Example 31:

### Preparation of methyl 2-acetamido-2-deoxy-3-O-[4-methoxyphenyl(ethoxy-L-sarcosinyl)]phosphoramidate-D-glucopyranoside (compound 36)

Prepared according to general procedure of Example 5, from 4-methoxyphenyl(ethoxy-L-sarcosinyl)phosphorochloridate and 1-*O*-methyl-N-acetyl-D-glucosamine.
¹H-NMR (CD₃OD, 500 MHz), mixture of two diastereoisomers: δ 7.09- 7.01 (2H, m), 6.79- 6.76 (2H, m), 4.57 (1H, d, *J*= 3.4 Hz), 4.41- 4.34 (1H, m), 4.07 (2H, q, *J*= 7.3 Hz), 4.01 (1H, dd, *J*= 10.5 Hz, *J*= 3.5 Hz), 3.82- 3.60 (4H, m), 3.67 (3H, s), 3.54- 3.47 (2H, m), 3.27 (3H, s), 3.26 (3H, s), 2.68 (1H, d, *J_{(H-P)}*= 9.6 Hz), 1.70 (3H, s), 1.16 (3H, t, *J*= 7.3 Hz); ¹³C-NMR (CD₃OD, 125 MHz): δ 174.43, 172.30 (d, *J_{(C-P)}*= 3.8 Hz), 158.40, 145.54 (d, *J_{(C-P)}*= 6.3 Hz), 122.38 (d, *J_{(C}-_{P)}*= 4.2 Hz), 115.74, 99.84, 80.58 (d, *J_{(C-P)}*= 6.9 Hz), 73.71, 70.69 (d, *J_{(C-P)}*= 2.7 Hz), 62.43, 62.36, 56.11, 55.66, 54.03 (d, *J_{(C-P)}*= 3.6 Hz), 51.91 (d, *J_{(C-P)}*= 5.6 Hz), 35.59 (d, *J_{(C-P)}*= 3.3 Hz), 22.78, 14.50; ³¹P-NMR (CD₃OD, 202 MHz): δ 6.20, 5.83 (α-diastereoisomer ratio 20.0 : 1.0); MS (ES+) m/z: 543.17 (MNa⁺).

### Example 32:

### Preparation of 2-acetamido-2-deoxy-3-O-[4-methoxyphenyl(ethoxy-L-sarcosinyl)]phosphoramidate-D-glucopyranose (compound 37)

Prepared according to general procedure of Example 5, from 4-methoxyphenyl(ethoxyL-sarcosinyl)phosphorochloridate and *N*-acetyl-D-glucosamine.
¹H-NMR (CD₃OD, 500 MHz), mixture of four diastereoisomers, two in the α and two in the β form: δ 7.76, 7.75, 7.62, 7.60 (bs), 7.10- 7.02 (2H, m), 6.81- 6.78 (2H, m), 5.02 (1H, d, *J*= 3.2 Hz), 5.00 (1H, d, *J*= 3.0 Hz), 4.65 (1H, d, *J*= 8.3 Hz), 4.58 (1H, d, *J*= 8.3 Hz), 4.46 (1H, dd, *J*= 10.6, 8.8 Hz), 4.44 (1H, dd, *J*= 10.4, 8.4 Hz), 4.31- 4.20 (1H, m), 4.12- 4.15 s (2H, m), 3.94 (1H, d, *J*= 3.2 Hz), 3.92 (1H, d, *J =* 3.1 Hz), 3.86- 3.60 (6H, m), 3.65 (3H, s), 3.52 (1H, dd), 3.49- 3.43 (1H, m), 2.71 (3H, s), 2.69 (1H, m), 2.67 (1H, m), 1.91 (3H, s), 1.90 (3H, s), 1.74 (3H, s), 1.70 (3H, s), 1.15 (3H, t, *J*= 7.1 Hz); ¹³C-NMR (CD₃OD, 125 MHz): δ 173.43, 172.38 (d, *J_{(C-P)}*= 3.8 Hz), 158.40, 145.60 (d, *J_{(C-P)}*= 7.5 Hz), 122.44, 122.40, 122.36, 122.32, 122.29, 115.76, 115.69, 96.61, 92.70, 80.84 (d, *J_{(C-P)}*= 6.9 Hz), 80.55 (d, *J_{(C-P)}*= 6.9 Hz), 73.14, 70.83 (d, *J_{(C-P)}*= 2.8 Hz), 62.47, 62.44, 56.13, 54.71 (d, *J_{(C-P)}*= 3.0 Hz), 51.94 (d, *J_{(C-P)}*= 5.4 Hz), 51.85 (d, *J_{(C-P)}*= 5.4 Hz), 35.61 (d, *J_{(C-P)}*= 3.4 Hz), 35.42 (d, *J_{(C-P)}*= 3.8 Hz), 35.31 (d, *J(_{C-P)}*= 3.8 Hz), 22.84, 14.50; ³¹P-NMR (CD₃OD, 202 MHz): δ: 6.34, 5.24 (α-isomers ratio 22.3 : 1.0); δ 5.98, 5.69 (β-isomers ratio 1.0 : 1.0). Ratio of α to β, 1.7:1.0; MS (ES+) *m*/*z* 529.15 (MNa⁺).

### Example 33:

### Preparation of 2-acetamido-2-deoxy-3-O-[4-methoxyphenyl(ethoxy-L-valinyl)]phosphoramidate-D-glucopyranose (compound 38) and 2-acetamido-2-deoxy-4-O-[4-methoxyphenyl(ethoxy-L-valinyl)]phosphoramidate-D-glucopyranose (compound 39)

Prepared according to general procedure of Example 5 (addition at room temperature), from 4-methoxyphenyl(ethoxy-L-valinyl)phosphorochloridate and *N*-acetyl-D-glucosamine, to give a mixture of compound 38 and compound 39.
¹HNMR (CD₃OD, 500 MHz): δ 7.06- 6.99 (2H, m), 6.79- 6.76 (2H, m), 5.38 (1H, app. s), 4.57 (1H, d, *J*= 8.4 Hz), 4.53- 4.47 (1H, m), 4.33- 4.23 (1H, m), 4.09- 3.97 (2H, m), 3.86- 3.48 (6H, m), 3.66 (3H, s), 1.97- 1.87 (1H, m), 1.90 (3H, s), 1.79 (3H, s), 1.75 (3H, s), 1.19- 1.13 (3H, m), 0.82- 0.75 (6H, m); ¹³C-NMR (CD₃OD, 125 MHz): δ 174.41 (d, *J_{(C-P)}*= 3.9 Hz), 173.85, 173.79, 173.52, 158.45, 158.30, 145.97 (d, *J_{(C-P)}*= 7.5 Hz), 145.86 (d, *J_{(C-P)}*= 7.5 Hz), 122.61 (d, *J_{(C-P)}*= 4.6 Hz), 122.45 (d, J*_{(C-P)}*= 4.6 Hz), 122.34 (d, *J_{(C-P)}*= 4.6 Hz), 115.65, 96.79, 96.66, 92.78, 92.40, 82.27 (d, *J_{(C-P)}*= 6.3 Hz), 80.08 (d, *J_{(C-P)}*= 6.2 Hz), 77.83 (d, *J_{(C-P)}*= 6.2 Hz), 77.51, 73.09, 71.61, 71.49 (d, *J_{(C-P)}*= 5.7 Hz), 71.10, 62.59, 62.48, 62.38, 62.29, 62.17, 61.80, 61.71, 61.63, 57.31, 57.28, 56.16, 56.02, 54.88, 54.53 (d, *J_{(C-P)}*= 3.1 Hz), 33.23 (d, *J_{(C-P)}*= 7.0 Hz), 33.14 (d, *J_{(C-P)}*= 7.0 Hz), 23.25, 23.03, 22.92, 22.73, 19.48, 19.45, 18.01, 17.91, 14.67, 14.61; ³¹P-NMR (CD₃OD, 202 MHz): δ 6.03, 5.84, 5.37

### Example 34:

### Preparation of 2-acetamido-2-deoxy-3-O-[4-methoxyphenyl(n-butoxy-L-prolinyl)]phosphoramidate-D-glucopyranose (compound 40) and 2-acetamido-2-deoxy-4-O-[4-methoxyphenyl(n-butoxy-L-prolinyl)]phosphoramidate-D-glucopyranose (compound 41)

Prepared according to general procedure of Example 5 (addition at room temperature), from *N*-acetyl-D-glucosamine and 4-methoxyphenyl(butoxy-L-prolinyl)phosphorochloridate. The crude product was purified by flash chromatography on silica gel (under gradient elution system of dichloromethane : methanol, 100:0 - 97:3). Compound 41 was isolated as a white solid.
¹H-NMR (CD₃OD, 500 MHz), mixture of two diastereoisomers, one major in α form and one minor in β form: δ 7.12 (2H, d, *J* = 9.1 Hz), 7.08 (2H, d, *J* = 9.1 Hz), 6.91 (2H, d, *J* = 9.1 Hz), 6.89 (2H, d, *J* = 9.1 Hz), 5.14 (1H, d, *J* = 3.3 Hz), 4.61 (1H, m), 4.51 (1H, m), 4.42 (1H, m), 4.38 (1H, m), 4.33 (2H, q, *J* = 7.3 Hz), 4.14 (2H, m), 3.75 (3H, m), 3.51 (1H, m), 3.45 (1H, m), 3.26 (1H, m, overlap with solvent), 2.24 (1H, m) 2.10 (1H, m), 1.95 (2H), 1.79 (3H, s), 1.75 (3H, s), 1.83 (2H, m), 1.55 (2H, m), 1.62 (2H, m), 0.82 (3H, t, *J* = 7.3 Hz); ¹³C-NMR (CD₃OD, 125 MHz): δ 10.69, 20.16, 22.99, 26.14 (d, *J*_{(C-P)} = 8.7 Hz), 31.78, 48.49, 54.85 (d, *J*_{(C-P)} = 3.0 Hz), 56.07, 62.06 (d, *J*_{(C-P)} = 6.5 Hz), 66.84, 66.81 (d, *J*_{(C-P)} = 6.4 Hz), 70.21, 71.75 (d, *J*_{(C-P)} = 3.0 Hz), 79.45 (d, *J*_{(C-P)}= 6.4 Hz), 92.39, 97.65, 115.67, 122.30 (d, *J*_{(C-P)} = 4.4 Hz),122.05 (d, *J*_{(C-P)} = 4.9 Hz), 122.25 (d, *J*_{(C-P)} = 4.6 Hz), 122.35 (d, *J*_{(C-P)} = 4.9 Hz), 145.51 (d, *J*_{(C-P)} = 8.1 Hz), 158.37, 173.44, 175.29, 176.18; ³¹P-NMR (CD₃OD, 202 MHz): δ 3.17 (one diastereoisomer as α-isomer), 2.50 (one diastereoisomer as α-isomer), 2.24 (one diastereoisomer as β-isomer); MS (ES+) *m*/*z* 583.20 (MNa⁺).

Further elution with dichloromethane : methanol 97:3 to 9:1 afforded compound 40.
¹H-NMR (CD₃OD, 500 MHz): δ 7.02 (2H, m), 6.98 (2H, m), 6.79 (2H), 5.09 (1H, d, *J =* 3.5 Hz), 4.56 (1H, d, *J* = 8.3 Hz), 4.52 (1H, m), 4.32 (1H, m), 4.18 (1H, m), 4.02 (2H, m), 3.91 (1H, dd, *J =* 10.4 Hz, *J =* 3.4 Hz), 3.82 (1H, m), 3.75 (1H, m), 3.69 (5H, m), 3.52 (1H, m), 3.45 (1H, m), 3.24 (1H, m, overlap with solvent), 2.08 (1H, m) 1.90 (3H, s), 1.87 (1H, m), 1.78 (2H), 1.75 (3H, s), 1.68 (3H, s), 1.51 (2H, m), 1.26 (2H, m), 0.82 (3H, t, *J* = 7.3 Hz); ¹³C-NMR (CD₃OD, 125 MHz): δ 14.04, 20.16, 22.67, 22.85, 26.24 (d, *J*_{(C-P)} = 8.75 Hz), 31.78, 32.47 (d, *J*_{(C-P)} = 8.7 Hz), 48.71, 54.91 (d, *J*_{(C-P)} = 3.0 Hz), 56.11, 56.32, 62.18 (d, *J*_{(C-P)} =6.5 Hz), 62.54, 66.47, 71.00 (d, *J*_{(C-P)} = 3.0 Hz), 73.38, 79.94 (d, *J*_{(C-P)} = 6.4 Hz), 92.39, 97.65, 96.76, 115.78, 122.02 (d, *J*_{(C-P)} = 4.4 Hz), 122.05 (d, *J*_{(C-P)} = 4.9 Hz), 122.25 (d, *J*_{(C-P)} = 4.6 Hz), 122.35 (d, *J*_{(C-P)} = 4.9 Hz), 145.51 (d, *J*_{(C-P)} = 8.1 Hz), 158.41, 173.29, 176.31; ³¹P-NMR (CD₃OD, 202 MHz): δ 3.18 (one diastereoisomer as α-isomer), 2.70 (one diastereoisomer as β-isomer), 2.57 (one diastereoisomer as β-isomer); MS (ES+) *m*/*z* 583.20 (MNa⁺).

### Example 35:

### Preparation of 2-acetamido-2-deoxy-3-O-[4-methoxyphenyl(isopropoxy-L-prolinyl)]phosphoramidate-D-glucopyranose (compound 42) and 2-acetamido-2-deoxy-4-O-[4-methoxyphenyl(isopropoxy-L-prolinyl)]phosphoramidate-D-glucopyranose (compound 43)

Prepared according to general procedure of Example 5 (addition at room temperature), from *N*-acetyl-D-glucosamine and 4-methoxyphenyl(isopropoxy-L-prolinyl)phosphorochloridate. The crude product was purified by flash chromatography on silica gel (under gradient elution system of dichloromethane : methanol, 100:0 - 97:3). Compound 43 was isolated as a white solid.
¹H-NMR (CD₃OD, 500 MHz), mixture of two diastereoiomers, one major in α form and one minor in β form: δ 7.12 (2H, d, *J* = 9.3 Hz), 7.08 (2H, d, *J* = 9.3 Hz), 6.91 (2H, d, *J* = 9.3 Hz), 6.89 (2H, d, *J* = 9.3 Hz), 5.12 (1H, d, *J* = 3.7 Hz), 5.03 (1H, m), 4.64 (1H, m), 4.51 (1H, m), 4.42 (1H, m), 4.28 (1H, m), 4.04 (2H, m), 3.79 (3H, s), 3.48 (1H, m), 3.49 (1H, m), 3.37 (1H, m), 2.21 (1H, m), 2.06-1.98 (3H, m), 1.68 (3H, s), 1.37 (6H, m); ¹³C-NMR (CD₃OD, 125 MHz): δ 21.89, 22.77, 26.12 (d, *J*_{(C-P)} = 8.4 Hz), 32.32 (d, *J*_{(C-P)} = 8.6 Hz), 48.49, 54.86, 56.09, 62.18 (d, *J*_{(C-P)} = 6.5 Hz), 65.46, 70.09, 70.60, 71.85, 73.42, 79.39 (d, *J*_{(C-P)} = 7.0 Hz), 82.48 (d, *J*_{(C-P)} = 7.0 Hz), 92.70, 96.77, 115.77, 122.35, 145.51 (d, *J*_{(C-P)} = 7.5 Hz), 158.49, 173.37, 175.67, 176.46; ³¹P-NMR (CD₃OD, 202 MHz): δ 3.17 (one diastereoisomer as α-isomer), 2.52 (one diastereoisomer as α-isomer), 2.23 (one diastereoisomer as β-isomer); MS (ES+) *m*/*z* 547.13 (MH⁺).

Further elution with dichloromethane : methanol 97:3 afforded compound 42.
¹H-NMR (CD₃OD, 500 MHz), mixture of two diastereoisomers, one major in α form and one minor in β form: δ 7.00 (2H, m), 6.78 (2H, m), 5.01 (1H, d, *J* = 3.7 Hz), 4.90 (1H, m), 4.51 (2H, m), 4.32 (1H, m), 4.15 (1H, m), 4.11 (1H, m), 3.90 (1H, m), 3.76 (2H, m), 3.67 (5H, m), 3.53 (1H, t, *J* = 9.7 Hz), 3.50 (1H, t, *J* = 9.71 Hz), 3.44 (1H, m), 3.22 (1H, m, overlap with the solvent), 2.07 (1H, m) 1.76 (6H, s), 1.68 (3H, s), 1.35 (6H, m); ¹³C-NMR (CD₃OD, 125 MHz): δ 21.90, 21.99, 22.88, 23.18, 26.20 (d, *J*_{(C-P)} = 8.4 Hz), 26.13 (d, *J*_{(C-P)} = 9 Hz), 32.29 (d, *J*_{(C-P)} = 8.6 Hz), 32.36 (d, *J*_{(C-P)} = 8.7 Hz), 54.83, 55.01, 56.14, 56.25, 62.33, 66.50, 62.61, 70.53, 70.60, 71.02, 71.75, 73.42, 77.70, 79.83 (d, *J*_{(C-P)} = 7.0 Hz), 82.48 (d, *J*_{(C-P)} = 7.0 Hz), 92.65, 96.77, 115.78, 122.08 (d, *J*_{(C-P)} = 4.5 Hz), 122.29 (d, *J*_{(C-P)} = 4.5 Hz), 145.5 (d, *J*_{(C-P)} = 7.5 Hz), 158.49, 173.37, 175.90; ³¹P-NMR (CD₃OD, 202 MHz): δ 3.27 (one diastereoisomer as α-isomer), 2.64 (one diastereoisomer as β-isomer); MS (ES+) m/z 547.13 (MH⁺).

### Example 36:

### Preparation of benzyl 2-acetamido-2-deoxy-4,6-O-benzylidene-3-O-[4-methoxyphenyl(ethoxyL-norvalinyl)]phosphoramidate-D-glucopyranoside (compound 44)

### 1. Preparation of benzyl 2-acetamido-2-deoxy-4,6-O-benzylidene-D-glucopyranoside.

To a solution of 1-*O*-benzyl-*N*-acetyl-D-glucosamine (5.0g, 16.07 mmol) in dry DMF (50 ml), *p*-toluenesulphonic acid (pTsOH) in catalytic quantities and benzaldehyde dimethylacetal (PHCH(OMe)₂) (4.8 ml, 32.12 mmol) was added and the mixture was subjected to reduced pressure (5 minutes each 1 hour period) during the first four hours whilst heated at 55°C, the mixture was then left stirring overnight at 55°C. The reaction was then neutralised with saturated NaHCO₃ solution, poured in to ice water and then kept stirring for 5 hours. The mixture was filtered and washed twice with 50% methanol in water and ether. The filter cake was dissolved in hot methanol and water was added to the hot methanol solution, it was then slowly cooled down to room temperature overnight. The precipitate was filtered, washed with cool methanol and ether and dried under high vacuum to give product (3.2 g, 50% yield) as a mixture of α and β anomers (ratio: 8.0: 1.0).
¹H-NMR ((CD₃)₂SO, 500 MHz): δ 8.00 (1H, d, *J* = 8.0 Hz), 7.92 (1H, d, *J* = 8.4 Hz), 7.44- 7.26 (10H, m), 5.59 (1H, s), 5.39, 5.31 (1H, bs), 5.31 (1H, *J* = 5.0 Hz), 4.78 (1H, d, *J* = 3.5 Hz), 4.68 (1H, d, *J* = 12.5 Hz), 4.58 (1H, d, *J* = 7.6 Hz), 4.48 (1H, d, *J* = 12.5 Hz), 4.12 (1H, d, *J* = 9.0 Hz), 4.11 (1H, d, *J* = 9.0 Hz), 3.95- 3.55 (3H, under solvent), 3.53- 3.47 (1H, m), 1.85 (3H, s), 1.83 (3H, s).

### 2. Preparation of benzyl 2-acetamido-2-deoxy-4,6-O-benzylidene-3-O-[4-methoxyphenyl(ethoxy-L-norvalinyl)]phosphoramidate-D-glucopyranoside (compound 44)

Prepared according to general procedure of Example 6, from p-methoxyphenyl(ethoxy-L-norvalinyl) phosphorochloridate (prepared according general procedure of Example 4) and benzyl-4,6-O-benzylidene-N-acetyl-D-glucosamine, to give 360.6 mg (69% yield) of compound 44.
¹H-NMR (CDCl₃, 500 MHz): δ 7.44- 7.36 (2H), 7.28- 7.16 (8H), 6.96- 6.85 (2H, m), 6.53- 6.40 (2H, m), 5.46 (1H, s), 5.45 (1H, bs), 5.42 (1H, s), 4.98 (1H, d, *J* = 3.0 Hz), 4.97 (1H, d, *J* = 3.0 Hz), 4.88- 4.78 (1H, m), 3.75- 4.68 (1H, m), 4.68- 4.63 (2H, m), 4.61 (1H, d, *J* = 12.0 Hz), 4.59 (1H, d, *J* = 12.0 Hz), 4.40 (1H, app s), 4.38 (1H, app s), 4.33- 4.26 (1H, m), 4.25- 4.18 (2H, m), 4.17- 4.12 (1H, m), 4.08- 3.98 (2H, m), 3.90- 3.81 (3H, m), 3.80- 3.73 (1H, m), 3.71- 3.60 (2H, m), 3.60 (3H, s), 3.59 (3H, s), 3.57 (3H, s), 3.54 (3H, s), 3.46- 3.39 (1H, m), 1.93 (3H, s), 1.87 (3H, s), 1.86 (3H, s), 1.82 (3H, s), 1.60- 1.46 (1H, m), 1.46- 1.36 (1H, m), 1.28- 1.06 (5H, m), 0.81- 0.69 (3H, m)¹³C-NMR (CDCl₃, 125 MHz): δ 173.80 (d, *J_{(C-P)}* = 5.5 Hz), 173.37, 172.93, 172.80 (d, *J_{(C-P)}* = 6.5 Hz), 171.15, 170.68, 170.61, 156.36, 156.32, 144.57 (d, *J_{(C-P)}* = 7.1 Hz), 144.50 (d, *J_{(C-P)}* = 7.1 Hz), 137.43, 137.08, 136.95, 136.82, 128.18, 128.15, 128.13, 128.04, 128.03, 127.92, 127.65, 127.44, 126.44, 126.38, 126.33, 121.26 (d, *J_{(C-P)}* = 5.0 Hz), 120.99, 120.91 (d, *J_{(C-P)}* = 5.0 Hz), 120.75 (d, *J_{(C-P)}* = 5.0 Hz), 114.37, 114.31, 102.07, 101.62, 101.56, 101.44, 100.79, 100.71, 97.49, 97.42, 80.39, 80.20 (d, *J_{(C-P)}* = 3.0 Hz), 80.12 (d, *J_{(C-P)}* = 3.7 Hz), 79.97, 74.34 (d, *J_{(C-P)}* = 6.3 Hz), 73.86 (d, *J_{(C-P)}* = 6.3 Hz), 71.21, 71.15, 70.22, 70.17, 68.81, 68.67, 65.62, 65.53, 63.12, 63.11, 61.58, 61.45, 61.30, 61.24, 55.52, 55.50, 54.33, 54.25, 53.61, 53.60, 36.40, 36.38, 23.34, 23.29, 23.08, 23.04, 18.11, 18.06, 14.17, 14.10, 13.58, 13.53; ³¹P-NMR (CDCl₃, 202 MHz): δ 4.04, 3.74 (α form, ratio 1.1 : 1.0) and 5.18, 5.16 (β form, ratio 1.1: 1.0). Ratio α: β forms 6.7: 1.0.

### Example 37:

### Preparation of 2-acetamido-2-deoxy-3-O-[4-methoxyphenyl(ethoxy-L-norvalinyl)]phosphoramidate-D-glucopyranose (compound 45)

Prepared as a white solid (114.9 mg, 77%) according to general procedure of Example 7, from benzyl 2-acetamido-2-deoxy-4,6-*O*-benzylidene-3-*O*-[4-methoxyphenyl(ethoxy-L-norvalinyl)]phosphoramidate-D-glucopyranoside (compound 44).
¹H-NMR (CD₃OD, 500 MHz): δ 7.09- 7.06 (2H, m), 7.01- 6.90 (2H, m), 6.80- 6.76 (2H, m), 6.68- 6.58 (2H, m), 5.04 (1H, d, *J* = 3.3 Hz), 5.02 (1H, d, *J* = 3.3 Hz), 4.65 (1H, d, *J* = 8.0 Hz), 4.64 (1H, d, *J* = 8.1 Hz), 4.52- 4.45 (1H, m), 4.41- 4.34 (1H, m), 4.08- 3.99 (2H, m), 3.99- 3.93 (1H, m), 3.81- 3.58 (4H, m), 3.67 (3H, s), 3.66 (3H, s), 3.56- 3.47 (1H, m), 3.28- 3.24 (1H, m), 1.88 (3H, s), 1.87 (3H, s), 1.86 (3H, s), 1.84 (3H, s), 1.62- 1.41 (1H, m), 1.29- 1.10 (5H, m), 0.82- 0.72 (3H, m);¹³C-NMR (CD₃OD, 125 MHz): δ 175.35 (d, *J_{(C-P)}* = 3.6 Hz), 174.68, 173.52, 173.48, 172.87, 158.38, 158.26, 145.92 (d, *J_{(C-P)}* = 7.8 Hz), 122.57 (d, *J_{(C-P)}* = 4.0 Hz), 122.47, 115.58, 115.56, 96.55, 96.36, 92.69, 92.58, 82.82, 82.46 , 80.96 (d, *J_{(C-P)}* = 7.6 Hz), 80.29 (d, *J_{(C-P)}* = 7.0 Hz), 77.44 (d, *J_{(C-P)}* = 6.7 Hz), 77.44 (d, *J(_{C-P)}* = 6.3 Hz), 70.96, 62.60, 62.47, 62.31, 56.13, 55.96, 55.87, 54.84 (d, *J_{(C-P)}* = 4.4 Hz), 54.71 (d, *J_{(C-P)}* = 3.6 Hz), 37.49 (d, *J_{(C-P)}* = 5.8 Hz), 37.31 (d, *J_{(C-P)}* = 6.7 Hz), 23.29, 22.99, 19.64, 19.57, 19.52, 19.43, 14.52, 14.00, 13.91; ³¹P-NMR (CD₃OD, 202 MHz): δ 5.02, 4.12 (α form, ratio 1.1: 1.0) and 3.73, 3.11 (β form, ratio 1.2: 1.0). Ratio α: β forms 5.3: 1.0; MS (ES+) *m*/*z* 557.18.

### Example 38:

### Preparation of benzyl 2-acetamido-2-deoxy-4,6-O-benzylidene-3-O-[4-methoxyphenyl(tert-butoxy-L-prolinyl)]phosphoramidate-D-glucopyranoside (compound 46)

Prepared as a white foamy solid (436.7 mg, 47%), according to general procedure of Example 6, from 4-methoxyphenyl(*tert*-butoxy-L-prolinyl)phosphorochloridate (prepared according general procedure of Example 4) and benzyl-4,6-*O*-benzylidene-*N*-acetyl-D-glucosamine.
¹H-NMR (CD₃OD, 500 MHz): δ 8.08, 8.06 (2bs), 8.00, 7.98 (2bs), 7.41- 7.38 (2H), 7.32- 7.16 (8H), 6.84- 6.77 (2H, m), 6.56- 6.51 (2H, m), 5.51 (1H, s), 5.48 (1H, s), 4.78 (1H, d, *J* = 3.3 Hz), 4.74- 4.58 (3H, m), 4.49- 4.41 (1H, m), 4.27- 4.18 (1H, m), 4.09(1H, dd, *J* = 10.0, 4.8 Hz), 3.95- 3.89 (1H, m), 3.88- 3.77 (1H, m), 3.76- 3.62 (2H), 3.59 (3H, s), 3.58 (3H, s), 3.18- 3.08 (2H, m), 1.94- 1.83 (1H, m), 1.88 (3H, s), 1.87 (3H, s), 1.78-1.69 (2H, m), 1.64- 1.57 (1H, m), 1.31 (9H, s); ¹³C-NMR (CD₃OD, 125 MHz): δ 174.16, 173.50, 173.42, 158.01, 145.76, 138.92, 138.50, 130.08, 129.57, 129.16, 128.84, 127.74, 127.56, 122.02 (d, *J_{(C-P)}* = 4.4 Hz), 115.70, 115.45, 102.97, 102.83, 102.05, 98.59, 82.43, 81.64, 81.22, 76.60 (d, *J_{(C-P)}* = 7.4 Hz), 72.27, 71.03, 69.73, 69.58, 67.15, 64.40, 62.12 (d, *J_{(C-P)}* = 6.4 Hz), 56.06, 54.43, 54.40, 32.34 (d, *J_{(C-P)}* = 8.7 Hz), 28.31, 26.00 (d, *J_{(C-P)}* = 8.3 Hz), 23.34, 22.92; ³¹P-NMR (CD₃OD, 202 MHz): δ 0.95 (α form), 0.49 (β form). Ratio α: β forms 4.7: 1.0.

### Example 39:

### Preparation of 2-acetamido-2-deoxy-3-O-[4-methoxyphenyl(tert-butoxy-L-prolinyl)]phosphoramidate-D-glucopyranose (compound 47)

Prepared according to general procedure of Example 7, from benzyl 2-acetamido-2-deoxy-4,6-*O*-benzylidene-3-*O*-[4-methoxyphenyl(*tert*-butoxy-L-prolinyl)]phosphoramidate-D-glucopyranoside (compound 46).
¹H-NMR (CD₃OD, 500 MHz), a mixture of two diastereoisomers, one in the α and one in the β form: δ 7.16- 7.14 (2H, m), 6.79- 6.78 (2H, m), 4.99 (1H, d, *J* = 3.2 Hz), 4.64 (1H, d, *J* = 8.1 Hz), 4.42- 4.35 (1H, m), 4.27- 4.21 (1H, m), 4.07- 3.98 (3H, m), 3.79- 3.74 (1H, m), 3.73- 3.59 (1H, m), 3.67 (3H, s), 3.55- 3.50 (1H, m), 3.47- 3.42 (1H, m), 3.31- 3.08 (2H, m), 2.27- 2.19 (1H, m), 1.91 (3H, s), 1.90 (3H, s), 1.91- 1.77 (3H, m), 1.33 (9H, s); ¹³C-NMR (CD₃OD, 125 MHz): δ 174.30, 171.17, 158.26, 146.01, 122.31 (d, *J_{(C-P)}* = 4.2 Hz), 115.60, 96.29, 92.83, 84.78, 82.52, 81.02 (d, *J_{(C-P)}* = 7.8 Hz), 72.94, 70.80, 62.50, 69.73 (d, *J_{(C-P)}* = 6.4 Hz), 62.45, 56.13, 54.57, 54.54, 47.48, 32.40 (d, *J_{(C-P)}* = 8.8 Hz), 30.16, 28.28, 28.24 (d, *J_{(C-P)}* = 8.3 Hz), 23.35, 23.03; ³¹P-NMR (CD₃OD, 202 MHz): δ 2.48 (α form), 1.85 (β form). Ratio α:β forms 3.6: 1.0; MS (ES+) *m*/*z* 583.20 (MNa⁺).

### Example 40:

### Preparation of 2-acetamido-2-deoxy-3-O-[4-methoxyphenyl(2-butoxy-L-prolinyl)]phosphoramidate-D-glucopyranose (compound 48) and 2-acetamido-2-deoxy-4-O-[4-methoxyphenyl-(2-butoxy-L-prolinyl)]phosphoramidate-D-glucopyranose (compound 49)

Prepared according to general procedure of Example 5 (addition at room temperature), from *N*-acetyl-D-glucosamine and 4-methoxyphenyl(2-butoxy-L-prolinyl)phosphorochloridate. The crude product was purified by flash chromatography on silica gel (under gradient elution system of dichloromethane : methanol 100:0 - 97:3). Compound 49 was isolated as a white solid.
¹H-NMR (CD₃OD, 500 MHz), mixture of two diastereoiomers, one major in α form and one minor in β form: δ 7.12 (2H, d, *J* = 9.1 Hz), 7.08 (2H, d, *J* = 9.1 Hz), 6.91 (2H, d, *J* = 9.1 Hz), 6.89 (2H, d, *J* = 9.1 Hz), 5.12 (1H, d, *J* = 3.7 Hz), 5.04 (1H, m), 4.67 (1H, m), 4.53 (1H, m), 4.47 (1H, m), 4.31 (1H, m), 4.04 (2H, m), 3.75 (3H, s), 3.46 (1H, m), 3.49 (1H, m), 3.37 (1H, m), 2.21 (1H, m), 2.06-1.98 (3H, m), 1.68 (3H, s), 1.40 (2H, m), 1.09 (3H, d, *J* = 6.25 Hz); ¹³C-NMR (CD₃OD, 125 MHz): δ 10.04, 19.57, 22.79, 26.12 (d, *J*_{(C-P)} = 8.4 Hz), 29.81, 32.30 (d, *J*_{(C-P)} = 8.6 Hz), 48.49, 54.75, 56.09, 62.18 (d, *J*_{(C-P)} = 6.5 Hz), 65.47, 71.89, 73.42, 74.56, 74.58, 79.41 (d, *J*_{(C-P)} = 7.0 Hz), 82.48 (d, *J*_{(C-P)} = 7.0 Hz), 92.70, 96.77, 115.77, 122.35, 145.51 (d, *J*_{(C-P)} = 7.5 Hz), 158.49, 173.37, 175.67, 176.46; ³¹P-NMR (CD₃OD, 202 MHz): δ 3.21 (one diastereoisomer as α-isomer), 2.49 (one diastereoisomer as α-isomer), 2.21 (one diastereoisomer as β-isomer); MS (ES+) *m*/*z* 583.20 (MNa⁺).

Further elution with dichloromethane: methanol 97:3 afforded compound 48.
¹H-NMR (CD₃OD, 500 MHz), mixture of two diastereoiomers, one major in α form and one minor in β form: δ 7.15 (2H, m), 6.78 (2H, d, *J* = 9.1 Hz), 4.99 (1H, d, *J* = 3.2 Hz ), 4.71 (1H, m), 4.51 (1H, d, *J* = 3.2 Hz), 4.39 (1H, m), 4.24 (1H, m), 4.12 (1H, m) 4.05 (1H, m), 3.76 (1H, m), 3.67 (5H, m), 3.51 (1H, m), 3.44 (1H, m), 3.29 (1H, m), 3.20 (1H, m, overlap with the solvent), 2.11 (1H, m), 1.91(3H, s), 1.87 (3H, s), 1.83 (3H), 1.40 (2H, m), 1.09 (3H, d, *J* = 6.25 Hz), 1.06 (3H, d, *J* = 6.2 Hz), 0.78 (3H, q, *J* = 8.0 Hz); ¹³C-NMR (CD₃OD, 125 MHz): δ 10.05, 10.09, 19.61, 19.72, 23.02, 23.34, 26.25 (d, *J*_{(C-P)} = 8.4 Hz), 29.79, 32.50 (d, *J*_{(C-P)} = 8.6 Hz), 32.58 (d, *J*_{(C-P)} = 8.7 Hz), 48.80 (d, *J*_{(C-P)} = 4.5 Hz), 54.55 (d, *J*_{(C-P)} = 3.9 Hz), 56.11, 61.96 (d, *J*_{(C-P)} = 6.7 Hz), 66.35, 62.61, 70.82 (d, *J*_{(C-P)} = 3.1 Hz), 70.91 (d, *J*_{(C-P)} = 3.1 Hz), 72.96, 74.55, 74.58, 77.52, 81.05 (d, *J*_{(C-P)} = 7.0 Hz), 82.87 (d, *J*_{(C-P)} = 8.7 Hz), 92.83, 96.29, 115.58, 122.34 (d, *J*_{(C-P)} = 5.1 Hz), 145.95 (d, *J*_{(C-P)} = 7.5 Hz), 158.27, 173.45, 174.73; ³¹P-NMR (CD₃OD, 202 MHz): δ 2.47 (one diastereoisomer as α-isomer), 2.38 (one diastereoisomer as β-isomer); MS (ES+) *m*/*z* 583.20 (MNa⁺).

### Example 41:

### Preparation of 2-acetamido-2-deoxy-3-O-[4-methoxyphenyl(tert-butoxy-L-valinyl)]phosphoramidate-D-glucopyranose (compound 50) and 2-acetamido-2-deoxy-4-O-[4-methoxyphenyl(tert-butoxy-L-valinyl)]phosphoramidate-D-glucopyranose (compound 51)

Prepared according to general procedure of Example 5 (addition at room temperature), from 4-methoxyphenyl(*tert*-butoxy-L-valinyl)phosphorochloridate and *N*-acetyl-D-glucosamine, to give a mixture of compound 50 and compound 51.
¹H-NMR (CD₃OD, 500 MHz), mixture of four diastereoiomers, two maior in α form and two minor in β form: δ 7.02 (2H, d, *J* = 9.32 Hz), 7.00 (2H, d, *J* = 8.9 Hz), 6.77 (2H, m), 5.00 (1H, d, *J* = 3.15 Hz), 4.56 (1H, d, *J* = 8.6 Hz), 4.46 (1H, m) 4.27 (1H, m) 3.98 (1H, dd, *J* = 10.4 and 3.3 Hz), 3.85 (3H, m), 3.78 (1H, m), 3.67 (5H, m), 3.57 (1H, m), 3.50 (2H, m), 1.89 (1H, m), 1.77 (3H, s), 1.73 (3H, s), 1.37, 1.36, 1.35, 0.82 (3H, d, *J* = 7.0 Hz), 0.80 (3H, d, *J* = 7.0 Hz), 0.77 (3H, d, *J* = 7.0 Hz), 0.76 (3H, d, *J* = 7.0 Hz); ¹³C-NMR (CD₃OD, 125 MHz): δ 17.97, 18.06, 19.43, 19.48, 22.73, 22.93, 23.26, 28.43, 28.46, 33.20 (d, *J*_{(C-P)}= 6.97 Hz), 33.40 (d, *J*_{(C-P)}= 6.39 Hz), 49.93, 54.57 (d, *J*_{(C-P)}= 3.75 Hz), 55.03, 56.15, 62.24, 62.29, 62.50, 62.61, 71.32 (d, *J*_{(C-P)}= 2.7 Hz), 71.45 (d, *J*_{(C-P)}= 5.7 Hz), 71.67, 73.12, 77.54, 77.78 (d, *J*_{(C-P)}= 6.81 Hz), 80.00 (d, *J*_{(C-P)}= 6.46 Hz), 82.29 (d, *J*_{(C-P)}= 6.86 Hz) 92.41, 92.76, 96.69, 96.78, 115.64, 122.35 (d, *J*_{(C-P)} = 4.6 Hz), 122.46 (d, *J*_{(C-P)} = 4.6 Hz), 122.61 (d, *J*_{(C-P)} = 4.58 Hz), 145.87 (d, *J*_{(C-P)}= 7.62 Hz), 145.99 (d, *J*_{(C-P)}= 7.54 Hz), 158.28, 158.43, 173.48, 173.70 (d, *J*_{(C-P)} = 4.6 Hz), 173.71 (d, *J*_{(C-P)} = 4 Hz), 173.79 (d, *J*_{(C-P)} = 4 Hz); ³¹P-NMR (CD₃OD, 202 MHz): δ 6.17, 5.99, 5.50; MS (ES+) *m*/*z* 585.21 (MNa⁺).

### Example 42:

### Preparation of benzyl 2-acetamido-2-deoxy-4,6-O-benzylidene-3-O-[4-methoxyphenyl(methoxy-L-valinyl)]phosphoramidate-D-glucopyranoside (compound 52)

Prepared according to general procedure of Example 6, from *p*-methoxyphenyl (methoxy-L-valinyl)phosphorochloridate and benzyl-4,6-*O*-benzylidene-*N*-acetyl-D-glucosamine, to give a white foamy solid (800.0 mg, 38%).
¹H-NMR (CD₃OD, 500 MHz): δ 7.91, 7.89 (2bs), 7.82, 7.80 (2bs ), 7.39- 7.13 (10H), 7.02- 6.54 (4H, m), 5.48 (1H, s), 5.45 (1H, s), 4.78 (1H, d, *J* = 3.3 Hz), 4.69- 4.57 (3H, m), 4.43- 4.38 (1H, m), 4.22- 4.12 (1H, m), 4.08 (1H, dd, *J* = 10.0, 5.0 Hz), 3.84- 3.77 (1H, m), 3.71- 3.36 (9H, m), 2.04- 1.75 (1H, m), 1.87 (3H, s), 1.76 (3H, m), 0.82- 0.74 (3H, m), 0.63- 0.49 (3H, m); ³¹P-NMR (CD₃OD, 202 MHz): δ 3.17, 3.12 (α form, ratio 1.0: 3.4) and 2.33, 2.29 (β form, ratio 1.3: 1.0). Ratio α: β forms 20.2: 1.0.

### Example 43:

### Preparation of 2-acetamido-2-deoxy-3-O-[4-methoxyphenyl(methoxy-L-valinyl)]phosphoramidate-D-glucopyranose (compound 53)

Prepared according to general procedure of Example 7, from benzyl 2-acetamido-2-deoxy-4,6-*O*-benzylidene-3-*O*-[4-methoxyphenyl(methoxy-L-valinyl)]phosphoramidate-D-glucopyranoside (compound 52), to give 0.010 g (29% yield) of compound 53.
¹H-NMR (CD₃OD, 500 MHz), a mixture of 4 diastereoisomers in the α and β form: δ 7.90 (2bs), 7.08- 6.59 (4H, m), 5.02 (1H, d, *J* = 3.4 Hz), 5.00 (1H, d, *J* = 3.4 Hz), 4.66 (1H, d, *J* = 8.4 Hz), 4.53- 4.47 (1H, m), 4.44- 4.37 (1H, m), 3.99- 3.96 (1H, m), 3.82- 3.49 (8H, m), 3.67 (3H, s), 1.97- 1.82 (1H, m), 1.86 (3H, s), 1.83 (3H, m), 0.84- 0.76 (6H, m); ³¹P-NMR (CD₃OD, 202 MHz): δ 4.33 (α form) and 3.36 (β form). Ratio α: β forms 6.6: 1.0.

### Example 44:

### Preparation of 2-acetamido-2-deoxy-3-O-[4-methoxyphenyl(cyclohexyloxy-L-prolinyl)]phosphoramidate-D-glucopyranose (compound 54) and 2-acetamido-2-deoxy-4-O-[4-methoxyphenyl(cyclohexyloxy-L-prolinyl)]phosphoramidate-D-glucopyranose (compound 55)

Prepared according to general procedure of Example 5 (addition at room temperature), from *N*-acetyl-D-glucosamine and 4-methoxyphenyl(cyclohexyloxy-L-prolinyl)phosphorochloridate. The crude product was purified twice by flash chromatography on silica gel (under gradient elution system of dichloromethane : methanol, 100:0 - 97:3). Compound 55 was isolated as a white solid.
¹H-NMR (CD₃OD, 500 MHz), mixture of two diastereoisomers, one major in α form and one minor in β form: δ 7.18 (2H, m), 7.09 (2H, m), 6.98 (2H, m), 6.81 (2H, m), 5.17 (1H, d, *J* = 3.7 Hz), 4.78 (1H, m), 4.67 (1H, m), 4.55 (1H, m), 4.49 (1H, m), 4.38 (1H, m), 4.04 (2H, m), 3.81 (3H, s), 3.80 (3H, s), 3.62 (1H, m), 3.46 (1H, m), 3.38 (1H, m), 2.21 (1H, m), 2.06-1.98 (3H, m), 1.79 (4H, m), 1.78 (3H, s), 1.38 (6H, m); ¹³C-NMR (CD₃OD, 125 MHz): δ 24.68, 26.13 (d, *J*_{(C-P)} = 8.2 Hz), 26.45, 32.44, 48.50 (d, *J*_{(C-P)} = 3.7 Hz, overlap with the solvent), 54.93 (d, *J*_{(C-P)} = 3.0 Hz), 56.11, 56.16, 62.01 (d, *J*_{(C-P)} = 6.5 Hz), 66.84, 70.18, 71.80, 74.41, 79.88 (d, *J*_{(C-P)} = 6.4 Hz), 92.70, 97.65, 115.78, 122.29 (d, *J*_{(C-P)} = 4.4 Hz), 145.51 (d, *J*_{(C-P)} = 8.1 Hz), 158.43, 173.25, 175.25, 176.31; ³¹P-NMR (CD₃OD, 202 MHz): δ 3.16 (one diastereoisomer as α-isomer), 2.53 (one diastereoisomer as β-isomer), 2.24 (one diastereoisomer as β-isomer); MS (ES+) *m*/*z* 587.11 (MNa⁺).

Further elution with dichloromethane: methanol 97:3 afforded compound 54.

¹H-NMR (CD₃OD, 500 MHz), mixture of two diastereoisomers, one major in α form and one minor in β form: δ 7.15 (2H, m), 7.01 (2H, d, *J* = 9.2 Hz), 6.78 (2H, d, *J* = 9.2 Hz), 6.70 (2H, d, *J* = 9.2 Hz), 4.98 (1H, d, *J* = 3.9 Hz), 4.62 (2H, m), 4.39 (1H, m), 4.22 (1H, m), 4.11 (1H, m) 3.99 (1H, m), 3.75 (1H, m), 3.67 (5H, m), 3.51 (1H, t, *J* = 9.6 Hz), 3.44 (1H, d, *J* = 9.6 Hz), 3.30 (1H, m), 3.20 (1H, m, overlap with the solvent), 2.10 (1H, m) 1.91 (3H, s), 1.90 (3H, s), 1.81 (3H), 1.63 (4H, m), 1.28 (6H, m); ¹³C-NMR (CD₃OD, 125 MHz): δ 23.01, 23.33, 24.62, 26.10 (d, *J*_{(C-P)} = 8.2 Hz), 26.25 (d, *J*_{(C-P)} = 8.2 Hz), 26.46, 32.46, 48.80 (d, *J*_{(C-P)} = 3.7 Hz, overlap with the solvent), 54.59 (d, *J*_{(C-P)} = 3.8 Hz), 56.05, 56.09, 61.96 (d, *J*_{(C-P)} = 7.3 Hz), 66.46, 62.61, 70.80 (d, *J*_{(C-P)} = 3.1 Hz), 70.90 (d, *J*_{(C-P)} = 3.1 Hz), 72.95, 74.44, 74.74, 77.52, 81.05 (d, *J*_{(C-P)} = 7.0 Hz), 82.87 (d, *J*_{(C-P)} = 8.7 Hz), 92.83, 96.29, 115.19, 115.58, 122.32 (d, *J*_{(C-P)} = 4.7 Hz), 122.56 (d, *J*_{(C-P)} = 4.3 Hz), 145.94 (d, *J*_{(C-P)} = 7.1 Hz), 158.28, 173.46, 173.78, 174.50 (d, *J*_{(C-P)}= 1.5 Hz), 175.06 (d, *J*_{(C-P)} = 1.5 Hz); ³¹P-NMR (CD₃OD, 202 MHz): δ 2.42 (one diastereoisomer as α-isomer), 1.80 (one diastereoisomer as β-isomer); MS (ES+) *m*/*z* 587.11 (MNa⁺).

### Example 45:

### Preparation of 2-acetamido-2-deoxy-3-O-[4-methoxyphenyl(cyclohexyloxy-L-valinyl)]phosphoramidate-D-glucopyranose (compound 56) and 2-acetamido-2-deoxy-4-O-[4-methoxyphenyl(cyclohexyloxy-L-valinyl)lphosphoramidate-D-glucopyranose (compound 57)

Prepared according to general procedure of Example 5 (addition at room temperature), from 4-methoxyphenyl(cyclohexyloxy-L-valinyl)phosphorochloridate and N-acetyl-D-glucosamine, to give a mixture of compound 56 and compound 57.
¹H-NMR (CD₃OD, 500 MHz): δ 7.67, 7.56, 7.53 (bs), 7.10- 7.00 (2H, m), 6.79- 6.75 (2H, m), 5.03 (1H, d, *J* = 3.0 Hz), 4.71- 4.63 (1H, m), 4.58 (1H, d, *J* = 8.0 Hz), 4.53- 4.47 (1H, m), 4.35-4.26 (1H, m), 4.04- 3.98 (1H, m), 3.89- 3.87 (1H, m), 3.82- 3.50 (5H, m), 3.65 (3H, s), 3.63 (3H, s), 2.01- 1.92 (1H, m), 1.90 (3H, s), 1.80 (3H, s), 1.76 (3H, s), 1.78- 1.69 (2H, m), 1.67-1.59 (2H, m), 1.47- 1.16 (6H, m), 0.81 (3H, d, *J* = 7.0 Hz), 0.79 (3H, d, *J* = 6.5 Hz); ¹³C-NMR (CD₃OD, 125 MHz): δ 173.82 (d, *J_{(C-P)}* = 3.7 Hz), 173.48, 158.43, 158.28, 145.98 (d, *J_{(C-P)}* = 7.3 Hz), 145.86 (d, *J_{(C-P)}* = 7.3 Hz), 122.62, 122.59, 122.47, 122.43, 122.36, 122.32, 115.64, 96.81, 96.68, 92.77, 92.40, 80.06 (d, *J_{(C-P)}*= 6.7 Hz), 77.83 (d, *J_{(C-P)}* = 6.1 Hz), 75.06, 73.09, 71.62, 71.48 (d, *J_{(C-P)}* = 6.3 Hz), 71.14, 62.50, 62.19, 61.86, 61.79, 56.17, 56.02, 54.56, 54.54, 33.31 (d, *J_{(C-P)}* = 6.4 Hz), 33.21 (d, *J_{(C-P)}* = 6.9 Hz), 32.59, 26.46, 24.72, 22.96, 22.77, 18.05, 19.54; ³¹P-NMR (CD₃OD, 202 MHz): δ 6.05 and 5.92, 5.41; MS (ES+) *m*/*z* 611.23. (MNa⁺).

### Example 46:

### Preparation of 2-acetamido-2-deoxy-3-O-[4-methoxyphenyl(2-butoxy-L-valinyl)]phosphoramidate-D-glucopyranose (compound 58) and 2-acetamido-2-deoxy-4-O-[4-methoxyphenyl(2-butoxy-L-valinyl)]phosphoramidate-D-glucopyranose (compound 59)

Prepared according to general procedure of Example 5, from 4-methoxyphenyl(2-butoxy-L-valinyl)phosphorochloridate and *N*-acetyl-D-glucosamine, to give a mixture of compound 58 and compound 59.
¹H-NMR (CD₃OD, 500 MHz): δ 7.52 (bs), 7.08- 7.00 (2H, m), 6.99- 6.75 (2H, m), 5.01 (1H, d, *J* = 3.0 Hz), 4.79- 4.71 (1H, m), 4.57 (1H, d, *J* = 8.5 Hz), 4.52- 4.46 (1H, m), 4.34- 4.43 (1H, m), 4.02- 3.96 (1H, m), 3.87- 3.48 (5H, m), 3.68, 3.66 (3H, 2s), 2.03- 1.92 (1H, m), 1.90 (3H, s), 1.78 (3H, s), 1.74 (3H, s), 1.61- 1.42 (2H, m), 1.16- 1.10 (3H, m), 0.87- 0.78 (6H, m); ¹³C-NMR (CD₃OD, 125 MHz): δ 174.13 (d, *J_{(C-P)}*= 4.2 Hz), 174.06 (d, *J_{(C-P)}*= 4.2 Hz), 173.77, 173.57, 173.48, 158.44, 158.30, 145.99 (d, *J_{(C-P)}* = 7.6 Hz), 145.88 (d, *J_{(C-P)}* = 7.6 Hz), 122.62 (d, *J_{(C-P)}*= 4.5 Hz), 122.48 (d, *J_{(C-P)}*= 4.5 Hz), 122.38 (d, *J_{(C-P)}*= 4.7 Hz), 115.65, 96.80, 96.69, 92.77, 92.41, 80.01 (d, *J_{(C-P)}*= 6.4 Hz), 77.88 (d, *J_{(C-P)}*= 6.2 Hz), 77.82 (d, *J_{(C-P)}*= 6.2 Hz), 75.00, 74.96, 74.94, 74.87, 73.10, 71.62, 71.50, 71.45, 71.11, 62.60, 62.58, 62.32, 62.20, 61.91, 61.82, 61.77, 61.67, 57.31, 56.15, 56.03, 54.65 (d, *J_{(C-P)}* = 3.5 Hz), 54.57 (d, = 3.0 Hz), 33.15 (d, *J_{(C-P)}*= 7.0 Hz), 29.84, 29.78, 23.25, 22.97, 22.92, 22.73, 19.84, 19.76, 19.59, 19.57, 19.51, 19.47, 18.01, 17.97, 17.91, 17.73, 10.25, 10.21, 10.17, 10.13; ³¹P-NMR (CD₃OD, 202 MHz): δ 6.15, 6.14; 5.93, 5.90, 5.48, 5.47, 4.43, 3.80; MS (ES+) *m*/*z* 585.21 (MNa⁺).

### Example 47:

### Preparation of 2-deoxy-2-(pent-4-enoylamino)-4-O-[4-methoxyphenyl(benzyloxy-L-valinyl)]phosphoramidate-D-glucopyranose (compound 60) and 2-deoxy-2-(pent-4-enoylamino)-3-O-[4-methoxyphenyl(benzyloxy-L-valinyl)]phosphoramidate-D-glucopyranose (compound 61)

Prepared according to general procedure of Example 5, from 2-deoxy-2-(pent-4-enoylamino)-D-glucopyranose (see Example 18) and 4-methoxyphenyl(benzyloxy-*L-*valinyl)phosphorochloridate. The crude product was purified twice by flash chromatography on silica gel . The first flash chromatography using a gradient of dichloromethane : methanol from 98:2 to 95:5 and the second column using EtOAc : methanol 95:5 like eluent afforded compound 60 in 1.5% yield.
¹H-NMR (CD₃OD, 500 MHz), mixture of two diastereoisomers, both in α form and in β form: δ 7.57 (m), 7.36 (d, *J* = 9.1 Hz), 7.09 (d, *J* = 9.1 Hz), 6.07 (m), 5.39 (AB system, *J* = 12.2 Hz), 5.32 (d, *J* = 2.1 Hz), 5.29 (d, *J* = 17.8 Hz), 5.20 (d, *J* = 10.4 Hz), 4.89 (d, *J* = 8.9 Hz), 4.56 ( m) 4.16 (m), 4.02 (m), 3.98 (m), 3.92 (m), 3.85 (m), 2.27 (m), 1.05 (d, *J* = 6.9 Hz), 1.05 (d, *J* = 7.0 Hz); ¹³C-NMR (CD₃OD, 125 MHz): δ 17.86, 19.44, 30.93, 33.17 (d, *J*_{(C-P)} = 6.6 Hz), 36.37, 55.90, 56.12, 61.12, 62.21, 68.05, 71.48 (d, *J*_{(C-P)}= 6.0 Hz), 71.57 (d, *J*_{(C-P)}= 2.44 Hz), 80.02 (d, *J*_{(C-P)} = 6.6 Hz), 92.41, 96.84, 115.63, 115.80, 122.61 (d, *J*_{(C-P)} = 4.80 Hz), 129.42, 129.63, 129.67, 137.22, 138.38, 145.83 (d, *J*_{(C-P)}= 7.6 Hz), 158.45, 174.24 (d, *J*_{(C-P)} = 3.6 Hz), 175.91; ³¹P-NMR (CD₃OD, 202 MHz): δ 6.11, 5.72; MS (ES+) *m*/*z* 659.23 (MNa⁺).

Further the first flash chromatography using a gradient of dichloromethane : methanol from 98:2 to 95:5, one fraction eluded was purified thrice by flash chromatography using EtOAc : methanol from 98:2 to 9 :1, EtOAc and dichloromethane : methanol 98:2 to 97:3 to give compound 61 in 5% yield.
¹H-NMR (CD₃OD, 500 MHz), mixture of two diastereoisomers, both in α form and in β form: δ 7.36 (m), 7.10 (d, *J* = 8.6 Hz), 6.87 (d, *J*= 8.6 Hz), 5.76 (m), 5.17 (s) 5.12 (s), 5.00 (d, *J* = 17.8 Hz), 4.93 (d, *J* = 10.4 Hz), 4.63 (m) 4.40 (d, *J* = 10.4 Hz) 4.11 (m), 3.88 (m), 3.76 (m), 3.64 (t, *J* = 10.1 Hz), 2.11 (m), 2.06 (m), 0.89 (d, *J* = 6.7 Hz), 0.85 (d, *J* = 6.7 Hz); ¹³C-NMR (CD₃OD, 125 MHz): δ 17.95, 19.44, 30.57, 33.28 (d, *J*_{(C-P)} = 6.6 Hz), 36.31, 36.36, 54.47 (d, *J*_{(C-P)} = 3.7 Hz), 54.55 (d, *J*_{(C-P)} = 3.72 Hz), 56.12, 61.62, 61.69, 62.47, 62.58, 68.01, 71.11 (d, *J*_{(C-P)}= 2.7 Hz), 73.03, 80.98 (d, *J*_{(C-P)} = 5.8 Hz), 92.80, 96.72 , 115.61, 115.70, 122.43 (d, *J*_{(C-P)} = 4.9 Hz), 122. 52 (d, *J*_{(C-P)} = 4.6 Hz), 129.43, 129.62, 129.65, 137.20, 138.33, 145.90 (d, *J*_{(C-P)}= 7.2 Hz), 158.30, 174.21 (d, *J*(_{C-P)} = 4.2 Hz), 175.51, 175.59; ³¹P-NMR (CD₃OD, 202 MHz): δ 6.09, 5.5 1; MS (ES+) *m*/*z* 659.23 (MNa⁺).

### Example 48:

### Preparation of 2-amino-2-deoxy-4-O-[4-methoxyphenyl(benzyloxy-L-valinyl)]phosphoramidate-D-glucopyranose (compound 62)

Prepared in 17% yield according to general procedure of Example 8, from 2-deoxy-2-(pent-4-enoylamino)-4-*O*-[4-methoxyphenyl(benzyloxy-*L*-valinyl)]phosphoramidate-*D-*glucopyranose (compound 60, Example 47).
¹H-NMR (CD₃OD, 500 MHz), mixture of two diastereoisomers, both in α form and in β form: δ 7.37 (m), 7.16 (m), 6.90 (m), 5.35 (d, *J* = 3.4 Hz), 5.17 (m), 4.82 (m, overlap with solvent), 4.32 (m), 4.06 (dd, *J =* 10.40 and 9.3 Hz), 3.94 (m) 3.74 (m), 3.07 (dd, *J* = 10.39 and 3.8 Hz), 2.83 (dd, *J* = 10.39 and 8.6 Hz), 2.07 (m), 0.84 (m); ¹³C-NMR (CD₃OD, 125 MHz): δ 17.96, 19.40, 33.24 (d, *J*_{(C-P)} = 6.6 Hz), 56.16, 56.23, 61.65, 61.96, 68.06, 70.58 (d, *J*_{(C-P)} = 2.1 Hz), 71.68 (d, *J*_{(C-P)} = 5.6 Hz), 77.13 (d, *J*_{(C-P)} = 6.2 Hz), 90.91, 95.30, 115.64, 115.69, 122.59 (d, *J*_{(C-P)} = 3.7 Hz), 129.47, 129.59, 129.66, 137.17, 145.64 (d, *J*_{(C-P)} = 7.1 Hz), 158.57, 174.21 (d, *J*_{(C-P)} = 3.3 Hz); ³¹P-NMR (CD₃OD, 202 MHz): δ 5.89, 5.66, 5.20; MS (ES+) *m*/*z* 577.19 (MNa⁺).

### Example 49:

### Preparation of 2-amino-2-deoxy-3-O-[4-methoxyphenyl(benzyloxy-L-valinyl)]phosphoramidate-D-glucopyranose (compound 63)

Prepared according to general procedure of Example 8, from 2-deoxy-2-(pent-4-enoylamino)-3-*O-*[4-methoxyphenyl(benzyloxy-L-valinyl)]phosphoramidate-*D*-glucopyranose (compound 61, Example 47).
¹H-NMR (CD₃OD, 500 MHz), mixture of two diastereoisomers, both in α form and in β form: δ 7.36 (m), 7.19 (d, *J* = 9.4 Hz), 6.90 (m, d, *J* = 9.4 Hz), 5.36 (d, *J* =3.5 Hz), 5.20 (m), 4.71 (m), 4.47 (m), 3.89 (m), 3.78, 3.65 (m), 3.22 (dd, *J* = 9.8 and 3.8 Hz), 2.91 (dd, *J =* 10.4 and 8.7 Hz), 2.07 (m), 0.82 (d, *J* = 7.1 Hz); ¹³C-NMR (CD₃OD, 125 MHz): δ 17.83, 19.33, 33.19 (d, *J*_{(C-P)} = 6.7 Hz), 55.73 (d, *J*_{(C-P)} = 3.5 Hz), 56.17, 61.80, 61.07, 62.27, 68.09, 70.28 (d, *J*_{(C-P)} = 3.5 Hz), 73.01, 80.44 (d, *J*_{(C-P)} = 6.4 Hz), 91.74, 96.03, 115.72, 122. 66 (d, *J*_{(C-P)} = 4.3 Hz), 129.47, 129.63, 129.66, 137.18, 145.76 (d, *J*_{(C-P)}= 7.3 Hz), 158.57, 174.28 (d, J_{*(*C-P)} = 2.9 Hz); ³¹P-NMR (CD₃OD, 202 MHz): δ 6.23, 6.08; MS (ES+) *m*/*z* 577.19 (MNa⁺).

### Example 50:

### Preparation of 2-acetamido-2-deoxy-3-O-[4-methoxyphenyl(methoxy-L-prolinyl)]phosphoramidate-D-glucopyranose (compound 64) and 2-acetamido-2-deoxy-4-O-[4-methoxyphenyl(methoxy-L-prolinyl)]phosphoramidate-D-glucopyranose (compound 65)

Prepared according to general procedure of Example 5 (addition at room temperature), from *N*-acetyl-D-glucosamine and 4-methoxyphenyl(metoxy-L-prolinyl)phosphorochloridate. The crude product was purified twice by flash chromatography on silica gel (under gradient elution system of dichloromethane : methanol 100:0 - 97:3). Compound 65 was isolated as a white solid in 2.2 % yield.
¹H-NMR (CD₃OD, 500 MHz), mixture of four diastereoisomers, two major in α form and two minor in β form: δ 7.18 (2H, m), 6.89 (2H, m), 5.13 (1H, d, *J* = 3.2 Hz), 4.67 (1H, m), 4.52 (1H, m), 4.51 (1H, m), 4.48 (1H, m), 4.31 (1H, m), 4.05 (2H, m), 3.79 (3H, s), 3.78 (3H, s), 3.71 (3H, m), 3.70 (3H, m), 3.57 (1H, m), 3.50 (1H, m), 3.37 (1H, m), 2.19 (1H, m), 1.97 (1H, m), 1.92 (2H), 1.77 (3H, s), 1.76 (3H, s);¹³C-NMR (CD₃OD, 125 MHz): δ 22.79, 26.18, 32.31, 48.50, 52.73, 52.95, 54.81, 56.09 (d, *J*_{(C-P)} = 3.97 Hz), 61.92 (d, *J*_{(C-P)} = 6.74 Hz), 65.02, 70.14, 71.74 (d, *J*_{(C-P)}= 3.6 Hz), 79.46 (d, *J*_{(C-P)} = 7.6 Hz), 92.73, 96.14, 115.77 (d, *J*_{(C-P)} = 4.2 Hz), 122.22 (d, *J*_{(C-P)} = 4.4 Hz), 144.10 (d, *J*_{(C-P)} = 7.57 Hz), 158.6, 173.91, 174.14, 175.37; ³¹P-NMR (CD₃OD, 202 MHz): δ 3.03 (one diastereoisomer as α-isomer), 2.40 (one diastereoisomer as α-isomer) 2.39 (one diastereoisomer as β-isomer), 2.14 (one diastereoisomer as β-isomer); MS (ES+) m/z 541.15 (MNa⁺).

Further elution with dichloromethane : methanol 97:3 afforded compound 64 (102mg, 1.8% yield).
¹H-NMR (CD₃OD, 500 MHz) mixture of four diastereoisomers, two major in α form and two minor in β form): δ 7.17 (2H, m), 6.88 (2H, m), 5.14 (1H, d, *J* = 3.9 Hz), 4.72 (1H, m), 4.63 (1H, m), 4.31 (1H, m), 4.07 (1H, m), 3.88 (1H, m), 3.82 (1H, m), 3.81 (8H, m), 3.79 (3H, s), 3.78 (3H, s), 3.64 (1H, m), 3.59 (1H, m), 3.49 (1H, m), 2.21 (1H, m), 1.87 (1H, m), 1.99 (2H, m), 1.79 (3H, s); ¹³C-NMR (CD₃OD, 125 MHz): δ 22.65, 26.15 (d, *J*_{(C-P)} = 9.2 Hz), 32.24 (d, *J*_{(C-P)} = 9.3 Hz), 48.73, 52.73, 54.88 (d, *J*_{(C-P)} = 2.97 Hz), 56.11, 61.98 (d, *J*_{(C-P)} = 6.74 Hz), 62.59, 70.94 (d, *J*_{(C-P)} = 3.6 Hz), 73.36, 79.99 (d, *J*_{(C-P)} = 7.6 Hz), 82.50 (d, *J*_{(C-P)}= 7.6Hz), 92.67, 96.72, 115.77, 122.20 (d, *J*_{(C-P)} = 4.4 Hz), 145.49 (d, *J*_{(C-P)} = 7.57 Hz), 158.40, 173.34, 176.43; ³¹P-NMR (CD₃OD, 202 MHz): δ 3.11 (one diastereoisomer as α-isomer), 2.75 (one diastereoisomer as β-isomer), 2.49 (one diastereoisomer as β-isomer); MS (ES+) *m*/*z* 541.15 (MNa⁺).

### Example 51:

### Preparation of 2-acetamido-2-deoxy-3-O-[4-methoxyphenyl(n-propoxy-L-prolinyl)]phosphoramidate-D-glucopyranose (compound 66) and 2-acetamido-2-deoxy-4-O-[4-methoxyphenyl(n-propoxy-L-prolinyl)]phosphoramidate-D-glucopyranose (compound 67)

Prepared according to general procedure of Example 5 (addition at room temperature), from *N*-acetyl-D-glucosamine and 4-methoxyphenyl(n-propoxy-L-prolinyl)phosphorochloridate. The crude product was purified by flash chromatography on silica gel under gradient elution system of dichloromethane : methanol 100:0 - 97:3. Compound 67 was isolated as a white solid.(107mg, 0.9% yield).
¹H-NMR (CD₃OD, 500 MHz), mixture of two diastereoisomers, one major in α form and one minor in β form: δ 7.12 (2H, d, *J* = 9.3 Hz), 7.08 (2H, d, *J* = 9.3 Hz), 6.91 (2H, d, *J* = 7.6 Hz), 6.89 (2H, d, *J* = 7.6 Hz), 5.11 (1H, d, *J* = 3.3 Hz), 4.62 (1H, m), 4.51 (1H, m), 4.42 (1H, m), 4.40 (1H, m), 4.31 (2H, q, *J* = 7.3 Hz), 4.11 (2H, m), 3.75 (3H, m), 3.52 (1H, m), 3.45 (1H, m), 3.29 (1H, m, overlap with solvent), 2.23 (1H, m) 2.05 (1H, m), 1.98 (2H), 1.79 (3H, s), 1.75 (3H, s), 1.83 (2H, m), 1.62 (2H, m), 0.82 (3H, t, *J*= 7.3 Hz); ¹³C-NMR (CD₃OD, 125 MHz): δ 10.69, 22.99, 26.14 (d, *J*_{(C-P)} = 8.7 Hz), 32.35, 48.49, 54.84 (d, *J*_{(C-P)} = 3.0 Hz), 56.09, 62.05 (d, *J*_{(C-P)} = 6.5 Hz), 66.84, 66.87 (d, *J*_{(C-P)} = 6.4 Hz), 70.16, 71.77 (d, *J*_{(C-P)} = 3.0 Hz), 79.43 (d, *J*_{(C-P)}= 6.4 Hz), 92.39, 97.65, 115.67, 122.30 (d, *J*_{(C-P)} = 4.4 Hz), 122.05 (d, *J*_{(C-P)} = 4.9 Hz), 122.25 (d, *J*_{(C-P)} = 4.6 Hz), 122.35 (d, *J*_{(C-P)} = 4.9 Hz), 145.51 (d, *J*_{(C-P)} = 8.1 Hz), 158.37, 173.27, 175.28, 176.18; ³¹P-NMR (CD₃OD, 202 MHz): δ 3.19 (one diastereoisomer as α-isomer), 2.50 (one diastereoisomer as α-isomer), 2.45 (one diastereoisomer as β-isomer), 2.20 (one diastereoisomer as β-isomer); MS (ES+) *m*/*z* 569.19 (MNa⁺).

Further elution with dichloromethane : methanol 97:3 afforded compound 66 (1.94 g, 21.6 % yield).
¹H-NMR (CD₃OD, 500 MHz), mixture of two diastereoisomers, one major in α form and one minor in β form: δ 7.12 (2H, m), 6.97 (2H), 5.11 (1H, d, *J* = 3.5 Hz), 4.56 (2H, m), 4.32 (2H, m), 4.05 (1H, m), 3.89 (1H, m), 3.81 (5H, m), 3.77, 3.71 (1H, m), 3.59 (1H, m), 3.47 (1H, m), 2.18 (1H, m), 2.01 (1H, m), 1.80 (2H), 1.68 (3H, s), 1.61 (2H, m), 1.24 (2H, m), 0.89 (3H, m); ¹³C-NMR (CD₃OD, 125 MHz): δ 10.03, 19.65, 22.91, 29.76 (d, *J*_{(C-P)} = 8.7 Hz), 32.48 (d, *J*_{(C-P)} = 8.7 Hz), 47.33, 53.85 (d, *J*_{(C-P)} = 3.0 Hz), 56.19, 61.95, 62.27 (d, *J*(_{C-P}) =6.5 Hz), 66.47, 71.01 (d, J_{(C-P)} = 3.0 Hz), 73.81, 79.84 (d, *J*_{(C-P)} = 6.4 Hz), 92.41, 96.80, 115.63, 122.44 (d, *J*_{(C-P)} = 4.4 Hz), 122.08 (d, *J*_{(C-P)} = 4.9 Hz), 145.46 (d, *J*_{(C-P)} = 8.1 Hz), 158.38, 173.22, 176.04; ³¹P-NMR (CD₃OD, 202 MHz): δ 3.24 (one diastereoisomer as α-isomer), 2.84 (one diastereoisomer as α-isomer), 2.64 (one diastereoisomer as β-isomer); MS (ES+) m/z 569.19 (MNa⁺).

### Example 52:

### Preparation of 2-acetamido-2-deoxy-3-O-[4-methoxyphenyl(n-pentoxy-L-prolinyl)lphosphoramidate-D-glucopyranose (compound 68) and 2-acetamido-2-deoxy-4-0-[4-methoxyphenyl(n-pentoxy-L-prolinyl)]phosphoramidate-D-glucopyranose (compound 69)

Prepared according to general procedure of Example 5 (addition at room temperature), from N acetyl-D-glucosamine and 4-methoxyphenyl(n-pentoxy-L-prolinyl) phosphorochloridate . The crude product was purified twice by flash chromatography on silica gel under gradient elution system of dichloromethane : methanol 100:0 - 97:3. Compound 69 was isolated as a white solid.
¹H-NMR (CD₃OD, 500 MHz), mixture of two diastereoisomers, one major in α form and one minor in β form: δ 7.12 (2H, m), 7.08 (2H, m), 6.91 (2H, m), 6.89 (2H, dm), 5.14 (1H, m), 4.61 (1H, m), 4.51 (2H, m), 4.38 (1H, m), 4.33 (2H, m), 4.14 (2H, m), 3.75 (3H, m), 3.51 (1H, m), 3.45 (1H, m), 3.35 (1H, m, overlap with solvent), 2.24 (1H, m) 2.07 (1H, m), 1.95 (2H), 1.79 (3H, s), 1.75 (3H, s), 1.83 (2H, m), 1.47 (4H, m), 0.82 (3H, m); ¹³C-NMR (CD₃OD, 125 MHz): δ 14.33, 22.80, 22.80, 26.15 (d, *J*_{(C-P)} = 8.75 Hz), 29.18, 29.44, 32.38 (d, *J*_{(C-P)} = 8.6 Hz), 48.28, 54.87, 56.10 (d, *J*_{(C-P)} = 4.5 Hz), 62.04 (d, *J*_{(C-P)} = 6.5 Hz), 66.39, 66.69 (d, *J*_{(C-P)} = 6.4 Hz), 70.17, 71.80 (d, *J*_{(C-P)} = 3.0 Hz), 79.40 (d, *J*_{(C-P)} = 6.4 Hz), 92.70, 97.65, 115.78, 122.34 (d, *J*_{(C-P)} = 4.4 Hz), 122.05 (d, *J*_{(C-P)} = 4.9 Hz), 145.51 (d, *J*_{(C-P)} = 8.1 Hz), 158.37, 173.44, 175.29, 176.18; ³¹P-NMR (CD₃OD, 202 MHz): δ 3.11 (one diastereoisomer as α-isomer), 2.50 (one diastereoisomer as α-isomer), 2.17 (one diastereoisomer as β-isomer); MS (ES+) m/z 597.23 (MNa⁺).

Further elution with dichloromethane methanol 97:3 afforded compound 68.
¹H-NMR (CD₃OD, 500 MHz), mixture of two diastereoisomers, one major in α form and one minor in β form: δ 7.12 (2H, m), 6.89 (2H), 5.11 (1H, d, *J* = 3.5 Hz), 4.61 (2H, m), 4.32 (2H, m), 4.12 (2H, m), 4.05 (1H, dd, *J* = 10.4 Hz and 3.4 Hz), 3.81 (1H, m), 3.89-3.85 (2H, m), 3.73, 3.71, 3.57 (1H, m), 3.54 (1H, m), 3.38 (1H, m), 2.18 (1H, m), 2.01 (1H, m), 1.80 (2H), 1.78 (3H, s), 1.61 (2H, m), 1.38 (4H, m), 0.89 (3H, m); ¹³C-NMR (CD₃OD, 125 MHz): δ 14.32, 22.84, 23.10, 26.18 (d, *J*_{(C-P)} = 8.7 Hz), 29.16, 29.38, 32.43 (d, *J*_{(C-P)} = 8.75 Hz), 45.98, 54.89 (d, *J*_{(C-P)} = 3.0 Hz), 56.04, 61.97 (d, *J*_{(C-P)} = 6.5 Hz), 62.12, 66.70, 70.96 (d, *J*_{(C-P)} = 3.0 Hz), 73.37 , 79.93 (d, *J*_{(C-P)} = 6.4 Hz), 92.64, 96.75, 115.77, 122.02 (d, *J*_{(C-P)} = 4.4 Hz), 122.27 (d, *J*_{(C-P)} = 4.6 Hz), 122.35 (d, *J*_{(C-P)} = 4.9 Hz), 145.48 (d, *J*_{(C-P)} = 8.1 Hz), 158.39, 173.29, 176.30; ³¹P-NMR (CD₃OD, 202 MHz): δ 3.19 (one diastereoisomer as α-isomer), 2.79 (one diastereoisomer as β-isomer), 2.58 (one diastereoisomer as β-isomer); MS (ES+) m/z 597.23 (MNa⁺).

### Example 53:

### Preparation of 2-acetamido-2-deoxy-3-O-[4-methoxyphenyl(3-pentoxy-L-prolinyl)]phosphoramidate-D-glucopyranose (compound 70) and 2-acetamido-2-deoxy-4-0-[4-methoxyphenyl(3-pentoxy-L-prolinyl)]phosphoramidate-D-glucopyranose (compound 71)

Prepared according to general procedure of Example 5 (addition at room temperature), from N acetyl-D-glucosamine and 4-methoxyphenyl(3-pentoxy-L-prolinyl)phosphorochloridate. The crude product was purified by flash chromatography on silica gel under gradient elution system of dichloromethane : methanol, 100:0 - 97:3. Compound 71 was isolated as a white solid.
¹H-NMR (CD₃OD, 500 MHz), mixture of two diastereoisomers, one major in α form and one minor in β form: δ 7.12 (2H, d, *J* = 9.1 Hz), 7.08 (2H, d, *J* = 9.1 Hz), 6.91 (2H, d, *J* = 9.1 Hz), 6.89 (2H, d, *J* = 9.1 Hz), 5.12 (1H, d, *J* = 3.7 Hz), 4.81 (1H, m), 4.67 (1H, m), 4.53 (1H, m), 4.47 (1H, m), 4.31 (1H, m), 4.04 (2H, m), 3.75 (3H, s), 3.62 (1H, m), 3.52 (1H, m), 3.31 (1H, m), 2.21 (1H, m), 2.06-1.98 (3H, m), 1.68 (3H, s), 1.63 (4H, m), 0.92 (6H, m); ¹³C-NMR (CD₃OD, 125 MHz): δ 10.02, 22.78, 26.10 (d, *J*_{(C-P)} = 8.75 Hz), 27.59, 32.59, 48.26, 54.93 (d, *J*_{(C-P)} = 3.0 Hz), 56.09, 56.17, 62.25 (d, *J*_{(C-P)} = 6.5 Hz), 66.87, 70.16 (d, *J*_{(C-P)} = 3.0 Hz), 71.82, 79.28, 79.77 (d, *J*_{(C-P)} = 6.4 Hz), 92.65, 97.65, 115.65, 122.41 (d, *J*_{(C-P)} = 4.4 Hz), 145.51 (d, *J*_{(C-P)} = 8.1 Hz), 158.43, 173.25, 175.25, 176.31; ³¹P-NMR (CD₃OD, 202 MHz): δ 3.24 (one diastereoisomer as α-isomer), 2.60 (one diastereoisomer as β-isomer), 2.23 (one diastereoisomer as β-isomer); MS (ES+) *m*/z 597.23 (MNa⁺).

Further elution with dichloromethane : methanol 97:3 afforded compound 70.
¹H-NMR (CD₃OD, 500 MHz), mixture of two diastereoisomers, one major in α form and one minor in β form: δ 7.12 (2H, m), 6.97 (2H), 5.11 (1H, d, *J* = 3.5 Hz), 4.81 (1H, m), 4.62 (2H, m), 4.33 (2H, m), 4.01 (1H, dd, *J* = 10.4 Hz and 3.4 Hz), 3.81 (1H, m), 3.89-3.85 (2H, m), 3.73, 3.71, 3.61 (1H, m), 3.52 (1H, m), 3.38 (1H, m), 2.21 (1H, m), 2.01 (1H, m), 1.80 (2H), 1.80 (3H, s), 1.64 (4H, m), 0.89 (6H); ¹³C-NMR (CD₃OD, 125 MHz): δ 10.05, 22.81, 26.15 (d, *J*_{(C-P)} = 8.7 Hz), 27.49, 27.59, 32.60 (d, *J*_{(C-P)} = 8.7 Hz), 48.51, 54.97, 56.10, 62.35 (d, *J*_{(C-P)} = 6.5 Hz), 62.48, 71.00, 73.41, 79.75, 92.60, 115.77, 122.35 (d, *J*_{(C-P)} = 4.4 Hz), 145.50 (d, *J*_{(C-P)} = 8.1 Hz), 158.38, 173.34, 176.30; ³¹P-NMR (CD₃OD, 202 MHz): δ 3.33 (one diastereoisomer as α-isomer), 3.01 (one diastereoisomer as α-isomer), 2.70 (one diastereoisomer as β-isomer); MS (ES+) *m*/z 597.23 (MNa⁺).

### Example 54:

### Preparation of 2-acetamido-2-deoxy-3-O-[4-methoxyphenyl(neopentoxy-L-prolinyl)lphosphoramidate-D-glucopyranose (compound 72) and 2-acetamido-2-deoxy-4-0-[4-methoxyphenyl(neopentoxy-L-prolinyl)]phosphoramidate-D-glucopyranose (compound 73)

Prepared according to general procedure of Example 5 (addition at room temperature), from N-acetyl-D-glucosamine and 4-methoxyphenyl(neopentoxy-L-prolinyl)phosphorochloridate. The crude product was purified by flash chromatography on silica gel (under gradient elution system of dichloromethane : methanol, 100:0 - 97:3). Compound 73 was isolated as a white solid.
¹H-NMR (CD₃OD, 500 MHz), mixture of two diastereoisomers, one major in α form and one minor in β form: δ 7.12 (2H, m), 7.08 (2H, m), 6.91 (2H, m), 6.87 (2H, m), 5.12 (1H, d, *J* = 3.7 Hz), 4.61 (1H, m), 4.49 (1H, m), 4.41 (1H, m), 4.31 (1H, m), 4.04 (2H, m), 3.88 (2H, m), 3.75 (3H, s), 3.74 (3H, s), 3.62 (1H, m), 3.48 (1H, m), 3.37 (1H, m), 2.21 (1H, m), 2.06-1.98 (3H, m), 1.79 (3H, s), 0.99 (9H, s); ¹³C-NMR (CD₃OD, 125 MHz): δ 22.79, 26.11 (d, *J*_{(C-P)} = 8.7 Hz), 27.79, 32.40, 48.24, 58.93 (d, *J*_{(C-P)} = 3.0 Hz), 56.09, 56.16, 62.01 (d, *J*_{(C-P)} = 6.5 Hz), 66.86, 70.13, 71.76, 75.36, 75.67, 79.77 (d, *J*_{(C-P)} = 6.4 Hz), 92.69, 97.65, 115.78, 122.29 (d, *J*_{(C-P)} = 4.4 Hz), 145.51 (d, *J*_{(C-P)} = 8.1 Hz), 158.43, 173.25, 175.25, 176.31; ³¹P-NMR (CD₃OD, 202 MHz): δ 3.03 (one diastereoisomer as α-isomer), 2.23 (one diastereoisomer as α-isomer), 2.13 (one diastereoisomer as β-isomer); MS (ES+) *m*/z 597.23 (MNa⁺).

Further elution with dichloromethane : methanol 97:3 afforded compound 72.
¹H-NMR (CD₃OD, 500 MHz), mixture of two diastereoisomers, one major in α form and one minor in β form: δ 7.11 (2H, m), 6.87 (2H), 5.13 (1H, d, *J* = 3.5 Hz), 4.63 (2H, m), 4.37 (2H, m), 4.05 (1H, dd, *J* = 10.4 Hz and 3.4 Hz), 3.81 (1H, m), 3.89-3.85 (4H, m), 3.79, 3.69, 3.57 (1H, m), 3.44 (1H, m), 3.38 (1H, m), 2.21 (1H, m), 1.99 (3H, s), 1.98 (1H, m), 1.91 (2H), 0.99 (9H, s); ¹³C-NMR (CD₃OD, 125 MHz): δ 22.79, 26.13 (d, *J*_{(C-P)} = 8.7 Hz), 27.01, 32.45, 48.30, 54.90 (d, *J*_{(C-P)} 3.0 Hz), 56.18, 62.20 (d, *J*_{(C-P)} = 6.5 Hz), 62.25, 71.55 (d, *J*_{(C-P)} = 3.0 Hz), 73.3, 79.97 (d, *J*_{(C-P)} = 6.4 Hz), 92.72, 96.78, 122.28 (d, *J*_{(C-P)} = 4.6 Hz), 122.37 (d, *J*_{(C-P)} = 4.9 Hz), 145.46 (d, *J_{(C-P)}* = 8.1 Hz), 158.38 (d, *J*_{(C-P)} = 8.1 Hz), 173.69, 176.26; ³¹P-NMR (CD₃OD, 202 MHz): δ 3.13 (one diastereoisomer as α-isomer), 2.89 (one diastereoisomer as β-isomer), 2.77 (one diastereoisomer as β-isomer); MS (ES+) *m*/z 597.23 (MNa⁺).

### Example 55:

### Preparation of 2-acetamido-2-deoxy-3-O-[phenyl(benzyloxy-L-prolinyl)]phosphoramidate-D-glucopyranose (compound 74) and 2-acetamido-2-deoxy-[4-phenyl(benzyloxy-L-prolinyl)]phosphoramidate-D-glucopyranose (compound 75)

Prepared according to general procedure of Example 5 (addition at room temperature), from N acetyl-D-glucosamine and phenyl(benzoxy-L-prolinyl)phosphorochloridate. The crude product was purified by flash chromatography on silica gel under gradient elution system of dichloromethane : methanol, 100:0 - 97:3. Compound 75 was isolated as a white solid.
¹H-NMR (CD₃OD, 500 MHz), mixture of two diastereoisomers, one major as α, form and one minor as β form: δ 7.37 (7H, m), 7.16 (3H, m), 5.21 (3H, m), 4.51 (1H, m), 4.37 (2H, m), 4.28 (1H, m), 4.19 (2H, m), 3.47 (1H, m), 3.39 (1H, m), 3.15 (1H, m), 2.07 (1H, m), 1.93 (3H, s), 1.82 (1H, m), 1.71 (2H, m); ¹³C-NMR (CD₃OD, 125 MHz): δ 23.15, 26.08 (d, *J*_{(C-P)} = 9.1 Hz), 32.37 (d, *J*_{(C-P)} = 8.6 Hz), 48.76 (d, *J*_{(C-P)} = 4.5 Hz), 55.90, 61.97, 62.89, 67.42, 68.18, 72.97, 79.49, 92.62, 96.79, 121.33, 126.22, 129.19, 129.34, 130.78, 137.26, 137.68, 152.13, 152.19, 173.90, 174.78, 176.14; ³¹P-NMR (CD₃OD, 202 MHz): δ 1.95 (one diastereoisomer in α-form), 1.81 (one diastereoisomer in β-form).

Further elution with dichloromethane : methanol 97:3 afforded compound 74.
¹H-NMR (CD₃OD, 500 MHz): mixture of two diastereoisomers, one major as α form and one minor as β form): δ 7.32 (7H, m), 7.16 (3H, m), 5.17 (3H, m), 4.72 (1H, m), 4.42 (1H, m), 4.05 (1H, dd, *J* = 10.3 Hz, *J* = 3.8 Hz), 3.85 (3H, m), 3.67 (1H, m), 3.59 (1H, m), 3.37 (1H, m), 2.11 (1H, m), 2.07 (1H, m), 1.85 (2H, m), 1.72 (3H, s); ¹³C-NMR (CD₃OD, 125 MHz): δ 22.65, 26.13 (d, *J*_{(C-P)} = 10.1 Hz), 32.25 (d, *J*_{(C-P)} = 8.7 Hz), 48.78, 54.83 (d, *J*_{(C-P)} = 2.5 Hz), 61.99, 62.10, 68.18, 70.93 (d, *J*_{(C-P)} = 2.5 Hz), 73.32, 80.23 (d, *J*_{(C-P)} = 6.2 Hz), 92.36, 96.69, 115.76, 121.37, 126.22, 129.37, 129.63, 130.87, 137.15, 152.13, 173.33, 175.76; ³¹P-NMR (CD₃OD, 202 MHz): δ 2.68 (one diastereoisomer in α-form), 2.12, 2.10 (two diastereoisomers in β-form).

### Example 56:

### Preparation of 2-acetamido-2-deoxy-3-O-[phenyl(ethoxy-L-prolinyl)]phosphoramidate-D-glucopyranose (compound 76) and 2-acetamido-2-deoxy-4-O-[phenyl(ethoxy-L-prolinyl)]phosphoramidate-D-glucopyranose (compound 77)

Prepared according to general procedure of Example 5 (addition at room temperature), from N-acetyl-D-glucosamine and phenyl(ethoxy-L-prolinyl)phosphorochloridate. The crude product was purified by flash chromatography on silica gel under gradient elution system of dichloromethane : methanol, 100:0 - 97:3. Compound 77 was isolated as a white solid.
¹H-NMR (CD₃OD, 500 MHz), mixture of four diastereoiomers, two major in α form and two minor in β form: δ 7.31 (2H, m), 7.21 (3H, m), 5.12 (1H, d, *J* = 3.2 Hz), 4.71 (2H, m), 4.59 (2H, m), (1H, m), 4.37, 4.21 (2H, m), 4.11 (2H, m), 3.61 (1H, m), 3.50 (1H, m), 3.39 (1H, m), 2.21 (1H, m), 2.00 (1H, m), 1.90 (2H, m), 1.91 (3H, s), 1.81 (3H, s), 1.25 (3H, t, J= 7.3 Hz); ¹³C-NMR (CD₃OD, 125 MHz): δ 14.56, 22.96, 26.25 (d, *J*_{(C-P)} = 9.2 Hz), 32.40 (d, *J*_{(C-P)} = 9.3 Hz), 49.28, 54.76, 61.77 (d, *J*_{(C-P)} = 6.7 Hz), 62.09, 62.69, 66.94, 70.22 (d, *J*_{(C-P)} = 3.6 Hz), 71.75, 79.56 (d, *J*_{(C-P)} = 7.6 Hz), 92.70, 96.81, 121.33, 126.21, 130.81, 152.14, 173.30, 175.18, 176.11; ³¹P-NMR (CD₃OD, 202 MHz): δ 2.64 (one diastereoisomer as α-isomer), 2.04 (one diastereoisomer as α-isomer), 1.81 (one diastereoisomer as β-isomer)

Further elution with dichloromethane : methanol 97:3 afforded compound 76.
¹H-NMR (CD₃OD, 500 MHz), mixture of four diastereoiomers, two major in α form and two minor in β form: δ 7.31 (2H, m), 7.21 (3H, m), 5.12 (1H, d, J = 3.2 Hz), 4.71 (2H, m), 4.59 (1H, m), 4.31 (1H, m), 4.21 (2H, m), 4.11 (1H, dd, J = 7.3 Hz, J = 3.2 Hz), 3.87 (2H, m), 3.50 (1H, m), 3.41 (2H, m), 2.21 (1H, m), 2.12 (3H, s), 2.00 (1H, m), 1.90 (2H, m), 2.01 (3H, s), 1.81 (3H, s), 1.25 (3H, t, *J* = 7.3 Hz); ¹³C-NMR (CD₃OD, 125 MHz): δ 14.45, 22.87, 26.18 (d, *J*_{(C-P)} = 9.2 Hz), 32.35 (d, *J*_{(C-P)} = 9.3 Hz), 48.73, 54.85 (d, *J*_{(C-P)} = 2.9 Hz), 62.15 (d, *J*_{(C-P)} = 6.7 Hz), 62.47, 62.71, 70.94 (d, *J*_{(C-P)} = 3.8 Hz), 73.35, 80.06 (d, *J*_{(C-P)} = 7.6 Hz), 92.69, 96.70, 121.17, 126.24, 130.88, 173.33, 176.18; ³¹P-NMR (CD₃OD, 202 MHz): δ 2.70 (one diastereoisomer as α-isomer) 2.32 (one diastereoisomer as β-isomer), 2.07 (one diastereoisomer as β -isomer).

### Example 57:

### Preparation of 2-acetamido-2-deoxy-3-O-[1-naphtyl(benzyloxy-L-prolinyl)]phosphoramidate-D-glucopyranose (compound 78) and 2-acetamido-2-deoxy-4-O-[1-naphtyl(benzyloxy-L-prolinyl)]phosphoramidate-D-glucopyranose (compound 79)

Prepared according to general procedure of Example 5 (addition at room temperature), from N-acetyl-D-glucosamine and 1-naphtyl(benzoxy-L-prolinyl)phosphorochloridate. The crude product was purified by flash chromatography on silica gel under gradient elution system of dichloromethane : methanol, 100:0 - 97:3. Compound 79 was isolated as a white solid.
¹H-NMR (CD₃OD, 500 MHz), mixture of two diastereoisomers, one major as α form and one minor as β form: δ 8.21 (2H, m), 7.90 (1H, m), 7.80 (1H, d, *J* = 6.5 Hz), 7.72 (1H, d, *J* = 6.5 Hz), 7.67 (1H, d, *J* = 6.5 Hz), 7.51 (2H, m), 7.37 (2H, m), 7.36-7.21 (5H), 5.21 (2H, m), 5.12 (1H, d, *J* = 3.5 Hz), 4.71 (1H, m), 4.47 (1H, m), 4.44 (3H, m), 3.91 (1H, m), 3.57 (2H, m), 3.49 (1H, m), 3.35 (1H, m), 2.21 (1H, m), 1.82 (1H, m), 1.71 (2H, m); 1.73 (3H, s); ¹³C-NMR (CD₃OD, 125 MHz): δ 22.72, 22.77, 26.04 (d, *J*_{(C-P)} = 10.0 Hz), 32.40 (d, *J*_{(C-P)} = 8.7 Hz), 49.92 (d, *J*_{(C-P)} = 4.5 Hz), 54.86 (d, *J*_{(C-P)} = 4.5 Hz), 61.97 (d, *J*_{(C-P)} = 5.1 Hz), 68.05, 69.88, 70.44, 71.81 (d, *J*_{(C-P)} = 5.6 Hz), 79.52 (d, *J*_{(C-P)} = 6.1 Hz), 92.63, 96.79, 116.23, 116.83, 122.78, 126.30 (d, *J*_{(C-P)} = 8.6 Hz), 127.59 (d, *J*_{(C-P)} = 8.6 Hz), 127.84 (d, *J*_{(C-P)} = 8.6 Hz), 129.19, 129.33, 129.59, 136.31, 137.07 (d, *J*_{(C-P)} = 10.1 Hz), 147.19, 173.32, (d, *J*_{(C-P)} = 9.3 Hz), 174.94, 175.90; ³¹P-NMR (CD₃OD, 202 MHz): δ 3.10 (one diastereoisomer in β-form), 2.99 (one diastereoisomer in α-form), 2.37 (one diastereoisomer in α-form), 1.89 (one diastereoisomer in β-form)

Further elution with dichloromethane : methanol 97:3 afforded compound 78.
¹H-NMR (CD₃OD, 500 MHz), mixture of two diastereoisomers, one major as α form and one minor as β form : δ 8.21 (1H, m), 7.90 (1H, m), 7.80 (1H, m), 7.72 (1H, d, J = 6.5 Hz), 7.60 (2H, m), 7.47 (2H, m), 7.36-7.21 (5H), 5.17 (1H, d, J = 3.1 Hz), 5.13 (2H, m), 4.72 (1H, m), 4.52 (1H, m), 4.05 (1H, dd, *J* = 10.3 Hz and 3.85 Hz), 3.85 (3H, m), 3.67 (1H, m), 3.59 (1H, m), 3.46 (1H, m), 2.14 (1H, m), 2.03 (1H, m), 1.89 (2H, m), 1.72 (3H, s); ¹³C-NMR (CD₃OD, 125 MHz): δ 22.69, 26.08 (d, *J*_{(C-P)} = 10.1 Hz), 32.39 (d, *J*_{(C-P)} = 8.7 Hz), 47.27, 55.01 (d, *J*_{(C-P)} = 2.5 Hz), 62.14, 62.50, 68.17, 70.97 (d, *J*_{(C-P)} = 2.5 Hz), 73.43, 80.11 (d, *J*_{(C-P)} = 6.2 Hz), 92.63, 96.84, 116.23, 122.78, 126.10 (d, *J*_{(C-P)} = 8.6 Hz), 127.54 (d, *J*_{(C-P)} = 8.6 Hz), 127.84 (d, *J*_{(C-P)} = 8.6 Hz), 128.52, 129.57, 129.73, 136.11, 137.06 (d, *J*_{(C-P)} = 10.1 Hz), 147.87, 173.25, 175.83; ³¹P-NMR (CD₃OD, 202 MHz): δ 3.13 (one diastereoisomer in α-form), 2.68, 2.48 (two diastereoisomers in β-form).

### Example 58:

### Preparation of 2-acetamido-2-deoxy-3-O-[1-naphtyl(ethoxy-L-prolinyl)]phosphoramidate-D-glucopyranose (compound 80) and 2-acetamido-2-deoxy-4-O-[1-naphtyl(ethoxy-L-prolinyl)]phosphoramidate-D-glucopyranose (compound 81)

Prepared according to general procedure of Example 5 (addition at room temperature), from N-acetyl-D-glucosamine and 1-naphtyl(ethoxy-L-prolinyl)phosphorochloridate. The crude product was purified by flash chromatography on silica gel under gradient elution system of dichloromethane : methanol, 100:0 - 97:3. Compound 81 was isolated as a white solid.
¹H-NMR (CD₃OD, 500 MHz), mixture of two diastereoisomers, one major as α form and one minor as β form: δ 8.21 (2H, m), 7.90 (1H, m), 7.80 (1H, d, *J* = 6.5 Hz), 7.72 (1H, d, *J* = 6.5 Hz), 7.67 (1H, d, *J* = 6.5 Hz), 7.51 (2H, m), 7.37 (2H, m), 7.36-7.21 (5H), 5.21 (2H, m), 5.12 (1H, d, J = 3.6 Hz), 4.71 (1H, m), 4.47 (1H, m), 4.44 (3H, m), 3.91 (1H, m), 3.57 (2H, m), 3.49 (1H, m), 3.35 (1H, m), 2.21 (1H, m), 1.82 (1H, m), 1.71 (2H, m); 1.73 (3H, s); ¹³C-NMR (CD₃OD, 125 MHz): δ 22.72, 22.77, 26.04 (d, *J*_{(C-P)} = 10.0 Hz), 32.40 (d, *J*_{(C-P)} = 8.7 Hz), 49.92 (d, *J*_{(C-P)} = 4.5 Hz), 54.86 (d, *J*_{(C-P)} = 4.5 Hz), 61.97 (d, *J*_{(C-P)} = 5.1 Hz), 68.05, 69.88, 70.44, 71.81 (d, *J*_{(C-P)} = 5.6 Hz), 79.52 *J*_{(C-P)} = 6.3 Hz), 92.63, 96.79, 115.77, 122.74, 126.00, 126.33, 126.60, 127.57, 128.92, 147.19, 173.32, (d, *J*_{(C-P)} = 9.3 Hz), 174.94, 175.90; ³¹P-NMR (CD₃OD, 202 MHz): δ 3.10 (one diastereoisomer in β-form), 2.99 (one diastereoisomer in α-form), 2.37 (one diastereoisomer in α-form), 1.89 (one diastereoisomer in β-form); MS (ES+) m/z 553.10 (MNa⁺).

Further elution with dichloromethane : methanol 97:3 afforded compound 80.
¹H-NMR (CD₃OD, 500 MHz), mixture of two diastereoisomers, one major as α form and one minor as β form: δ 8.21 (1H, d, J = 6.5 Hz), 7.90 (1H, d, J = 6.5 Hz), 7.75 (1H, d, *J* = 6.5 Hz), 7.51 (2H, m), 7.42 (2H, m), 5.17 (1H, d, *J* = 3.1 Hz), 4.72 (1H, m), 4.50 (1H, m), 4.12 (2H, q, *J* = 7.6 Hz), 4.05 (1H, dd, J = 10.3 Hz and *J* = 3.8 Hz), 3.85 (3H, m), 3.67 (1H, m), 3.59 (1H, m), 3.46 (1H, m), 2.14 (1H, m), 1.93 (1H, m), 1.81 (2H, m), 1.69 (3H, s), 1.30 (3H, t, *J* = 6.3 Hz); ¹³C-NMR (CD₃OD, 125 MHz): δ 14.37, 22.79, 26.14 (d, *J*_{(C-P)} = 10.1 Hz), 32.37 (d, *J*_{(C-P)} = 8.7 Hz), 47.97, 54.85, 55.01 (d, *J*_{(C-P)} = 2.5 Hz), 62.18, 62.50, 70.96 (d, *J*_{(C-P)} = 2.5 Hz), 73.46, 79.99 (d, *J*_{(C-P)} = 6.2 Hz), 92.64, 96.83, 115.77, 122.74, 126.00, 126.33, 126.60, 127.57, 128.92, 147.19, 147.87, 173.31, 176.29; ³¹P-NMR (CD₃OD, 202 MHz): δ 3.17 (one diastereoisomer in α-form), 2.76, 2.46 (two diastereoisomers in β-form); MS (ES+) *m*/z 553.10 (MNa⁺).

### Example 59:

### Preparation of 2-acetamido-2-deoxy-3-O-[4-chlorophenyl(benzyloxy-L-prolinyl)]phosphoramidate-D-glucopyranose (compound 82)

Prepared according to general procedure of Example 5 (addition at room temperature), from N-acetyl-D-glucosamine and 4-chlorophenyl(benzyloxy-L-prolinyl)phosphorochloridate. The crude product was purified by flash chromatography on silica gel under gradient elution system of dichloromethane : methanol, 100:0 - 95:5. Compound 82 was isolated as a white solid.
¹H-NMR (CD₃OD, 500 MHz), mixture of two diastereoisomers, one major as α form and one minor as β form: δ 7.37 (7H, m), 7.16 (2H, d, *J* = 9.2 Hz), 5.17 (3H, m), 4.69 (1H, m), 4.49 (1H, m), 4.05 (1H, dd, *J* = 10.3 and 3.85 Hz), 3.80 (1H, m), 3.71 (2H, m), 3.60 (1H, m), 3.57 (1H, m), 3.49 (1H, m), 2.21 (1H, m), 2.20 (3H, s), 2.08 (3H, s), 2.07 (1H, m), 1.95 (2H, m), 1.94 (3H, s), 1.72 (3H, s); ¹³C-NMR (CD₃OD, 125 MHz): δ 22.72, 23.20, 26.07 (d, *J*_{(C-P)} = 10.1 Hz), 32.37 (d, *J*_{(C-P)} = 8.7 Hz), 49.93, 54.73 (d, *J*_{(C-P)} = 2.5 Hz), 62.03, 62.14, 68.02, 71.36 (d, *J*_{(C-P)} = 2.5 Hz), 73.29, 80.49 (d, *J*_{(C-P)} = 6.2 Hz), 82.80 (d, *J*_{(C-P)} = 6.2 Hz), 92.72, 96.88, 122.81, 129.35, 129.40, 130.83, 131.43, 137.13, 150.73, 173.31, 175.45; ³¹P-NMR (CD₃OD, 202 MHz): δ 2.55 (one diastereoisomer in α-form), 1.95, 1.84 (two diastereoisomers in β-form)

### Example 60:

### Preparation of 2-acetamido-2-deoxy-3-O-[4-chlorophenyl(ethoxy-L-prolinyl)]phosphoramidate-D-glucopyranose (compound 83)

Prepared according to general procedure of Example 5 (addition at room temperature), from N-acetyl-D-glucosamine and 4-chlorophenyl(ethoxy-L-prolinyl)phosphorochloridate. The crude product was purified by flash chromatography on silica gel under gradient elution system of dichloromethane : methanol, 100:0 - 95:5. Compound 83 was isolated as a white solid.
¹H-NMR (CD₃OD, 500 MHz), mixture of four diastereoiomers, two major in α form and two minor in β form: 7.40 (2H, m), 7.39 (2H, d, *J* = 9.2 Hz), 7.21 (2H, d, *J* = 9.2 Hz), 6.17 (2H, d, *J* = 9.2 Hz), 5.12 (1H, d, *J* = 3.2 Hz), 4.71 (2H, m), 4.59 (1H, m), 4.44 (1H, m), 4.29 (2H, m), 4.11 (1H, dd, *J* = 7.3 Hz, *J* = 3.2 Hz), 3.87 (3H, m), 3.60 (1H, m), 3.51 (2H, m), 3.33 (1H, m), 2.21 (1H, m), 2.02 (3H, s), 2.00 (1H, m), 1.90 (2H, m), 1.81 (3H, s), 1.30 (3H, t, J = 7.3 Hz); ¹³C-NMR (CD₃OD, 125 MHz): δ 14.50, 22.81, 26.17 (d, *J*_{(C-P)} = 9.2 Hz), 32.40 (d, *J*_{(C-P)} = 9.3 Hz), 48.73, 54.67 (d, *J*_{(C-P)} = 2.9 Hz), 61.83 (d, *J*_{(C-P)} = 6.7 Hz), 62.39, 62.67, 62.71, 70.59 (d, *J*_{(C-P)} = 3.6 Hz), 71.70, 80.01 (d, *J*_{(C-P)} = 7.6 Hz), 92.74, 96.73, 123.05, 130.74, 150.88, 173.30, 176.08; ³¹P-NMR (CD₃OD, 202 MHz): δ 2.55 (one diastereoisomer as α-isomer), 2.01 (one diastereoisomer as β-isomer), 1.97 (one diastereoisomer as β-isomer)

### Example 61:

### Preparation of 2-deoxy-2-(pent-4-enoylamino)-3-O-[4-methoxyphenyl(benzyloxy-L-prolinyl)]phosphoramidate-D-glucopyranose (compound 84) and 2-deoxy-2-(pent-4-enoylamino)-4-O-[4-methoxyphenyl(benzyloxy-L-prolinyl)]phosphoramidate-D-glucopyranoside (compound 85)

Prepared according to general procedure of Example 5 (addition at room temperature), from N-pentenoyl-D-glucosamine and 4-methoxyphenyl(benzyloxy-L-prolinyl)phosphorochloridate. The crude product was purified twice by flash chromatography on silica gel under gradient elution system of dichloromethane : methanol, 100:0 - 97:3. Compound 85 was isolated as a white solid.
¹H-NMR (CD₃OD, 500 MHz), mixture of two diastereoisomers, one major as α form and one minor as β form: δ 7.3 (5H, m), 7.19 (2H, m), 5.87 (1H, m), 5.17 (3H, m), 5.12 (1H, d, *J* = 9.2 Hz), 5.10 (1H, d, *J* = 3.5 Hz), 4.77 (1H, m), 4.49 (1H, m), 4.44 (3H, m), 3.95 (1H, m), 3.56 (1H, m), 3.47 (1H, m), 3.35 (1H, m), 2.41 (4H), 2.14 (1H, m), 1.97 (1H, m), 1.84 (1H, m), 1.75 (1H, m); ¹³C-NMR (CD₃OD, 125 MHz): δ 26.14 (d, *J*_{(C-P)} = 8.7 Hz), 30.97, 32.31 (d, *J*_{(C-P)} = 8.6 Hz), 36.41, 48.27 (d, *J*_{(C-P)} = 4.0 Hz), 54.88 (d, *J*_{(C-P)} = 3.8 Hz), 56.10, 56.26, 61.17, 63.04, 68.12, 69.75, 71.99, 80.01 (d, *J*_{(C-P)} = 6.2 Hz), 92.31, 96.79, 115.83, 121. 30 (d, *J*_{(C-P)} = 4.4 Hz), 126.51 (d, *J*_{(C-P)} = 4.7 Hz), 129.37, 129.41, 130.96, 137.31, 138.47, 152.14, 174.91, 175.84; ³¹P-NMR (CD₃OD, 202 MHz): δ 3.21 (one diastereoisomer in β-form), 2.90 (one diastereoisomer in α-form), 2.31 (one diastereoisomer in α-form), 2.09 (one diastereoisomer in β-form).

Further elution afforded compound 84.
¹H-NMR (CD₃OD, 500 MHz), mixture of two diastereoisomers, one major as α form and one minor as β form : δ 7.38 (5H, m), 7.19 (2H, m), 5.87 (1H, m), 5.17 (3H, m), 5.12 (1H, d, *J* = 9.2 Hz), 5.12 (1H, d, *J =* 3.5 Hz), 4.62 (1H, d, J = 8.6 Hz), 4.52 (1H, m), 4.41 (1H, m), 4.31 (2H, m), 4.15 (1H, m) 3.85 (7H, m), 3.37 (2H, m), 3.30 (2H, m), 3.28 (1H, m), 2.41 (4H), 2.14 (1H, m), 1.97 (1H, m), 1.84 (1H, m), 1.75 (1H, m); ¹³C-NMR (CD₃OD, 125 MHz): δ 26.14 (d, *J*_{(C-P)} = = 8.7 Hz), 30.97, 32.31 (d, *J*_{(C-P)} = 8.6 Hz), 36.41, 48.27 (d, *J*_{(C-P)} = 4.0 Hz), 54.88 (d, *J*_{(C-P)} = 2.5 Hz), 56.10 , 56.26, 61.09, 62.50, 68.19, 68.95 (d, *J*_{(C-P)} = 2.5 Hz), 73.33, 80.05 (d, *J*_{(C-P)} = 6.2 Hz), 92.37, 92.65, 96.79, 115.83, 121. 30 (d, *J*_{(C-P)} = 4.4 Hz), 126.51 (d, *J*_{(C-P)} = 4.7 Hz), 129.37, 129.41, 130.96, 137.31, 138.47, 152.14, 174.91, 175.84; ³¹P-NMR (CD₃OD, 202 MHz): δ 3.22 (one diastereoisomer in α-form), 2.65, 2.61 (two diastereoisomers in β-form).

### Example 62:

### Preparation of 2-amino-2-deoxy-3-O-[4-methoxyphenyl(benaloxy-L-prolinyl)]phosphoramidate-D-glucopyranose (compound 86)

Prepared, from compound 84, according to general procedure of Example 8, to obtain compound 86 as a white solid (6mg, 26% yield).
¹H-NMR (CD₃OD, 500 MHz), mixture of two diastereoisomers, one major as α form and one minor as β form: δ 7.37 (5H, m), 7.12 (2H, m), 6.88 (2H, m), 5.31 (1H, m), 5.17 (2H, m), 4.31 (1H, m), 3.91 (2H, m), 3.81 (5H, m), 3.37 (1H, m), 3.30 (1H, m), 3.25 (1H, m), 3.24 (1H, m), 2.15 (1H, m), 1.91 (3H, m); ¹³C-NMR (CD₃OD, 125 MHz): δ 26.17 (d, *J*_{(C-P)} = 8.9 Hz), 32.27 (d, *J*_{(C-P)} = 8.6 Hz), 48.61 (d, *J*_{(C-P)} = 4.5 Hz), 49.63, 56.26, 62. 11 (d, *J*_{(C-P)} = 6.9 Hz), 67.01 (d, *J*_{(C-P)} = 4.9 Hz), 68.05, 71.18, 71.45, 80.71 (d, *J*_{(C-P)} = 6.8 Hz), 90.96, 94.88, 115.83, 115.81, 120.55 (d, *J*_{(C-P)} = 4.7 Hz), 126.25, 129.38, 129.44, 130.97, 137.24, 150.41 (d, *J*_{(C-P)} = 7.1 Hz), 174.97; ³¹P-NMR (CD₃OD, 202 MHz): δ 2.28 (one diastereoisomer in α-form), 2.10, 2.08 (two diastereoisomers in β-form); MS (ES+) m/z 553.10 (MH⁺).

### Example 63:

### Preparation of 2-deoxy-2-(pent-4-enoylamino)-3-O-[4-methoxyphenyl(ethoxy-L-prolinyl)]phosphoramidate-D-glucopyranose (compound 87)

Prepared according to general procedure of Example 5 (addition at room temperature), from N-pentenoyl-D-glucosamine and 4-methoxyphenyl(ethoxy-L-prolinyl)phosphorochloridate.
¹H-NMR (CD₃OD, 500 MHz), mixture of two diastereoisomers, one major as α form and one minor as β form: δ 7.18 (2H, m), 6.91 (2H, m), 5.79 (1H, m), 5.17 (1H, d, *J* = 3.5 Hz), 5.12 (1H, m), 5.02 (1H, d, J = 9.2 Hz), 4.41 (2H, m), 4.38 (2H, m), 4.25 (2H, m), 3.97 (1H, m), 3.83 (4H, m), 3.75 (3H), 3.67 (2H, m), 3.51 (1H, m), 3.38 (1H, m), 2.41 (2H), 2.25 (3H, m), 2.10 (1H, m), 1.84 (1H, m), 1.81 (1H, m); ¹³C-NMR (CD₃OD, 125 MHz): δ 14.53, 25.49 (d, *J*_{(C-P)} = 8.7 Hz), 30.89, 32.33 (d, *J*_{(C-P)} = 8.6 Hz), 36.89, 48.30 (d, *J*_{(C-P)} = 4.0 Hz), 54.98 (d, *J*_{(C-P)} = 2.5 Hz), 56.10, 61.09, 61.83, 61.89, 62.57, 68.95 (d, *J*_{(C-P)} = 2.5 Hz), 73.41, 80.01 (d, *J*_{(C-P)} = 6.2 Hz), 92.38, 92.65, 115.57, 115.63 (d, *J*_{(C-P)} = 4.4 Hz), 122.25 (d, *J*_{(C-P)} = 4.4 Hz), 138.55, 152.14, 175.15, 176.01, 176.28; ³¹P-NMR (CD₃OD, 202 MHz): δ 3.36 (one diastereoisomer in α-form), 2.81, 2.62 (two diastereoisomers in β-form); MS (ES+) *m*/z 537.20 (MH⁺).

### Example 64:

### Preparation of 2-acetamido-2-deoxy-3-O-[methyl(benzyloxy-L-alanyl)]phosphoramidate-D-glucopyranose (compound 88) and 2-acetamido-2-deoxy-4-O-[methyl(benzyloxy-L-alanyl)]phosphoramidate-D-glucopyranose (compound 89)

Prepared according to general procedure of Example 5, from methyl(benzyloxy-L-alanyl) phosphorochloridate and N-acetyl-D-glucosamine, to give a mixture of compound 88 and compound 89.
¹H-NMR (CD₃OD, 500 MHz): δ 7.38 (5H, m), 5.20 (2H, s), 5.11 (1H, *J* = 3.30 Hz), 5.10 (1H, J= 3.30 Hz), 4.66 (1H, *J* = 7.93 Hz), 4.63 (1H, *J* = 7.93 Hz), 4.47 (1H, m), 4.22 (1H, m), 4.08 (dd, *J* = 10.32 and 3.8 Hz), 4.01 (1H, m), 3.93 (1H, m), 3.86 (1H, m), 3.76 (3H, m), 3.68 (3H, d, *J*_{HP} = 11.68 Hz), 3.62 (3H, d, *J*_{HP} = 11.68 Hz), 3.58 (1H, t, *J* = 9.37 Hz), 2.01 (3H, s), 1.98 (3H, s), 1.97 (3H, s), 1.40 (3H, m). ¹³C-NMR (CD₃OD, 125 MHz): δ 20.58, 20.63, 22.77, 22.96, 51.46, 51.54, 54.28 (d, *J*_{(C-P)} = 6.2 Hz), 54.33 (d, *J*_{(C-P)} = 6.2 HZ), 54.50 (d, *J*_{(C-P)} = 3 Hz), 51.97, 62.28, 62.35, 62.51, 62.61, 68.03, 68.06, 70.99 (d, *J*_{(C-P)} = 2.4 Hz), 71.51, 71.53 (d, *J*_{(C-P)} = 5.5 Hz), 73.0, 77.50 (d, *J*_{(C-P)} = 6.0 Hz), 79.93 (d, *J*_{(C-P)} = 6.4 Hz), 82.04 (d, *J*_{(C-P)} = 6.6 Hz), 92.37, 92.81, 96.54, 96.86, 129.42, 129.66, 129.67, 137.31, 173.57, 173.66, 173.79, 173.88, 175.34 (d, *J*_{(C-P)} = 3.75 Hz), 175.38 (d, *J*_{(C-P)} = 3.75 Hz). MS (ES+) m/z 499.14 (MNa⁺).

### Example 65:

### Preparation of 2-acetamido-2-deoxy-3-O-[4-methoxyphenyl(benzyloxy-D-prolinyl)]phosphoramidate-D-glucopyranose (compound 90)

Prepared according to general procedure of Example 5, from from N-acetyl-D-glucosamine and 4-methoxyphenyl(benzoxy-D-prolinyl)phosphorochloridate. The crude product was purified twice by flash chromatography on silica gel under gradient elution system of dichloromethane : methanol, 100:0 - 95:5. Compound 90 was isolated as a white solid.
¹H-NMR (CD₃OD, 500 MHz), mixture of two diastereoisomers, one major as α form and one minor as β form: δ 7.34 (1H, m), 7.21 (4H, m), 6.89 (2H, d, *J* = 8.6 Hz), 6.71 (2H, d, *J* = 9.0 Hz), 5.11 (2H, m), 4.97 (1H, m), 4.76 (1H, m), 4.06 (2H, m), 3.96 (2H, m), 3.68 (5H, m), 3.16 (1H, m), 2.96 (1H, m), 1.88 (3H, s), 1.59 (1H, m), 1.50 (1H, m), 1.30 (1H, m), 1.24 (1H, m); ¹³C-NMR (CD₃OD, 125 MHz): δ 22.98, 25.90 (d, *J*_{(C-P)} = 8.5 Hz), 32.18, 48.74 (d, *J*_{(C-P)} = 5.5 Hz, overlap with the solvent), 55.59, 56.18, 61.80 (d, *J*_{(C-P)} = 6.5 Hz), 63.82, 67.82, 70.07, 74.01 (d, *J*_{(C-P)} = 3.5 Hz), 80.32 (d, *J*_{(C-P)} = 7.4 Hz), 93.46, 103.49, 115.71, 122.26 (d, *J*_{(C-P)} = 4.4 Hz), 128.01, 129.27, 129.46, 129.36, 129.77, 130.33, 137.45, 138.98, 145.5 (d, *J*_{(C-P)} = 6.7 Hz), 158.31, 173.43, 175.09; ³¹P-NMR (CD₃OD, 202 MHz): δ 3.18 (one diastereoisomer in α-form), 2.81, 2.67 (two diastereoisomers in β-form); MS (ES+) *m*/z 617.31 (MNa⁺).

### Example 66:

### Preparation 2-acetamido-2-deoxy-3-O-[4-methoxyphenyl(ethoxy-D-prolinyl)]phosphoramidate-D-glucopyranose (compound 91) and 2-acetamido-2-deoxy-4-O-[4-methoxyphenyl(ethoxy-D-prolinyl)]phosphoramidate-D-glucopyranose (compound 92)

Prepared according to general procedure of Example 5 (addition at room temperature), from N-acetyl-D-glucosamine and 4-methoxyphenyl(ethoxy-L-prolinyl)phosphorochloridate. The crude product was purified twice by flash chromatography on silica gel under gradient elution system of dichloromethane : methanol, 100:0 - 97:3. Compound 92 was isolated as a white solid.
¹H-NMR (CD₃OD, 500 MHz), mixture of four diastereoisomers, two major in α form and two minor in β form: δ 7.17 (2H, m), 7.11 (2H, m), 6.91 (4H, m), 5.12 (1H, d, J= 3.2 Hz), 4.61 (1H, m), 4.49 (1H, m), 4.41 (2H, m), 4.22 (2H, m), 4.09 (2H, m), 3.71 (3H, m), 3.51 (1H, m), 3.48 (1H, m), 3.31 (1H, m), 2.21 (1H, m), 2.00 (1H, m), 2.01 (3H, s), 1.90 (2H, m), 1.29 (3H, m); ¹³C-NMR (CD₃OD, 125 MHz): δ 14.45, 22.81, 26.16 (d, *J*_{(C-P)} = 9.2 Hz), 32.27 (d, *J*_{(C-P)} = 9.3 Hz), 48.29, 54.85, 56.12 (d, *J*_{(C-P)} = 6.7 Hz), 61.78 (d, *J*_{(C-P)} = 6.7 Hz), 62.46, 62.71, 66.83, 70.61 (d, *J*_{(C-P)} = 3.6 Hz), 71.78 (d, *J*_{(C-P)} = 3.9 Hz), 79.43 (d, *J*_{(C-P)} = 7.6 Hz), 92.72, 96.88, 115.68, 122.10 (d, *J*_{(C-P)} = 4.4 Hz), 145.48 (d, *J*_{(C-P)} = 7.6 Hz), 158.40, 173.29, 175.22, 176.13; ³¹P-NMR (CD₃OD, 202 MHz): δ 2.92 (one diastereoisomer as α-isomer), 2.81 (one diastereoisomer as β-isomer) 2.47 (one diastereoisomer as β-isomer), 2.13 (one diastereoisomer as α-isomer); MS (ES+) *m*/*z* 555.17 (MNa⁺).

Further elution with dichloromethane : methanol 97:3 afforded compound 91.
¹H-NMR (CD₃OD, 500 MHz), mixture of four diastereoisomers, two major in α form and two minor in β form): δ 7.01 (2H, d, *J* = 8.6 Hz), 6.98 (2H, d, *J* = 8.6 Hz), 6.83 (2H, d, *J* = 9.1 Hz), 6.79 (2H, d, *J* = 9.1 Hz), 5.00 (1H, d, *J* = 3.0 Hz), 4.53 (1H, m), 4.17 (1H, m), 4.06 (1H, q, *J* = 7.4 Hz), 3.97 (1H, m), 3.72 (1H, m), 3.69 (5H, m), 3.54 (1H, m), 3.46 (1H, m), 3.25 (1H, m), 2.09 (1H, m), 1.90 (3H, s), 1.88 (1H, m), 1.80 (5H, m), 1.69 (3H, s), 1.11 (3H, t, *J* = 7.3 Hz); ¹³C-NMR (CD₃OD, 125 MHz): δ 14.45, 22.81, 26.25 (d, *J*_{(C-P)} = 9.2 Hz), 32.33 (d, *J*_{(C-P)} = 9.3 Hz), 48.71, 54.91 (d, *J*_{(C-P)} = 3.0 Hz), 56.14, 62.16 (d, *J*_{(C-P)} = 6.7 Hz), 62.52, 62.70, 70.93 (d, *J*_{(C-P)} = 3.6 Hz), 73.93, 79.98 (d, *J*_{(C-P)} = 7.6 Hz), 92.61, 115.76, 122.02 (d, *J*_{*(*C-P)} = 4.4 Hz), 145.49 (d, *J*_{(C-P)} = 7.6 Hz), 158.39, 173.39, 175.26; ³¹P-NMR (CD₃OD, 202 MHz): δ 3.17 (one diastereoisomer as α-isomer), 2.83 (one diastereoisomer as α-isomer) 2.71 (one diastereoisomer as β-isomer), 2.62 (one diastereoisomer as β -isomer); MS (ES+) *m*/*z* 555.17 (MNa⁺).

### Example 67:

### Preparation of 2-deoxy-2-(pent-4-enoylamino)-4-O-[phenyl(ethoxy-L-prolinyl)]phosphoramidate-D-glucopyranose (compound 93)

Prepared according to general procedure of Example 5 (addition at room temperature), from *N*-pentenoyl-D-glucosamine and phenyl(ethoxy-L-prolinyl)phosphorochloridate. The crude product was purified twice by flash chromatography on silica gel under gradient elution system of dichloromethane : methanol 100:0 - 94:6. Compound 93 was isolated as a white solid.
¹H-NMR (CD₃OD, 500 MHz), mixture of two diastereoisomers, one major as α form and one minor as β form): δ 7.20 (2H, m), 7.01 (3H, m), 5.78 (1H, m), 5.11 (1H, d, *J* = 3.5 Hz), 5.10 (1H, m), 5.01 (1H, d, *J* = 9.2 Hz), 4.69 (1H, m), 4.41 (1H, m), 4.40 (3H, m), 4.25 (2H, m), 3.94 (1H, m), 3.55 (1H, m), 3.41 (1H, m), 3.35 (1H, m), 2.41 (2H), 2.21 (3H, m), 2.03 (1H, m), 1.83 (1H, m), 1.77 (1H, m); ¹³C-NMR (CD₃OD, 125 MHz) δ 14.53, 25.70 (d, *J*_{(C-P)} = 8.7 Hz), 30.91, 32.33 (d, *J*_{(C-P)} = 8.6 Hz), 36.79, 48.30 (d, *J*_{(C-P)} = 4.0 Hz), 54.98 (d, *J*_{(C-P)} = 2.9 Hz), 61.11, 61.83, 61.89, 62.77, 68.95, 72.01, 80.00 (d, *J*_{(C-P)} = 6.5 Hz), 92.32, 115.77, 121.36, 126.23, 130.80, 138.55, 152.16, 175.13, 176.00, 176.28; ³¹P-NMR (CD₃OD, 202 MHz): δ 3.19 (one diastereoisomer in β-form), 2.87 (one diastereoisomer in α-form), 2.30 (one diastereoisomer in α-form), 2.11 (one diastereoisomer in β-form).

### Example 68:

### Preparation of 2-amino-2-deoxy-4-O-[phenyl(ethoxy-L-prolinyl)]phosphoramidate-D-glucopyranose (compound 94)

Prepared as a white solid (8mg, 32% yield) according to general procedure of Example 8, starting from 2-deoxy-2-(pent-4-enoylamino)-4-*O*-[phenyl(ethoxy-L-prolinyl)]phosphoramidate-D-glucopyranose (compound 93 of Example 67).
¹H-NMR (CD₃OD, 500 MHz), mixture of two diastereoisomers, one major as α form and one minor as β form): δ 7.21 (2H, m), 7.03 (3H, m), 5.11 (1H, d, *J* = 3.5 Hz), 4.61 (1H, m), 4.40 (1H, m), 4.37 (3H, m), 4.23 (2H, m), 3.91 (1H, m), 3.51 (1H, m), 3.40 (1H, m), 3.35 (1H, m), 2.11 (1H, m), 1.90 (3H, m); ¹³C-NMR (CD₃OD, 125 MHz): δ 14.53, 25.70 (d, *J*_{(C-P)} = 8.7 Hz), 32.31 (d, *J*(_{C-P}) = 8.6 Hz), 48.34 (d, *J*(_{C}-_{P}) = 4.1 Hz), 50.01, 61.71, 61.80, 61.88, 62.99, 69.19, 72.00, 80.04 (d, *J*_{(C-P)} = 6.8 Hz), 90.72, 121.33, 126.13, 130.85, 152.01, 175.13; ³¹P-NMR (CD₃OD, 202 MHz): δ 3.19 (one diastereoisomer in β-form), 2.87 (one diastereoisomer in α-form), 2.30 (one diastereoisomer in α-form), 2.11 (one diastereoisomer in β-form).

### Biology

### Example 69: Effect of monosaccharide phosphoramidates on glycosaminoglycan release

### 1. Determination of the cytotoxicity of the compounds

A cytotoxicity assay was performed previous to the glycosaminoglycan release assay in order to determine the toxic effect of the compounds on the cells.

Cytotoxicity was quantified using the commercially available WST-1 assay (Roche Diagnostics GmbH).

The WST-1 assay measures the enzymatic activity of mitochondrial succinate-tetrazolium reductases, thus measuring the metabolic activity of the cells. The colour reaction only takes place when reductase enzymes are active and, therefore the conversion is used as a measure of viable cells. Because it determines the number of viable cells, the WST-1 assay is often used to determine cell proliferation and cytotoxicity.

On day 8 after collection of the culture medium, WST-1 (dissolved in culture medium) was added to the cartilage and allowed to be converted for 0.5 to 1 hour. After this, the cartilage was collected for GAG analysis and the amount of converted WST-1 is determined in the medium. The metabolic activity was expressed as a percentage of the control condition with DMSO 0.25%.

Metabolic activity values superior to 50% were considered acceptable in this study.

### 2. Effect of monosaccharide phosphoramidates on glycosaminoglycan release

Glycosaminoglycan (GAG) release is a test used to evaluate the effects of the compounds of the present invention on proteoglycan degradation induced by IL-1.

Full-depth articular cartilage were dissected from the metacarpophalangeal joints of calves of approximately 6 months of age or human cartilage from joint replacement surgery (approximately 4 mm diameter punches for bovine cartilage and 2 mm for human cartilage). After an equilibration period of 24 hours, explants were cultured for one day in 200 µl DMEM + hydrolyzed lactalbumin + 50 µg/ml vitamin C + penicillin/streptomycin + ITS (Insulin-Transferrin-Selenium) in the presence or absence of the compounds. After one day, GAG release was induced by addition of 10 ng of IL-1. Six days after IL-1 induction, the cartilage and culture medium were collected for analysis. During this six day period the culture medium was replaced every two days.

GAG release was analyzed using the Blyscan® colorimetric assay (Biocolor, Belfast, UK). To measure GAG content of the cartilage explants, the tissue was digested with papain. The culture medium of day 4, 6 and 8 was pooled before GAG analysis. GAG release was expressed as percentage of the total GAG content of the explants (i.e. GAG medium + GAG in papain digest).

A control condition with 0.25% DMSO was included because the test compounds were dissolved in DMSO. A condition with glucosamine hydrochloride was added for comparison.

Each culture condition was performed with cartilage of five different paws/patients to account for biological variation.

ANOVA followed by LSD *post-hoc* tests were used for determining differences between groups (p≤ 0.05 was considered significant).

Results in Tables 3 and 4 were expressed as a percentage reduction in GAG release compared to the control condition with DMSO stimulated with IL-1. Compounds that showed a non statistically significant reduction of the GAG release compared to IL-1-stimulated in the presence of 0.25% DMSO condition were considered inactive.

### Results

Tables 3 and 4 summarize the results of the GAG release inhibition assay. None of the compounds in these tables showed cytotoxic effect at the studied doses.

**Table 3: GAG release inhibition in bovine cartilage explants**

| | **25 µM** | **50 µM** | **100 µM** | **250 µM** |
|---|---|---|---|---|
| Compound 31 | ND | ND | 23%* | ND |
| Compound 32 | ND | ND | 27.3%* | 40.9%* |
| Reference compound 1 | ND | ND | 0 (Inactive) | ND |
| Compound 1 | ND | ND | 32%* | ND |
| Reference compound 2 | ND | ND | 0 (Inactive) | ND |
| Compound 69 | 22.7%* | ND | ND | ND |
| Reference compound 3 | ND | 4.5% (Inactive) | 4.5% (Inactive) | 4.5% (Inactive) |
| Compound 16 | ND | ND | 27%* | ND |
| Reference compound 4 | ND | ND | 3% (Inactive) | ND |
| Compound 63 | ND | ND | 31%* | ND |
| Reference compound 5 | ND | ND | 4% (Inactive) | ND |
| Compound 74 | ND | 10% (Inactive) | 25%* | 30%* |
| Compound 80 | ND | ND | ND | 44.4%* |
| Compound 87 | ND | ND | ND | 33.3%* |
| Compound 58 | ND | ND | 41%* | ND |
| Compound 61 | ND | ND | 47%* | ND |
| Glucosamine Hydrochloride | ND | ND | 0 (Inactive) | 0 (Inactive) |

| | | | | |
|---|---|---|---|---|
| The asterisk indicates p≤ 0.05 versus the 0.25% DMSO + IL1 condition; ND= Not determined Reference compounds 1-5 are phosphoramidates in position 6- of the glucosamine ring (see Table 5). | | | | |

**Table 4: GAG release inhibition in human cartilage explants**

| | **50 µM** | **100 µM** | **250 µM** |
|---|---|---|---|
| Compound 1 | 23.5%* | 29.5%* | 32.8%* |
| Glucosamine Hydrochloride | 7.8% (Inactive) | 11.9% (Inactive) | 11.9% (Inactive) |

| | | | |
|---|---|---|---|
| The asterisk indicates p≤ 0.05 versus the 0.25% DMSO + IL1 condition | | | |

**Table 5: Reference compounds 1-5**

| | | | | | | |
|---|---|---|---|---|---|---|
| 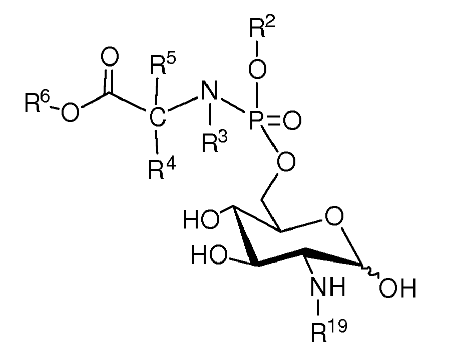 | | | | | | |

| **Reference Compound** | **R²** | **R⁵** | **R³** | **R⁴** | **R⁶** | **R¹⁹** |
|---|---|---|---|---|---|---|
| 1 | 4-(MeO)Ph | H | -(CH₂)₃- | | Et | Ac |
| 2 | 4-(MeO)Ph | H | -(CH₂)₃- | | PhCH₂ | Ac |
| 3 | 4-(MeO)Ph | H | -(CH₂)₃- | | nPen | Ac |
| 4 | 4-(MeO)Ph | H | H | iPr | PhCH₂ | Ac |
| 5 | 4-(MeO)Ph | H | H | iPr | PhCH₂ | H |

As can be observed in Table 3 reference compounds 1-5 (phosphoramidates in position 6 of the glucosamine ring) and the aminosugar hydrochloride did not caused any statistically significant reduction in the GAG release induced by IL-1 in bovine explants. Thus, these compounds can be considered inactive. On the contrary,compounds 31, 32, 1, 69, 16, 63, 74, 80, 87, 58 and 61, which are phosphoramidates in position 4- and 3- of the glucosamine ring showed a statistically significant reduction of the GAG release compared to the culture stimulated with IL-1 in the presence of 0.25% DMSO.

Additionally, the anti-catabolic activity of compound 1 was confirmed in human cartilage explants. Compound 1 showed a statistically significant activity in the reduction of GAG release at the doses 50 µM, 100 µM and 250 µM, whereas glucosamine hydrochloride was inactive at the same doses (see Table 4).

Considering the reduction in the GAG release induced by IL-1 in both, bovine or human cartilage, it can be stated that the compounds of the present invention may be useful for the treatment of osteoarthritis and other musculoskeletal diseases.

## Claims

1. A compound of formula (I), 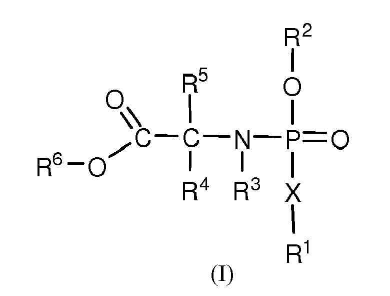 wherein:
R¹ is selected from A and B, 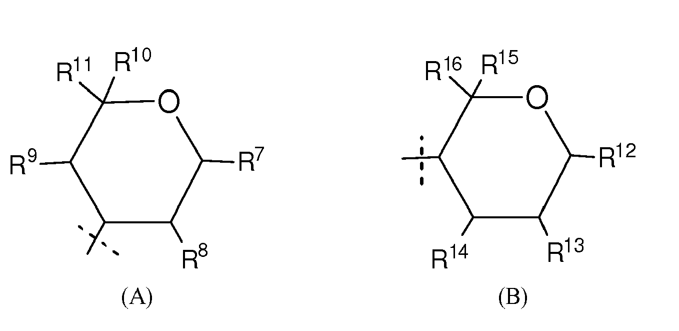
X is selected from -O- and -CH₂-;
R² is selected from the group consisting of hydrogen, C₁-C₁₆ alkyl, C₂-C₁₆ alkenyl, C₂-C₁₆ alkynyl, C₃-C₂₀ cycloalkyl, C₃-C₂₀ cycloalkenyl, C₄-C₂₀ cycloalkynyl, C₇-C₂₀ aralkyl, C₇-C₂₀ alkaryl and C₅-C₂₀ aryl, wherein each of said aralkyl, alkaryl or aryl is optionally substituted with one or more substituents independently selected from the group consisting of halogen, C₁-C₁₀ alkyl, hydroxy, C₁-C₁₀ alcoxy, alcoxyalkyl, amino, C₁-C₁₀ alkylamino, hydroxyakylamino and C₁-C₁₀ alcanoyloxyl;
R³ is selected from the group consisting of hydrogen, C₁-C₁₀ alkyl and C₅-C₁₀ aryl; or R³ and R⁴ together are (CH₂)₃; or R³ and R⁵ together are (CH₂)₃, wherein the group (CH₂)₃ is optionally substituted with one or more substituents independently selected from the group consisting of C₁-C₆ alkyl, hydroxy, C₁-C₆ alcoxy, amino, carboxyl and esters thereof, alcanoyloxyl and halogen;
R⁴ and R⁵ are independently selected from the group consisting of hydrogen, C₁-C₁₆ alkyl, C₂-C₁₆ alkenyl, C₂-C₁₆ alkynyl, C₃-C₂₀ cycloalkyl, C₃-C₂₀ cycloalkenyl, C₄-C₂₀ cycloalkynyl, C₇-C₂₀ aralkyl, C₇-C₂₀ alkaryl and C₅-C₂₀ aryl, wherein each of said alkyl, alkenyl, alkynyl, cycloalkyl, cycloalkenyl, cycloalkynyl, aralkyl, alkaryl or aryl is optionally substituted with one or more substituents independently selected from the group consisting of hydroxy, C₁-C₆ alcoxy, thiol, C₁-C₆ alkylthio, carboxyl and esters thereof, amido, carbamoyl, imino, H₂NC(=NH)NH-, amino, aminoalkyl and C₁-C₆ alkylamino; or R⁴ and R⁵ together with the carbon atom to which they are attached, form a ring from 3 to 6 members, selected from the group consisting of C₃-C₆ cycloalkyl, C₃-C₆ cycloalkenyl and C₄-C₆ cycloalkynyl; or R⁴ and R³ together are (CH₂)₃; or R⁵ and R³ together are (CH₂)₃, wherein the group (CH₂)₃ is optionally substituted with one or more substituents independently selected from the group consisting of C₁-C₆ alkyl, hydroxy, C₁-C₆ alcoxy, amino, carboxyl and esters thereof, alcanoyloxyl and halogen;
R⁶ is selected from the group consisting of hydrogen, protecting group, C₁-C₁₆ alkyl, C₂-C₁₆ alkenyl, C₂-C₁₆ alkynyl, C₃-C₂₀ cycloalkyl, C₃-C₂₀ cycloalkenyl, C₄-C₂₀ cycloalkynyl, C₇-C₂₀ aralkyl, C₇-C₂₀ alkaryl and C₅-C₂₀ aryl, wherein each of said alkyl, alkenyl, alkynyl, cycloalkyl, cycloalkenyl, cycloalkynyl, aralkyl, alkaryl or aryl is optionally substituted with one or more substituents independently selected from the group consisting of halogen, hydroxy, C₁-C₆ alcoxy, thiol and amino;
R⁷, R⁸, R⁹, R¹⁰, R¹², R¹³, R¹⁴ and R¹⁵ are independently selected from the group consisting of hydrogen, N₃, halogen, -OR¹⁷, -SR¹⁸, -NHR¹⁹, -NR¹⁹₂ and C 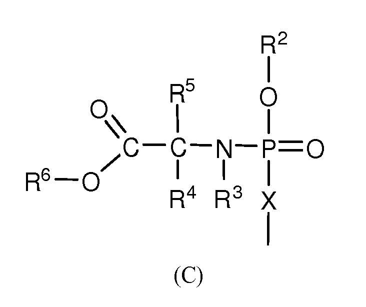
wherein X, R², R³, R⁴, R⁵ and R⁶ are as hereinbefore defined for formula (I), and wherein R¹⁷, R¹⁸ and R¹⁹ are independently selected from the group consisting of hydrogen, protecting group, C₁-C₁₅ alkyl, C₂-C₁₅ alkenyl, C₂-C₁₅ alkynyl, C₃-C₂₀ cycloalkyl, C₃-C₂₀ cycloalkenyl, C₄-C₂₀ cycloalkynyl, C₇-C₂₀ aralkyl, C₇-C₂₀ alkaryl, C₅-C₂₀ aryl, -C(O)R²⁰ and -C(O)OR²⁰, wherein R²⁰ is selected from the group consisting of C₁-C₁₅ alkyl, C₂-C₁₅ alkenyl, C₂-C₁₅ alkynyl, C₇-C₂₀ aralkyl, C₇-C₂₀ alkaryl and C₅-C₂₀ aryl;
R¹¹ and R¹⁶ are independently selected from the group consisting of -CH₃, -CH₂-halogen, -CH₂OR¹⁷, -CH₂SR¹⁸, -CH₂NHR¹⁹, -CH₂NR¹⁹₂ and -C(O)OR²¹, wherein R¹⁷, R¹⁸ and R¹⁹ are independently selected from the group consisting of hydrogen, protecting group, C₁-C₁₅ alkyl, C₂-C₁₅ alkenyl, C₂-C₁₅ alkynyl, C₃-C₂₀ cycloalkyl, C₃-C₂₀ cycloalkenyl, C₄-C₂₀ cycloalkynyl, C₇-C₂₀ aralkyl, C₇-C₂₀ alkaryl, C₅-C₂₀ aryl, -C(O)R²⁰ and -C(O)OR²⁰, wherein R²⁰ is selected from the group consisting of C₁-C₁₅ alkyl, C₂-C₁₅ alkenyl, C₂-C₁₅ alkynyl, C₇-C₂₀ aralkyl, C₇-C₂₀ alkaryl and C₅-C₂₀ aryl, and wherein R²¹ is selected from the group consisting of hydrogen, protecting group, C₁-C₁₅ alkyl, C₇-C₂₀ aralkyl and C₇-C₂₀ alkaryl;
or any pharmaceutically acceptable salts, solvates and prodrugs thereof.

2. The compound according to claim 1,
wherein:
R² is selected from the group consisting of C₁-C₆ alkyl, C₇-C₁₂ aralkyl, C₇-C₁₂ alkaryl and C₅-C₁₂ aryl, wherein each of said aralkyl, alkaryl or aryl is optionally substituted with one or more substituents independently selected from the group consisting of halogen, C₁-C₆ alkyl, hydroxy, C₁-C₆ alcoxy, amino and C₁-C₆ alcanoyloxyl;
R³ is selected from hydrogen and C₁-C₆ alkyl; or R³ and R⁴ together are (CH₂)₃; or R³ and R⁵ together are (CH₂)₃, wherein the group (CH₂)₃ is optionally substituted with one or more substituents independently selected from the group consisting of C₁-C₆ alkyl, hydroxy, C₁-C₆ alcoxy, amino, carboxyl and esters thereof, alcanoyloxyl and halogen;
R⁴ and R⁵ are independently selected from the group consisting of hydrogen, C₁-C₆ alkyl and C₇-C₁₂ aralkyl, wherein each of said alkyl or aralkyl is optionally substituted with a substituent selected from the group consisting of hydroxy, C₁-C₆ alcoxy, thiol, C₁-C₆ alkylthio, carboxyl and esters thereof, amido, carbamoyl, imino, H₂NC(=NH)NH-, amino, aminoalkyl and C₁-C₆ alkylamino; or R⁴ and R³ together are (CH₂)₃; or R⁵ and R³ together are (CH₂)₃, wherein the group (CH₂)₃ is optionally substituted with one or more substituents independently selected from the group consisting of C₁-C₆ alkyl, hydroxy, C₁-C₆ alcoxy, amino, carboxyl and esters thereof, alcanoyloxyl and halogen;
R⁶ is selected from the group consisting of hydrogen, C₁-C₆ alkyl, C₂-C₆ alkenyl, C₃-C₇ cycloalkyl, C₇-C₁₂ aralkyl, C₇-C₁₂ alkaryl and C₅-C₁₂ aryl, wherein each of said alkyl, alkenyl, cycloalkyl, aralkyl, alkaryl or aryl is optionally substituted with one or more substituents independently selected from the group consisting of halogen, hydroxy, C₁-C₆ alcoxy, thiol and amino;
R⁷, R⁸, R⁹, R¹⁰, R¹², R¹³, R¹⁴ and R¹⁵ are independently selected from the group consisting of hydrogen, -OR¹⁷, -SR¹⁸, -NHR¹⁹ and C 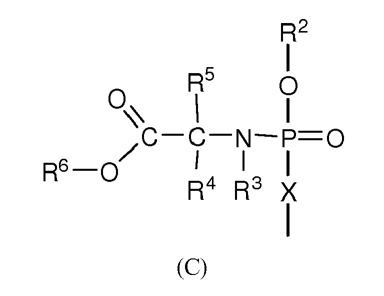
wherein X, R², R³, R⁴, R⁵ and R⁶ are as hereinbefore defined for formula (I), and wherein R¹⁷, R¹⁸ and R¹⁹ are independently selected from the group consisting of hydrogen, C₁-C₆ alkyl, C₇-C₁₂ aralkyl, C₇-C₁₂ alkaryl, C₅-C₁₂ aryl, -C(O)R²⁰ and -C(O)OR²⁰, wherein R²⁰ is selected from the group consisting of C₁-C₁₂ alkyl, C₂-C₁₂ alkenyl, C₇-C₁₂ aralkyl, C₇-C₁₂ alkaryl and C₅-C₁₂ aryl;
R¹¹ and R¹⁶ are independently selected from the group consisting of -CH₃, -CH₂-halogen, -CH₂OR¹⁷, -CH₂SR¹⁸, -CH₂NHR¹⁹, -CH₂NR¹⁹₂ and -C(O)OR²¹, wherein R¹⁷, R¹⁸ and R¹⁹ are independently selected from the group consisting of hydrogen, C₁-C₆ alkyl, C₇-C₁₂ aralkyl, C₇-C₁₂ alkaryl, C₅-C₁₂ aryl, -C(O)R²⁰ and -C(O)OR²⁰, wherein R²⁰ is selected from the group consisting of C₁-C₁₂ alkyl, C₂-C₁₂ alkenyl, C₇-C₁₂ aralkyl, C₇-C₁₂ alkaryl and C₅-C₁₂ aryl, and wherein R²¹ is selected from the group consisting of hydrogen, C₁-C₁₀ alkyl, C₇-C₁₂ aralkyl and C₇-C₁₂ alkaryl.

3. The compound according to claim 1 or 2, wherein R² is selected from the group consisting of methyl, ethyl, n-propyl, isopropyl, phenyl, naphthyl, pyridyl and benzyl, wherein each of said phenyl, naphthyl, pyridyl or benzyl is optionally substituted with a substituent selected from the group consisting of halogen, C₁-C₆ alkyl, hydroxy and C₁-C₆ alcoxy.

4. The compound according to anyone of claims 1 to 3, wherein R³ is selected from hydrogen and methyl; or R³ and R⁴ together are (CH₂)₃; or R³ and R⁵ together are (CH₂)₃, wherein the group (CH₂)₃ is optionally substituted with one or more substituents independently selected from the group consisting of C₁-C₆ alkyl, hydroxy, C₁-C₆ alcoxy, amino, carboxyl and esters thereof, alcanoyloxyl and halogen.

5. The compound according to anyone of claims 1 to 4, wherein R⁴ and R⁵ are independently selected from the group consisting of hydrogen, methyl, ethyl, n-propyl, isopropyl, butyl, isobutyl, 1-methylpropyl, hydroxymethyl, 1-hydroxyethyl, mercaptomethyl, methyltioethyl, aminobutyl, benzyl, hydroxybenzyl, indolylmethyl, imidazolylmethyl, carboxymethyl, carboxyethyl, H₂NC(=NH)NH-(CH₂)₃-, carbamoylmethyl and carbamoylethyl; or R⁴ and R³ together are (CH₂)₃; or R⁵ and R³ together are (CH₂)₃, wherein the group (CH₂)₃ is optionally substituted with one or more substituents independently selected from the group consisting of C₁-C₆ alkyl, hydroxy, C₁-C₆ alcoxy, amino, carboxyl and esters thereof, alcanoyloxyl and halogen.

6. The compound according to anyone of claims 1 to 5, wherein R⁶ is selected from the group consisting of hydrogen, methyl, ethyl, n-propyl, isopropyl, n-butyl, 2-butyl, tert-butyl, n-pentyl, 3-pentyl, 2,2-dimethylpropyl, cyclohexyl, vinyl, propenyl, benzyl, phenylethyl, phenylpropyl and phenyl.

7. The compound according to anyone of claims 1 to 6, wherein R³ and R⁴ or R⁵, together are (CH₂)₃-

8. The compound according to anyone of claims 1 to 7, wherein X is -O-

9. The compound according to anyone of claims 1 to 8, wherein the compound of formula (I) has the formula (Ia), 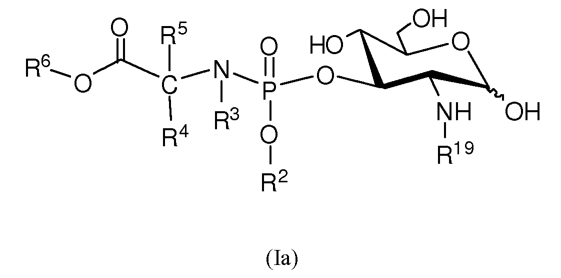
wherein R¹⁹ is selected from the group consisting of hydrogen, -C(O)R²⁰ and -C(O)OR²⁰, wherein R²⁰ is selected from the group consisting of C₁-C₁₀ alkyl, C₂-C₁₀ alkenyl, C₇-C₁₂ aralkyl, C₇-C₁₂ alkaryl and C₅-C₁₂ aryl;
R², R³, R⁴, R⁵ and R⁶ are as defined in anyone of claims 1 to 8.

10. The compound according to claim 9, wherein the compound of formula (Ia) has the formula (Ib), 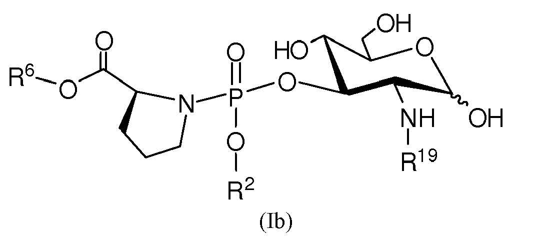
wherein R² is selected from the group consisting of methyl, ethyl, phenyl, naphthyl, pyridyl, benzyl, 4-metoxyphenyl and 4-chlorophenyl;
R⁶ is selected from the group consisting of hydrogen, methyl, ethyl, n-propyl, isopropyl, n-butyl, 2-butyl, tert-butyl, n-pentyl, 3-pentyl, 2,2-dimethylpropyl, cyclohexyl, vinyl, benzyl, phenylethyl and phenyl;
R¹⁹ is selected from the group consisting of hydrogen, -C(O)CH₃, -C(O)OCH₂Ph and -C(O)CH₂CH=CH₂.

11. The compound according to anyone of claims 1 to 8, wherein the compound of formula (I) has the formula (Ic), 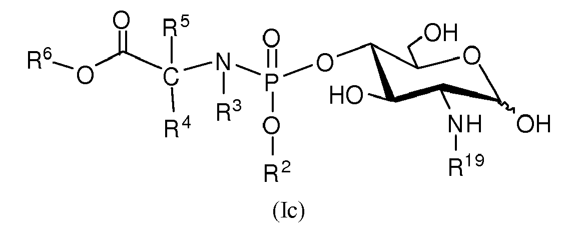
wherein R¹⁹ is selected from the group consisting of hydrogen, -C(O)R²⁰ and -C(O)OR²⁰, wherein R²⁰ is selected from the group consisting of C₁-C₁₀ alkyl, C₂-C₁₀ alkenyl, C₇-C₁₂ aralkyl, C₇-C₁₂ alkaryl and C₅-C₁₂ aryl;
R², R³, R⁴, R⁵ and R⁶ are as defined in anyone of claims 1 to 8.

12. The compound according to claim 11, wherein the compound of formula (Ic) has the formula (Id), 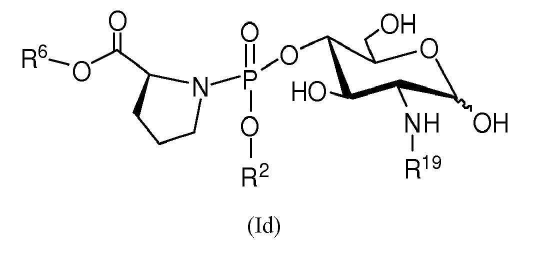
wherein R² is selected from the group consisting of methyl, ethyl, phenyl, naphthyl, pyridyl, benzyl, 4-metoxyphenyl and 4-chlorophenyl;
R⁶ is selected from the group consisting of hydrogen, methyl, ethyl, n-propyl, isopropyl, n-butyl, 2-butyl, *tert*-butyl, n-pentyl, 3-pentyl, 2,2-dimethylpropyl, cyclohexyl, vinyl, benzyl, phenylethyl and phenyl;
R¹⁹ is selected from the group consisting of hydrogen, -C(O)CH₃, -C(O)OCH₂Ph and -C(O)CH₂CH=CH₂.

13. The compound according to claim 9 or 11, wherein
R² is selected from the group consisting of methyl, ethyl, phenyl, naphthyl, pyridyl, benzyl, *p*-metoxyphenyl and *p*-chlorophenyl;
R³ is selected from hydrogen and methyl;
R⁴ and R⁵ are independently selected from the group consisting of hydrogen, methyl, ethyl, n-propyl, isopropyl, isobutyl, 1-methylpropyl, hydroxymethyl, 1-hydroxyethyl, mercaptomethyl, methyltioethyl, aminobutyl, benzyl, hydroxybenzyl, indolylmethyl, imidazolylmethyl, carboxymethyl, carboxyethyl, H₂NC(=NH)NH-(CH₂)₃-, carbamoylmethyl and carbamoylethyl;
R⁶ is selected from the group consisting of hydrogen, methyl, ethyl, n-propyl, isopropyl, n-butyl, 2-butyl, *tert*-butyl, n-pentyl, 3-pentyl, 2,2-dimethylpropyl, cyclohexyl, vinyl, benzyl, phenylethyl and phenyl;
R¹⁹ is selected from the group consisting of hydrogen, -C(O)CH₃, -C(O)OCH₂Ph and -C(O)CH₂CH=CH₂.

14. The compound according to claim 1, wherein the compound of formula (I) is selected from
2-acetamido-2-deoxy-3-*O*-[4-methoxyphenyl(benzyloxy-L-alanyl)]phosphoramidate-D-glucopyranose;
2-acetamido-2-deoxy-4-*O*-[4-methoxyphenyl(benzyloxy-L-alanyl)]phosphoramidate-D-glucopyranose;
benzyl 2-acetamido-2-deoxy-3-*O*-[4-methoxyphenyl(methoxy-L-alanyl)]phosphoramidate-D-glucopyranoside;
benzyl 2-acetamido-2-deoxy-4-*O*-[4-methoxyphenyl(methoxy-L-alanyl)]phosphoramidate-D-glucopyranoside;
methyl 2-acetamido-2-deoxy-3-*O*-[4-methoxyphenyl(benzyloxy-L-alanyl)]phosphoramidate-D-glucopyranoside;
methyl 2-benzyloxycarbonylamino-2-deoxy-3-*O*-[4-methoxyphenyl(ethoxy-L-alanyl)]phosphoramidate-D-glucopyranoside;
2-acetamido-2-deoxy-3-*O*-[4-methoxyphenyl(benzyloxy-dimethylglycinyl)]phosphoramidate-D-glucopyranose;
2-acetamido-2-deoxy-4-*O*-[4-methoxyphenyl(benzyloxy-dimethylglycinyl)]phosphoramidate-D-glucopyranose;
2-acetamido-2-deoxy-3-*O*-[4-methoxyphenyl(benzyloxy-L-isoleucinyl)]phosphoramidate-D-glucopyranose;
2-acetamido-2-deoxy-4-*O*-[4-methoxyphenyl(benzyloxy-L-isoleucinyl)]phosphoramidate-D-glucopyranose;
2-acetamido-2-deoxy-3-*O*-[4-methoxyphenyl(benzyloxy-L-leucinyl)]phosphoramidate-D-glucopyranose;
2-acetamido-2-deoxy-4-*O*-[4-methoxyphenyl(benzoxy-L-leucinyl)]phosphoramidate-D-glucopyranose;
2-acetamido-2-deoxy-3-*O*-[4-methoxyphenyl(benzyloxy-L-glycinyl)]phosphoramidate-D-glucopyranose;
2-acetamido-2-deoxy-4-*O*-[4-methoxyphenyl(benzoxy-L-glycinyl)]phosphoramidate-D-glucopyranose;
2-acetamido-2-deoxy-3-*O*-[4-methoxyphenyl(benzyloxy-L-methionyl)]phosphoramidate-D-glucopyranose;
2-acetamido-2-deoxy-4-*O*-[4-methoxyphenyl(benzoxy-L-methionyl)]phosphoramidate-D-glucopyranose;
2-deoxy-2-(pent-4-enoylamino)-3-*O*-[4-methoxyphenyl(benzyloxy-L-alanyl)]phosphoramidate-D-glucopyranose;
2-amino-2-deoxy-3-*O*-[4-methoxyphenyl(benzoxy-L-alanyl)]phosphoramidate-D-glucopyranose;
2-acetamido-2-deoxy-3-*O*-[4-methoxyphenyl(benzyloxy-D-alanyl)]phosphoramidate-D-glucopyranose;
2-acetamido-2-deoxy-4-*O*-[4-methoxyphenyl(benzyloxy-D-alanyl)]phosphoramidate-D-glucopyranose;
2-acetamido-2-deoxy-3-*O*-[1-naphtyl(benzyloxy-L-alanyl)]phosphoramidate-D-glucopyranose;
2-acetamido-2-deoxy-3-*O*-[4-methoxyphenyl(ethoxy-L-glycinyl)]phosphoramidate-D-glucopyranose;
2-acetamido-2-deoxy-4-*O*-[4-methoxyphenyl(ethoxy-L-glycinyl)]phosphoramidate-D-glucopyranose;
2-acetamido-2-deoxy-3-*O*-[methyl(benzyloxy-L-alanyl)]phosphoramidate-D-glucopyranose;
2-acetamido-2-deoxy-4-*O*-[methyl(benzyloxy-L-alanyl)]phosphoramidate-D-glucopyranose; benzyl 2-acetamido-2-deoxy-4,6-*O*-benzylidene-3-*O*-[4-methoxyphenyl(ethoxy-L-norvalinyl)]phosphoramidate-D-glucopyranoside;
2-acetamido-2-deoxy-3-*O*-[4-methoxyphenyl(ethoxy-L-norvalinyl)]phosphoramidate-D-glucopyranose;
2-acetamido-2-deoxy-3-*O*-[4-methoxyphenyl(benzyloxy-L-phenylalanyl)]phosphoramidate-D-glucopyranose;
2-acetamido-2-deoxy-3-*O*-[4-methoxyphenyl(benzyloxy-L-prolinyl)]phosphoramidate-D-glucopyranose;
2-acetamido-2-deoxy-4-*O*-[4-methoxyphenyl(benzyloxy-L-prolinyl)]phosphoramidate-D-glucopyranose;
methyl 2-acetamido-2-deoxy-3-*O*-[4-methoxyphenyl(benzyloxy-L-prolinyl)]phosphoramidate-D-glucopyranoside;
2-acetamido-2-deoxy-3-*O*-[4-methoxyphenyl(ethoxy-L-prolinyl)]phosphoramidate-D-glucopyranose;
2-acetamido-2-deoxy-4-*O*-[4-methoxyphenyl(ethoxy-L-prolinyl)]phosphoramidate-D-glucopyranose;
2-acetamido-2-deoxy-3-O-[4-methoxyphenyl(n-butoxy-L-prolinyl)]phosphoramidate-D-glucopyranose;
2-acetamido-2-deoxy-4-*O*-[4-methoxyphenyl(n-butoxy-L-prolinyl)]phosphoramidate-D-glucopyranose;
2-acetamido-2-deoxy-3-*O*-[4-methoxyphenyl(isopropoxy-L-prolinyl)]phosphoramidate-D-glucopyranose;
2-acetamido-2-deoxy-4-*O*-[4-methoxyphenyl(isopropoxy-L-prolinyl)]phosphoramidate-D-glucopyranose;
benzyl 2-acetamido-2-deoxy-4,6-*O*-benzylidene-3-*O*-[4-methoxyphenyl(*tert*-butoxy-L-prolinyl)]phosphoramidate-D-glucopyranoside;
2-acetamido-2-deoxy-3-*O*-[4-methoxyphenyl(*tert*-butoxy-L-prolinyl)]phosphoramidate-D-glucopyranose;
2-acetamido-2-deoxy-3-*O*-[4-methoxyphenyl(2-butoxy-L-prolinyl)]phosphoramidate-D-glucopyranose;
2-acetamido-2-deoxy-4-*O*-[4-methoxyphenyl-(2-butoxy-L-prolinyl)]phosphoramidate-D-glucopyranose;
2-acetamido-2-deoxy-3-*O*-[4-methoxyphenyl(cyclohexyloxy-L-prolinyl)]phosphoramidate-D-glucopyranose;
2-acetamido-2-deoxy-4-*O*-[4-methoxyphenyl(cyclohexyloxy-L-prolinyl)]phosphoramidate-D-glucopyranose;
2-acetamido-2-deoxy-3-*O*-[4-methoxyphenyl(methoxy-L-prolinyl)]phosphoramidate-D-glucopyranose;
2-acetamido-2-deoxy-4-*O*-[4-methoxyphenyl(methoxy-L-prolinyl)]phosphoramidate-D-glucopyranose;
2-acetamido-2-deoxy-3-*O*-[4-methoxyphenyl(n-propoxy-L-prolinyl)]phosphoramidate-D-glucopyranose;
2-acetamido-2-deoxy-4-*O*-[4-methoxyphenyl(n-propoxy-L-prolinyl)]phosphoramidate-D-glucopyranose;
2-acetamido-2-deoxy-3-*O*-[4-methoxyphenyl(n-pentoxy-L-prolinyl)]phosphoramidate-D-glucopyranose;
2-acetamido-2-deoxy-4-*O*-[4-methoxyphenyl(n-pentoxy-L-prolinyl)]phosphoramidate-D-glucopyranose;
2-acetamido-2-deoxy-3-*O*-[4-methoxyphenyl(3-pentoxy-L-prolinyl)]phosphoramidate-D-glucopyranose;
2-acetamido-2-deoxy-4-*O*-[4-methoxyphenyl(3-pentoxy-L-prolinyl)]phosphoramidate-D-glucopyranose;
2-acetamido-2-deoxy-3-*O*-[4-methoxyphenyl(neopentoxy-L-prolinyl)]phosphoramidate-D-glucopyranose;
2-acetamido-2-deoxy-4-*O*-[4-methoxyphenyl(neopentoxy-L-prolinyl)]phosphoramidate-D-glucopyranose;
2-acetamido-2-deoxy-3-*O*-[phenyl(benzyloxy-L-prolinyl)]phosphoramidate-D-glucopyranose;
2-acetamido-2-deoxy-4-*O*-[4-phenyl(benzyloxy-L-prolinyl)]phosphoramidate-D-glucopyranose;
2-acetamido-2-deoxy-3-*O*-[phenyl(ethoxy-L-prolinyl)]phosphoramidate-D-glucopyranose;
2-acetamido-2-deoxy-4-*O*-[phenyl(ethoxy-L-prolinyl)]phosphoramidate-D-glucopyranose;
2-acetamido-2-deoxy-3-*O*-[1-naphtyl(benzyloxy-L-prolinyl)]phosphoramidate-D-glucopyranose;
2-acetamido-2-deoxy-4-*O*-[1-naphtyl(benzyloxy-L-prolinyl)]phosphoramidate-D-glucopyranose;
2-acetamido-2-deoxy-3-*O*-[1-naphtyl(ethoxy-L-prolinyl)]phosphoramidate-D-glucopyranose;
2-acetamido-2-deoxy-4-*O*-[1-naphtyl(ethoxy-L-prolinyl)]phosphoramidate-D-glucopyranose;
2-acetamido-2-deoxy-3-*O*-[4-chlorophenyl(benzyloxy-L-prolinyl)]phosphoramidate-D-glucopyranose;
2-acetamido-2-deoxy-4-*O*-[4-chlorophenyl(benzoxy-L-prolinyl)]phosphoramidate-D-glucopyranose;
2-acetamido-2-deoxy-3-*O*-[4-chlorophenyl(ethoxy-L-prolinyl)]phosphoramidate-D-glucopyranose;
2-acetamido-2-deoxy-4-*O*-[4-chlorophenyl(ethoxy-L-prolinyl)]phosphoramidate-D-glucopyranose;
2-deoxy-2-(pent-4-enoylamino)-3-*O*-[4-methoxyphenyl(benzyloxy-L-prolinyl)]phosphoramidate-D-glucopyranose;
2-deoxy-2-(pent-4-enoylamino)-4-*O*-[4-methoxyphenyl(benzyloxy-L-prolinyl)]phosphoramidate-D-glucopyranoside;
2-amino-2-deoxy-3-*O*-[4-methoxyphenyl(benzyloxy-L-prolinyl)]phosphoramidate-D-glucopyranose;
2-amino-2-deoxy-4-*O*-[4-methoxyphenyl(benzyloxy-L-prolinyl)]phosphoramidate-D-glucopyranose;
2-deoxy-2-(pent-4-enoylamino)-3-*O*-[4-methoxyphenyl(ethoxy-L-prolinyl)]phosphoramidate-D-glucopyranose;
2-deoxy-2-(pent-4-enoylamino)-4-*O*-[4-methoxyphenyl(ethoxy-L-prolinyl)]phosphoramidate-D-glucopyranose;
2-acetamido-2-deoxy-3-*O*-[4-methoxyphenyl(benzyloxy-D-prolinyl)]phosphoramidate-D-glucopyranose;
2-acetamido-2-deoxy-4-*O*-[4-methoxyphenyl(benzoxy-D-prolinyl)]phosphoramidate-D-glucopyranose;
2-acetamido-2-deoxy-3-*O*-[4-methoxyphenyl(ethoxy-D-prolinyl)]phosphoramidate-D-glucopyranose;
2-acetamido-2-deoxy-4-*O*-[4-methoxyphenyl(ethoxy-D-prolinyl)]phosphoramidate-D-glucopyranose;
methyl 2-acetamido-2-deoxy-3-*O*-[4-methoxyphenyl(ethoxy-L-sarcosinyl)]phosphoramidate-D-glucopyranoside;
methyl 2-acetamido-2-deoxy-3-*O*-[4-methoxyphenyl(benzyloxy-L-valinyl)]phosphoramidate-D-glucopyranoside;
methyl 2-acetamido-2-deoxy-4-*O*-[4-methoxyphenyl(benzyloxy-L-valinyl)]phosphoramidate-D-glucopyranoside;
methyl 2-acetamido-2-deoxy-3-*O*-[4-methoxyphenyl(ethoxy-L-valinyl)]phosphoramidate-D-glucopyranoside;
2-acetamido-2-deoxy-3-*O*-[4-methoxyphenyl(ethoxy-L-sarcosinyl)]phosphoramidate-D-glucopyranose;
2-acetamido-2-deoxy-3-*O*-[4-methoxyphenyl(benzyloxy-L-valinyl)]phosphoramidate-D-glucopyranose;
2-acetamido-2-deoxy-4-*O*-[4-methoxyphenyl(benzyloxy-L-valinyl)]phosphoramidate-D-glucopyranose;
2-acetamido-2-deoxy-3-*O*-[4-methoxyphenyl(isopropoxy-L-valinyl)]phosphoramidate-D-glucopyranose;
2-acetamido-2-deoxy-4-*O*-[4-methoxyphenyl(isopropoxy-L-valinyl)]phosphoramidate-D-glucopyranose;
2-acetamido-2-deoxy-3-*O*-[4-methoxyphenyl(ethoxy-L-valinyl)]phosphoramidate-D-glucopyranose;
2-acetamido-2-deoxy-4-*O*-[4-methoxyphenyl(ethoxy-L-valinyl)]phosphoramidate-D-glucopyranose;
2-acetamido-2-deoxy-3-*O*-[4-methoxyphenyl(ethoxy-L-valinyl)]phosphoramidate-D-glucopyranose;
2-acetamido-2-deoxy-3-*O*-[4-methoxyphenyl(tert butoxy-L-valinyl)]phosphoramidate-D-glucopyranose;
2-acetamido-2-deoxy-4-*O*-[4-methoxyphenyl(tert butoxy-L-valinyl)]phosphoramidate-D-glucopyranose;
benzyl 2-acetamido-2-deoxy-4,6-*O*-benzylidene-3-O-[4-methoxyphenyl(methoxy-L-valinyl)]phosphoramidate-D-glucopyranoside;
2-acetamido-2-deoxy-3-*O*-[4-methoxyphenyl(methoxy-L-valinyl)]phosphoramidate-D-glucopyranose;
2-acetamido-2-deoxy-3-*O*-[4-methoxyphenyl(cyclohexyloxy-L-valinyl)]phosphoramidate-D-glucopyranose;
2-acetamido-2-deoxy-4-*O*-[4-methoxyphenyl(cyclohexyloxy-L-valinyl)]phosphoramidate-D-glucopyranose;
2-acetamido-2-deoxy-3-*O*-[4-methoxyphenyl(2-butoxy-L-valinyl)]phosphoramidate-D-glucopyranose;
2-acetamido-2-deoxy-4-*O*-[4-methoxyphenyl(2-butoxy-L-valinyl)]phosphoramidate-D-glucopyranose;
2-deoxy-2-(pent-4-enoylamino)-4-*O*-[4-methoxyphenyl(benzyloxy-L-valinyl)]phosphoramidate-*D*-glucopyranose;
2-deoxy-2-(pent-4-enoylamino)-3-*O*-[4-methoxyphenyl(benzyloxy-*L-*valinyl)]phosphoramidate-*D*-glucopyranose;
2-amino-2-deoxy-4-*O*-[4-methoxyphenyl(benzyloxy-*L*-valinyl)]phosphoramidate-*D-*glucopyranose;
2-amino-2-deoxy-3-*O*-[4-methoxyphenyl(benzyloxy-*L*-valinyl)]phosphoramidate-*D-*glucopyranose;
2-deoxy-2-(pent-4-enoylamino)-4-*O*-[phenyl(ethoxy-L-prolinyl)]phosphoramidate-D-glucopyranose;
2-deoxy-2-(pent-4-enoylamino)-3-*O*-[phenyl(ethoxy-L-prolinyl)]phosphoramidate-D-glucopyranose;
2-amino-2-deoxy-4-*O*-[phenyl(ethoxy-L-prolinyl)]phosphoramidate-D-glucopyranose;
2-amino-2-deoxy-3-O-[phenyl(ethoxy-L-prolinyl)]phosphoramidate-D-glucopyranose
and pharmaceutically acceptable derivatives thereof.

15. A compound of formula (I) as defined in anyone of claims 1 to 14, or a pharmaceutically acceptable salt, solvate and prodrug thereof, for use as a medicament.

16. A pharmaceutical composition comprising at least one compound of formula (I) as defined in anyone of claims 1 to 14, or a pharmaceutically acceptable salt, solvate or prodrug thereof, and at least one pharmaceutically acceptable excipient.

17. A compound of formula (I) as defined in anyone of claims 1 to 14, or a pharmaceutically acceptable salt, solvate or prodrug thereof, for use in the treatment or prevention of osteoarthritis in a mammal.

18. A compound of formula (I) as defined in anyone of claims 1 to 14, or a pharmaceutically acceptable salt, solvate or prodrug thereof, for use in the treatment or prevention of a virus infection in a mammal.

19. A compound of formula (I) as defined in anyone of claims 1 to 14, or a pharmaceutically acceptable salt, solvate or prodrug thereof, for use in the treatment or prevention of cancer in a mammal.

20. Use of a compound of formula (I) as defined in anyone of claims 1 to 14, or a pharmaceutically acceptable salt, solvate or prodrug thereof, in the preparation of a medicament for the treatment or prevention of osteoarthritis in a mammal.

21. Use of a compound of formula (I) as defined in anyone of claims 1 to 14, or a pharmaceutically acceptable salt, solvate or prodrug thereof, in the preparation of a medicament for the treatment or prevention of a virus infection in a mammal.

22. Use of a compound of formula (I) as defined in anyone of claims 1 to 14, or a pharmaceutically acceptable salt, solvate or prodrug thereof, in the preparation of a medicament for the treatment or prevention of cancer in a mammal.

23. A process for the preparation of a compound of formula (I) as defined in anyone of claims 1 to 14 comprising the step of reacting a compound of formula (II), 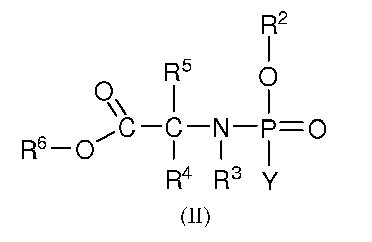 wherein
R², R³, R⁴, R⁵ and R⁶ are independently selected and are as defined in anyone of claims 1 to 13; and
Y is a leaving group;
with a compound of formula (III) or (IV), 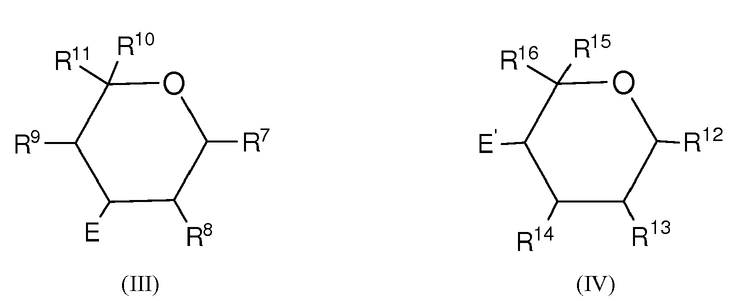 wherein
E and E' are independently selected from groups capable of displacing a group Y from a compound of formula (II); and
R⁷-R¹⁶ are independently selected and are as defined in anyone of claims 1 to 13.
